# EUROPEAN PATENT APPLICATION

(11) **EP 3 006 029 A1**
(43) Date of publication of application: **13.04.2016**
(21) Application number: 14808338.9
(22) Date of filing: 06.06.2014
(51) Int. Cl.: A61K 31/4439, A61K 31/444, A61K 35/14, A61P 7/00

(54) **COMPOSITION FOR MAINTAINING PLATELET FUNCTION**

(30) Priority: 07.06.2013 JP 2013120696
(71) Applicant: Kaken Pharmaceutical Co., Ltd., Tokyo 113-8650 (JP); Kyoto University, Kyoto-shi, Kyoto 606-8501 (JP)
(72) Inventor: HIRATA, Shinji, Kyoto-shi Kyoto 607-8042 (JP); MURATA Takahiko, Kyoto-shi Kyoto 607-8042 (JP); ETO Koji, Kyoto-shi Kyoto 606-8501 (JP)
(74) Representative: Clegg, Richard Ian
(86) International application number: PCT/JP2014/065061
(87) International publication number: WO 2014/196624

(57) **Abstract**

Provided is a composition for maintaing a function of platelets, having as an active ingredient a pyridone derivative represented by the following general formula (I), or a salt thereof (in the formula, ring A, R¹, R², R³, and R⁴ represent the definitions given in the description).

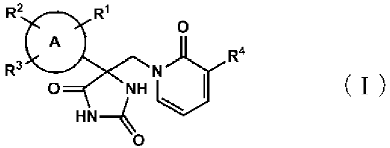

## Description

### Technical Field

The present invention relates to a composition for maintaining a function of platelets, a method for preparing platelets, a platelet-containing blood product, and a method for maintaining a function of platelets in a blood product, which utilize a pyridone derivative.

### Background Art

Platelets are essential for blood coagulation (hemostasis). Accordingly, there is an extremely high demand for platelets for leukemia, bone marrow transplantation, anticancer therapy, and so forth

Attempts have been made to produce platelets by collecting platelets from blood donated from donors, differentiating umbilical cord blood or bone marrow cells into megakaryocytes, and growing hematogenic precursor cells and preparing platelets from those precursor cells. For example, the present inventors have reported a method for producing platelets efficiently and in a relatively large amount from a net-like structure enclosing hematopoietic progenitor cells, after the net-like structure was prepared from human embryonic stem cells (ES cells) (Patent Literature 1), and a method for efficiently preparing mature megakaryocytes and platelets from induced pluripotent stem cells (iPS cells) in an in-vitro culture system (Patent Literature 2), and the like.

Binding between platelets and an extracellular matrix is an important process for inducing blood coagulation (hemostasis, thrombus formation, and the like). It is believed that this process is initiated when a platelet receptor GPIb (glycoprotein Ib) binds to von Willebrand factor (VWF) via an α-subunit (GPIbα). However, it has been reported that under a condition of around 37°C, a metalloproteinase ADAM17 (a disintegrin and metallopeptidase domain 17) sheds an extracellular region of GPIbα (release by cleavage), thereby inhibiting the association between GPIbα and VWF, and the platelets lose blood coagulating ability (Non-Patent Literature 1 and 2).

Consequently, it is anticipated that at least the activity of ADAM 17 needs to be suppressed in order to maintain a function of platelets that have been prepared in vitro. Actually, the present inventors have reported that a function of platelets prepared in vitro can be maintained by adding at an appropriate timing an inhibitor (such as GM6001) directly inhibiting metalloproteinase activity (Patent Literature 3). It has also been reported that even N-hydroxyformamide derivatives having an ADAM 17 inhibitory action can maintain platelet function (Patent Literature 4). In that report, it is clarified that the number of functional platelets that are produced is increased by increasing ADAM 17 specificity.

Thus, a method for maintaining platelet function is important for the production of platelets and as a storage method of a platelet preparation to be used for transfusion. Research into such a method has been started by the present inventors, and is now continuing.

### Citation List

### Patent Literature

[Patent Literature 1] WO 08/041370 Pamphlet
[Patent Literature 2] WO 09/122747 Pamphlet
[Patent Literature 3] WO 09/119105 Pamphlet
[Patent Literature 4] WO 12/036257 Pamphlet

### Non Patent Literature

[Non-Patent Literature 1] Bergmeier et al., Circulation Research, 2004, vol. 95, pp. 677-683
[Non-Patent Literature 2] Bergmeier et al., Blood, 2003, vol. 102, pp. 4229-4235

### Summary of Invention

### Technical Problem

In view of such circumstances, although research into methods for preparing platelets themselves outside of the cell is developing, there is a continuing need for a novel compound having an action for maintaining the function of prepared platelets.

Therefore, the present invention is directed to identifying a novel compound capable of maintaining a function of platelets by specifically suppressing metalloprotease activity of ADAM 17 and suppressing GPIbα shedding. The present invention is also directed to providing a composition for maintaining a function of platelets that utilizes such identified compound, efficiently growing platelets, and improving quality maintenance of a blood product.

In order to achieve the above-described objects, the present inventors searched for a compound capable of differentiating megakaryocytes from hematogenic precursor cells derived from iPS cells and the like, and specifically suppressing ADAM17-mediated GPIbα shedding that occurs during culturing in platelet production from such megakaryocytes. As a result, the present inventors discovered that a compound group having a given specific structure has a specific inhibitory action on ADAM17, and that when such a compound is added to a culture system for producing platelets or human peripheral blood-derived platelets, GPIbα shedding is suppressed and a function of platelets can be maintained even under a temperature condition of around 37°C. In other words, the present inventors discovered that, based on such effects of this compound, the compound, and an analog thereof, is very useful for efficient production of platelets and in improving quality maintenance of a platelet-containing blood product, thereby completing the present invention.

More specifically, the present invention relates to at least the following aspects.
(1) A composition for maintaining a function of platelets, the composition comprising as an active ingredient a pyridone derivative, or a salt thereof, represented by formula (I), 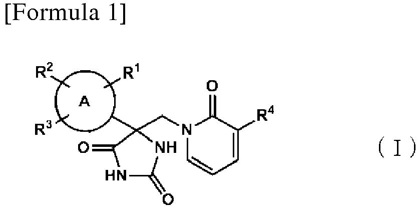 [wherein ring A represents an aryl, a heteroaryl, or a group represented by the following formula (a), 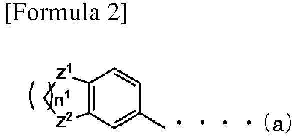
   (wherein Z¹ and Z² each independently represent -CH₂- or -O-, and n¹ denotes an integer of 1 to 3),
   R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or
   -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, - SO₂NR⁶-, -NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
   R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 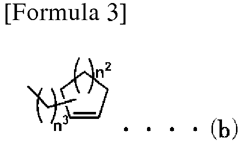
   (wherein n² denotes an integer of 1 to 3 and n³ denotes an integer of 0 to 3),
   an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
   R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
   R³ represents a hydrogen atom, a halogen atom, or a C1-C6 alkyl group, and
   R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].
(2) The composition according to (1), wherein a compound represented by the formula (I) is (+)-5-(3,4-difluorophenyl)-5-[(3-methyl-2-oxopyridin-1(2H)-yl)methyl]imidazolidine-2,4-dione.
(3) The composition according to (1) or (2), wherein the composition is a reagent for maintaining a function of platelets or is an additive to a platelet-containing blood product.
(4) A method for preparing platelets, the method comprising adding, to a culture system for differentiating megakaryocytes from cells capable of differentiating into megakaryocytes and producing platelets from the megakaryocytes, a pyridone derivative, or a salt thereof, represented by formula (I), 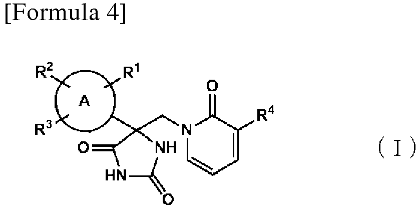 [wherein ring A represents an aryl, a heteroaryl, or a group represented by the following formula (a), 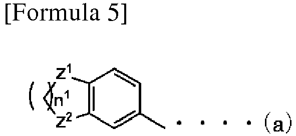
   (wherein Z¹ and Z² each independently represent -CH₂- or -O-, and n¹ denotes an integer of 1 to 3),
   R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO₂NR⁶-, - NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
   R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 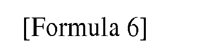 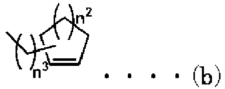
   (wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
   R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
   R³ represents a hydrogen atom, a halogen atom, or a C1-C6 alkyl group, and
   R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].
(5) The method according to (4), wherein a compound represented by the formula (I) is (+)-5-(3,4-difluorophenyl)-5-[(3-methyl-2-oxopyridin-1(2H)-yl)methyl]imidazolidine-2,4-dione.
(6) The method according to (4) or (5), wherein a culture temperature in the culture system is from 35 to 39°C.
(7) A culture obtained by adding, to a culture system for differentiating megakaryocytes from cells capable of differentiating into megakaryocytes and producing platelets from the megakaryocytes, a pyridone derivative, or a salt thereof, represented by formula (I), 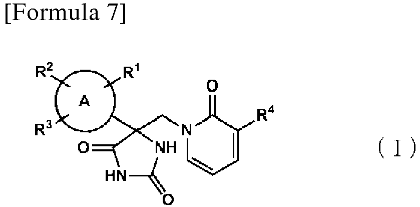 [wherein ring A represents an aryl, a heteroaryl, or a group represented by the following formula (a), 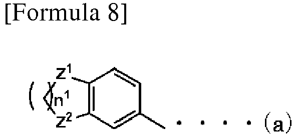
   (wherein Z¹ and Z² each independently represent -CH₂- or -O-, and n¹ denotes an integer of 1 to 3),
   R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO₂NR⁶-, - NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
   R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 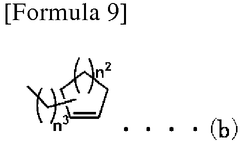
   (wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
   R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
   R³ represents a hydrogen atom, a halogen atom, or a C1-C6 alkyl group, and
   R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].
(8) The culture according to (7), wherein a compound represented by the formula (I) is (+)-5-(3,4-difluorophenyl)-5-[(3-methyl-2-oxopyridin-1(2H)-yl)methyl]imidazolidine-2,4-dione.
(9) A platelet-containing blood product to which has been added a pyridone derivative, or a salt thereof, represented by formula (I), 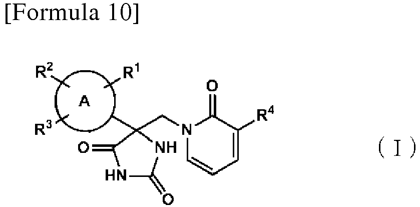 [wherein ring A represents an aryl, a heteroaryl, or a group represented by the following formula (a), 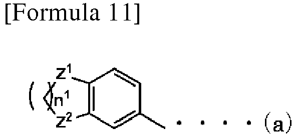
   (wherein Z¹ and Z² each independently represent -CH₂- or -O-, and n¹ denotes an integer of 1 to 3),
   R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO₂NR⁶-, - NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
   R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 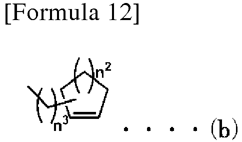
   (wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
   R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
   R³ represents a hydrogen atom, a halogen atom, or a C1-C6 alkyl group, and
   R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].
(10) The blood product according to (9), wherein a compound represented by the formula (I) is (+)-5-(3,4-difluorophenyl)-5-[(3-methyl-2-oxopyridin-1(2H)-yl)methyl]imidazolidine-2,4-dione.
(11) A method for maintaining a function of platelets in a blood product, the method comprising adding, to a platelet-containing blood product, a pyridone derivative, or a salt thereof, represented by formula (I), 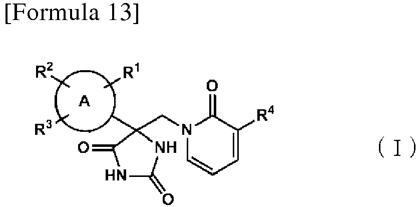 [wherein ring A represents an aryl, a heteroaryl, or a group represented by the following formula (a), 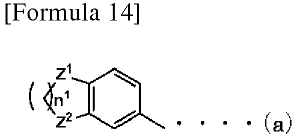
   (wherein Z¹ and Z² each independently represent -CH₂- or -O-, and n¹ denotes an integer of 1 to 3),
   R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO₂NR⁶-, - NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
   R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 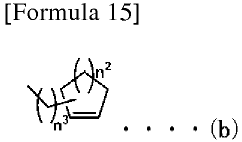
   (wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
   R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
   R³ represents a hydrogen atom, a halogen atom, or a C1-C6 alkyl group, and
   R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].
(12) The method according to (11), wherein a compound represented by the formula (I) is (+)-5-(3,4-difluorophenyl)-5-[(3-methyl-2-oxopyridin-1(2H)-yl)methyl]imidazolidine-2,4-dione.

### Advantageous Effects of the Invention

The pyridone derivative according to the present invention is capable of maintaining a function of platelets even under a temperature condition of around 37°C, by specifically suppressing metalloprotease activity of ADAM 17 and suppressing GPIbα shedding. Therefore, with this derivative, in in vitro, functionally stable platelets can be produced, and the quality of a platelet-containing blood product can be stabilized.

### Brief Description of Drawings

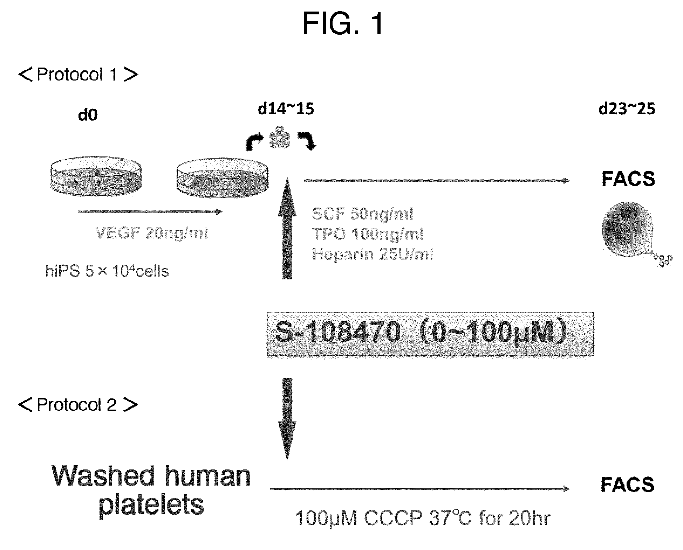

[FIG. 1] <Protocol 1> illustrates addition timing of a compound (S-108470) and the like according to the present invention in an induction process from iPS cell-derived hematopoietic progenitor cells into megakaryocytes/platelets. <Protocol 2> illustrates that washed platelets were prepared from human peripheral blood, and the compound (S-108470) and the like according to the present invention was added thereto.

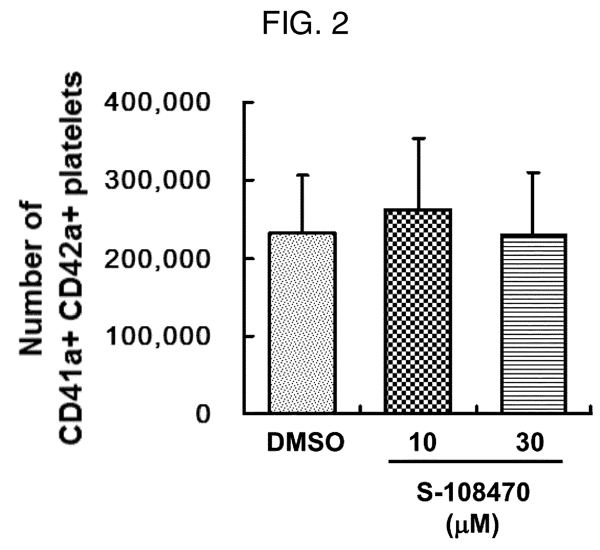

[Fig. 2] Fig. 2 is a graph for showing the effect of S-108470 on the number of produced iPS cell-derived platelets. The platelets were induced from iPS cells, and the number of cells having the surface marker CD41a (+) and CD42a (+) counted. The test was repeated 7 times, and the average and standard deviation are shown.

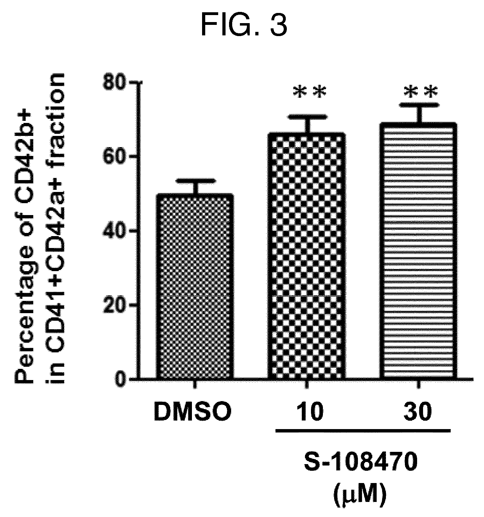

[Fig. 3] Fig. 3 is a graph for showing a function-maintaining effect of S-108470 on iPS cell-derived platelets. The vertical axis represents the percentage of CD42b (GPIbα) (+) cells to CD41a (+) and CD42a (+) platelets. In FIG. 3, the symbol ** indicates that p<0.01 with respect to the medium control (DMSO), and S-108470 increased the percentage of CD42b (+) cells.

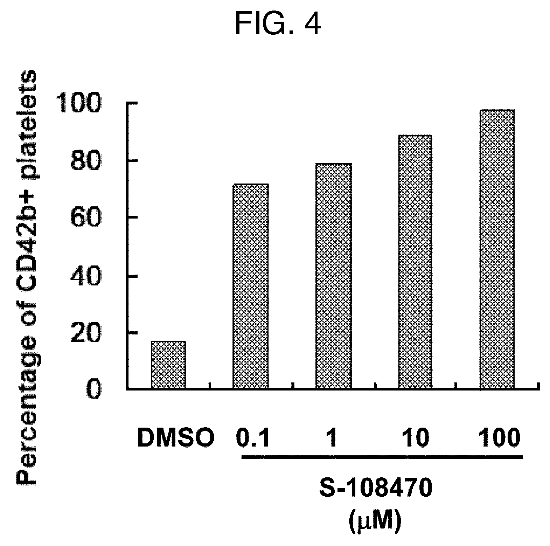

[Fig. 4] Fig. 4 is a graph for showing a function-maintaining effect of S-108470 on concentration-dependent peripheral blood-derived platelets. The vertical axis represents the percentage of the number of CD42b (GPIbα) (+) cells to CD41a (+) platelets. When fresh platelets were treated with CCCP, CD42b (GPIbα) of the platelets was shed, and the percentage of the number of CD42b (GPIbα) (+) cells decreased similar to the medium control (DMSO). This indicates that S-108470 is suppressed in a concentration-dependent manner.

### Description of Embodiments

The composition according to the present invention is a compound for maintaining a function of platelets, the composition including as an active ingredient a pyridone derivative, or a salt thereof, represented by formula (I), 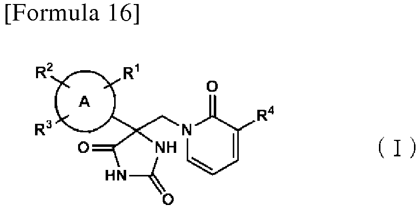 [wherein ring A represents an aryl, a heteroaryl, or a group represented by the following formula (a), 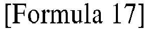 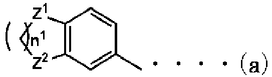
(wherein Z¹ and Z² each independently represent -CH₂- or -O-, and n¹ denotes an integer of 1 to 3),
R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or
-J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, - SO₂NR⁶-, -NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 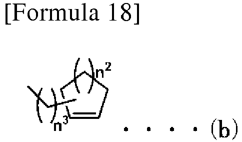
(wherein n² denotes an integer of 1 to 3 and n³ denotes an integer of 0 to 3),
an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
R³ represents a hydrogen atom, a halogen atom, or a C1-C6 alkyl group, and
R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].

To describe the respective substituents in the above-described formula (I), the meaning of the terms used in the definition of these substituents is as follows.

A "halogen atom" is a fluorine atom, a chlorine atom, a bromine atom or an iodine atom.

The term "C1-C6 alkyl group" means a straight or branched alkyl group having 1 to 6 carbon atoms. Specific examples include a methyl group, an ethyl group, an n-propyl group, an isopropyl group, an n-butyl group, an isobutyl group, a tert-butyl group, a sec-butyl group, an n-pentyl, a tert-pentyl group, a 3-methylbutyl group (isopentyl group), a neopentyl group, an n-hexyl group and the like.

The term "C1-C6 haloalkyl group" means a group in which one or more of the above-described halogen atoms are substituted at any substitutable position(s) on the above-described C1-C6 alkyl group. Specific examples include a trifluoromethyl group, a pentafluoroethyl group, a trichloromethyl group, a 3-chloropropyl group, a 4-bromobutyl group, a 1,1,1,3,3,3-hexafluoropropan-2-yl group and the like.

The term "C1-C6 alkoxy group" means an alkoxy group in which the alkyl moiety is the same meaning as that of the above-described "C1-C6 alkyl group". Examples include a straight or branched alkoxy group, such as a methoxy group, an ethoxy group, an n-propoxy group, an isopropoxy group, an n-butoxy group, an isobutoxy group, a tert-butoxy group, a sec-butoxy group, an n-pentyloxy group, a tert-amyloxy group, a 3-methylbutoxy group, a neopentyloxy group, and an n-hexyloxy group.

The term "C1-C6 alkoxycarbonyl group" means an alkoxycarbonyl group in which the alkyl moiety is the above-described "C1-C6 alkyl group". Examples include a straight or branched C1-C6 alkoxycarbonyl group, such as a methoxycarbonyl group, an ethoxycarbonyl group, an n-propoxycarbonyl group, an isopropoxycarbonyl group, an n-butoxycarbonyl group, an isobutoxycarbonyl group, a tert-butoxycarbonyl group, a sec-butoxycarbonyl group, an n-pentyloxycarbonyl group, a tert-amyloxycarbonyl group, a 3-methylbutoxycarbonyl group, a neopentyloxycarbonyl group, and an n-hexyloxy carbonyl group.

The term "cycloalkyl group" refers to a monocyclic saturated carbon ring having 3 to 7 carbon atoms, examples thereof including a cyclopropyl group, a cyclobutyl group, a cyclopentyl group, a cyclohexyl group, a cycloheptyl group, and the like.

The term "carbon ring" refers to a 3- to 10-membered monocyclic or bicyclic ring composed of carbon atoms. Specific examples include, but are not limited thereto, a cyclopentene ring, a cyclohexene ring, a benzene ring and the like.

It should be noted that "carbon ring" herein means the above-described "carbon ring".

The term "cycloalkylalkyl group" refers to the above-described "C1-C6 alkyl group" which is substituted with an above-described "cycloalkyl group". Such substitution may be carried out at any of the substitutable position(s). Examples include a cyclopropylmethyl group, a cyclobutylmethyl group, a cyclopentylmethyl group, a cyclopentylethyl group (e.g., a 2-cyclopentylethyl group), a cyclohexylmethyl group, a cyclohexylpropyl group (e.g., a 3-cyclohexylpropyl group) and the like, each of which may have an optional substituent in cycloalkyl moiety.

The term "heterocycloalkyl group" represents a saturated monocyclic heterocyclic ring. Examples include a pyrrolidinyl group (e.g., a 1-pyrrolidinyl group, a 2-pyrrolidinyl group, and a 3-pyrrolidinyl group), a piperidinyl group (e.g., a 1-piperidinyl group and a 4-piperidinyl group), a homopiperidinyl group (e.g., a 1-homopiperidinyl group and a 4-homopiperidinyl group), a tetrahydrofuranyl group (e.g., a 2-tetrahydrofuranyl group, a 3-tetrahydrofuranyl group), a tetrahydropyranyl group (e.g., a 4-tetrahydropyranyl group), a piperazinyl group (e.g., a 1-piperazinyl group), a homopiperazinyl group (1-homopiperazinyl group) and the like.

The term "heterocyclic ring" represents a 3- to 10-membered monocyclic ring or bicyclic ring composed of carbon atoms and 1 to 3 hetero atoms independently selected from N, O, and S. In this heterocyclic ring, the nitrogen atom and the sulfur atom may be optionally oxidized, and the nitrogen atom may also be optionally quaternized. In addition, this heterocyclic ring may be optionally substituted, and optionally fused with a carbon ring or another heterocyclic ring. Furthermore, this heterocyclic ring can be bonded at any of the substitutable position. Specific examples include, but are not limited thereto, a dioxole ring, an oxathiole ring, a dihydroxathiine ring, a dihydrodioxine ring, a dihydrofuran ring, a dihydrothiophene ring, a dihydropyrrole ring, a furan ring, a thiophene ring, a pyrrole ring, an oxazole ring, a thiazole ring, a pyridine ring and the like.

It should be noted that "heterocyclic ring" herein means the above-described "heterocyclic ring".

The term "heterocycloalkylalkyl group" refers to the above-described "C1-C6 alkyl group" which is substituted with the above-described "heterocycloalkyl group". Such substitution may be carried out at any of the substitutable position(s). Examples include a pyrrolidinylmethyl group (e.g., a 1-pyrrolidinylmethyl group, a 2-pyrrolidinylmethyl group, and a 3-pyrrolidinylmethyl group), a piperidinylmethyl group (e.g., a 1-piperidinylmethyl group and a 4-piperidinylmethyl group), a piperidinylethyl group (e.g., a 1-piperidinylethyl group and a 4-piperidinyl-2-ethyl group), a homopiperidinylmethyl group (e.g., a 1-homopiperidinylmethyl group and a 4-homopiperidinylmethyl group), a tetrahydrofuranylmethyl group (e.g., a 2-tetrahydrofuranylmethyl group and a 3-tetrahydrofuranylmethyl group), a tetrahydropyranylmethyl group (e.g., a 4-tetrahydropyranyl group), a piperazinylmethyl group (e.g., a 1-piperazinylmethyl group), a homopiperazinylmethyl group (e.g., a 1-homopiperazinylmethyl group) and the like.

The term "aryl group" represents an aromatic carbon ring. Examples include a phenyl group, a naphthyl group and the like.

The term "aralkyl group" refers to the above-described "C1-C6 alkyl group" which is substituted with the above-described "aryl group". Such substitution may be carried out at any of the substitutable position(s). Examples include a benzyl group, a phenethyl group, a 1-phenylethyl group, a 1-phenylpropyl group, a 3-phenylpropyl group, an α-naphthylmethyl group, a β-naphthylmethyl group, a 1-(α-naphthyl)ethyl group, a 2-(α-naphthyl)ethyl group and the like, each of which may have an optional substituent in aryl moiety.

The term "heteroaryl group" represents a 5- to 10-membered monocyclic or bicyclic aromatic heterocyclic ring. Examples include a pyrrolyl group (e.g., a 2-pyrrolyl group), a furyl group (e.g., a 3-furyl group), a thienyl group (e.g., a 2-thienyl group), an imidazolyl group (e.g., a 4-imidazolyl group), a pyrazolyl group (e.g., a 3-pyrazolyl group), an oxazolyl group (e.g., a 2-oxazolyl group), an isoxazolyl group (e.g., a 3-isoxazolyl group), a thiazolyl group (e.g., a 2-thiazolyl group), an isothiazolyl group (e.g., a 3-isothiazolyl group), a pyridyl group (e.g., a 2-pyridyl group, a 3-pyridyl group, and a 4-pyridyl group), a pyridazinyl group (e.g., a 3-pyridazinyl group), a pyrimidyl group (e.g., a 4-pyrimidyl group), a pyrazinyl group (e.g., a 2-pyrazinyl group), an indolyl group (e.g., a 2-indolyl group, a 3-indolyl group, and a 4-indolyl group), a benzofuryl group (e.g., a 2-benzofuryl group and a 5-benzofuryl group), a benzothienyl group (e.g., a 2-benzothienyl group and a 5-benzothienyl group), a benzimidazolyl group (e.g., a 2-benzimidazolyl group), an indazolyl group (e.g., a 4-indazolyl group), a benzooxazolyl group (e.g., a 4-benzoxazolyl group), a benzothiazolyl group (e.g., a 4-benzothiazolyl group), a benzoisoxazolyl group (e.g., a 4-benzoisoxazolyl group), a benzoisothiazolyl group (e.g., a 4-benzoisothiazolyl group), a quinolyl group (e.g., a 2-quinolyl group, a 4-quinolyl group, a 5-quinolyl group, and an 8-quinolyl group), an isoquinolyl group (e.g., a 1-isoquinolyl group, a 4-isoquinolyl group, a 5-isoquinolyl group, and an 8-isoquinolyl group), a cinnolinyl group (e.g., a 4-cinnolinyl group, a 5-cinnolinyl group, and an 8-cinnolinyl group), a quinazolinyl group (e.g., a 4-quinazolinyl group, a 5-quinazolinyl group, and an 8-quinazolinyl group), a tetrazolyl group (e.g., a 2H-tetrazol-5-yl group) and the like. Such heteroaryl group may have an optional substituent.

The term "heteroarylalkyl group" refers to the above-described "C1-C6 alkyl group" which is substituted with the above-described "heteroaryl group". Such substitution may be carried out at any of the substitutable position(s). Examples include a pyridylmethyl group (e.g., a 2-pyridylmethyl group), an oxazolylethyl group (e.g., a 2-oxazolyl-2-ethyl group), a thiazolylmethyl group (e.g., a 4-thiazolylmethyl group), an indolylmethyl group (e.g., a 2-indolylmethyl group, a 3-indolylmethyl group, and a 4-indolylmethyl group), a benzofurylmethyl group (e.g., a 3-benzofurylmethyl group and a 4-benzofurylmethyl group), a benzothienylpyridinemethyl group (e.g., a 3-benzothienylpyridinemethyl group and a 4-benzothienylpyridinemethyl group), a benzothiazolylmethyl group (e.g., a 2-benzothiazolylmethyl group), a quinolylmethyl group (e.g., a 2-quinolylmethyl group, a 4-quinolylmethyl group, a 5-quinolylmethyl group, and a 8-quinolylmethyl group), an isoquinolylmethyl group (e.g., a 1-isoquinolylmethyl group, a 4-isoquinolylmethyl group, a 5-isoquinolylmethyl group, and an 8-isoquinolylmethyl group), a cinnolinylmethyl group (e.g., a 4-cinnolinylmethyl group, a 5-cinnolinylmethyl group, and an 8-cinnolinylmethyl group), a quinazolinylmethyl group (e.g., a 4-quinazolinylmethyl, a 5-quinazolinylmethyl group, and an 8-quinazolinylmethyl group) and the like. Such heteroaryl group may have an optional substituent.

The term "C2-C6 alkenyl group" means a straight or branched alkenyl group having 2 to 6 carbon atoms and having one or more double bonds. Specific examples include a vinyl group, a 1-propenyl group, an isopropenyl group, a 1-butenyl group, an isobutenyl group, a 1,3-butadienyl group, a 2-methyl-1-propenyl group, a 1-methyl-1-propenyl group, a 1-pentenyl group, a 1-hexenyl group and the like.

The term "alkenylalkyl group" refers to the above-described "C1-C6 alkyl group" which is substituted with the above-described "C2-C6 alkenyl group". Such substitution may be carried out at any of the substitutable position(s). Examples include an allyl group, a 2-pentenyl group, a 4-pentenyl group, a prenyl group, a 2-hexenyl group, a 5-hexenyl, a 2-methylallyl group, a but-3-en-1-yl group, a 2-methylbut-3-en-1-yl group and the like.

The term "heterocycloalkenyl group" represents a monocyclic heterocyclic ring having one double bond at any position of the ring. Examples include a dihydrofuryl group (e.g., a 2,5-dihydrofuran-3-yl group), a dihydropyranyl group (e.g., a 5,6-dihydro-2H-pyran-4-yl group), a dihydropyrrolyl group (e.g., 3-pyrrolin-3-yl group), a tetrahydropyridyl group (e.g., a 1,2,3,6-tetrahydropyridin-4-yl group), a dihydrothienyl group (e.g., a 2,5-dihydrothiophen-3-yl group), a dihydrothiopyranyl group (e.g., a 5,6-dihydro-2H-thiopyran-4-yl group), a dehydrohomopiperidinyl group (e.g., a 4,5-dehydrohomopiperidin-4-yl group) and the like.

The term "heterocycloalkenylalkyl group" refers to the above-described "C1-C6 alkyl group" which is substituted with the above-described "heterocycloalkenyl group". Such substitution may be carried out at any of the substitutable position(s). Examples include a dihydrofurylmethyl group (e.g., 2,5-dihydrofuran-3-ylmethyl group), a dihydropyranylmethyl group (e.g., a 5,6-dihydro-2H-pyran-ylmethyl group), a dihydropyrrolylmethyl group (a 3-pyrrolin-3-ylmethyl group), a tetrahydropyridylmethyl group (e.g., a 1,2,3,6-tetrahydropyridin-4-ylmethyl group), a tetrahydropyridylethyl group (e.g., a 1,2,3,6-tetrahydropyridin-4-yl-2-ethyl group), a dihydrothienylmethyl group (e.g., a 2,5-dihydrothiophen-3-ylmethyl group), a dihydrothiopyranylmethyl group (e.g., a 5,6-dihydro-2H-thiopyran-4-ylmethyl group), a dehydrohomopiperidinylmethyl group (e.g., a 4,5-dehydrohomopiperidin-4-ylmethyl group) and the like.

The term "C2-C6 alkynyl group" means a straight or branched alkynyl group having 2 to 6 carbon atoms and having one or more triple bonds. Specific examples include an ethynyl group, a 1-propynyl group, a 1-butynyl group, a 3-methyl-1-butynyl group, a 1,3-butadiynyl group, a 1-pentynyl group, a 3-methyl-1-pentynyl group, a 1-hexynyl group and the like.

The term "alkynyl alkyl group" refers to the above-described "C1-C6 alkyl group" which is substituted with the above-described "C2-C6 alkynyl group". Such substitution may be carried out at any of the substitutable position(s). Examples include a 2-propynyl group, a 2-butynyl group, a 2-pentynyl group, a 4-methyl-2-pentynyl group and the like.

The term "alkylene" means a straight or branched alkylene group having 1 to 6 carbon atoms. Specific examples include -CH₂-, -CH₂CH₂-, -CH₂CH₂CH₂-, -CH₂CH(CH₃)CH₂- and the like.

The term "alkenylene" means a straight or branched alkenylene group having 2 to 6 carbon atoms and having one or more double bonds. Specific examples include -CH=CH-,-CH=CH-CH₂-, -CH=CH-CH₂CH₂-, -CH=C(CH₃)-CH₂- and the like.

The term "alkynylene" means a straight or branched alkynylene group having 2 to 6 carbon atoms and having one or more triple bonds. Specific examples include the following.

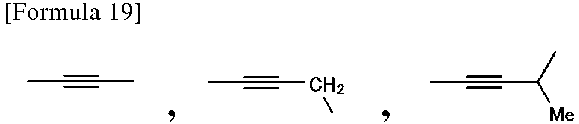

Examples of substituents on the "optionally substituted C1-C6 alkyl group", "optionally substituted C2-C6 alkenyl group", "optionally substituted alkenylalkyl group", "optionally substituted C2-C6 alkynyl group", "optionally substituted alkynylalkyl group", "optionally substituted cycloalkyl group", "optionally substituted cycloalkylalkyl group", "optionally substituted heterocycloalkyl group", "optionally substituted heterocycloalkylalkyl group", "optionally substituted heterocycloalkenyl group", and "optionally substituted heterocycloalkenylalkyl group" include a hydroxyl group, a halogen atom, a cyano group, a nitro group, a trifluoromethyl group, an optionally substituted C1-C6 alkoxy group, a cycloalkyl group, a carboxyl group, a C1-C6 alkoxycarbonyl group, an -NR⁸R⁹ {wherein R⁸ and R⁹ each independently represent a hydrogen atom, a C1-C6 alkyl group, a formyl group, an optionally substituted acyl group, -CONR¹⁰R¹¹ [wherein R¹⁰ and R¹¹ each independently represent a hydrogen atom, a C1-C6 alkyl group, an aryl group optionally substituted with R¹² (wherein R¹² represents a C1-C6 alkyl group, a C1-C6 alkoxy group, or a halogen atom), a heteroaryl group optionally substituted with R¹² (R¹² being defined as described above), or form a nitrogen-containing heterocyclic ring together with the nitrogen atom to which R¹⁰ and R¹¹ are attached], a cycloalkyl group, or form a nitrogen-containing heterocyclic ring together with the nitrogen atom to which R⁸ and R⁹ are attached}, or -OCNOR¹³ [wherein R¹³ represents a C1-C6 alkyl group, an aryl group optionally substituted with R¹² (R¹² being defined as described above), a heteroaryl group optionally substituted with R¹² (R¹² being defined as described above), or -NR¹⁴R¹⁵ (wherein R¹⁴ and R¹⁵ each independently represent a hydrogen atom, a C1-C6 alkyl group, an aryl group optionally substituted with R¹² (R¹² being defined as described above), a heteroaryl group optionally substituted with R¹² (R¹² being defined as described above), or form a nitrogen-containing heterocyclic ring together with the nitrogen atom to which R¹⁴ and R¹⁵ are attached)] and the like. At least one substituent of these substituents may be attached to any of the substitutable position(s).

The term "acyl group" means an alkylcarbonyl group, in which the alkyl moiety is the same meaning as that of the above-described "C1-C6 alkyl group". Examples include straight or branched alkylcarbonyl groups such as an acetyl group, a propionyl group, a butyryl group, an isobutyryl group, a valeryl group, an isovaleryl group, a pivaloyl group and the like.

The term "nitrogen-containing heterocyclic ring" represents a saturated or unsaturated heterocyclic ring that includes at least one nitrogen atom. Examples include, but are not limited thereto, an azetidine ring, a pyrrolidine ring, a piperidine ring, a thiazolidine ring, a morpholine ring, a thiomorpholine ring, a dihydropyrrole ring and the like. Examples of the substituents include a C1-C6 alkyl group, a C1-C6 alkoxy group, a hydroxyl group, a nitro group, a cyano group, a trifluoromethyl group, a hydroxymethyl group and the like.

Examples of substituents on the aromatic rings in the "optionally substituted aryl group", the "optionally substituted aralkyl group", the "optionally substituted heteroaryl group", and the "optionally substituted heteroarylalkyl group" include a hydrogen atom, a halogen atom, a cyano group, a nitro group, a trifluoromethyl group, an optionally substituted C1-C6 alkyl group, an optionally substituted C1-C6 alkoxy group, a cycloalkyl group, a carboxyl group, a C1-C6 alkoxycarboxyl group, -NR¹⁴R¹⁵ (wherein R¹⁴ and R¹⁵ are defined as described above), -OCOR¹³ (wherein R¹³ is defined as described above) and the like. At least one substituent of these substituents may be attached to any of the substitutable position(s).

Examples of substituents on the "optionally substituted C1-C6 alkoxy group", the "optionally substituted acyl group", and the "optionally substituted C1-C6 alkoxycarbonyl group" include a hydrogen atom, a halogen atom, a cyano group, a nitro group, a C1-C6 alkoxy group and the like. At least one substituent of these substituents may be attached to any of the substitutable position(s).

The compound represented by formula (I), which contains at least one asymmetric carbon, may be in any form of its racemic form (i.e., the mixture of each optically-active substance), its diastereomeric form or its individual optically-active form. Further, if a geometric isomer is present, the compound of the present invention represented by formula (I) may be in any form of the (E) form, the (Z) form or their mixture form.

In addition, the salt of the pyridone derivative according to the present invention represented by formula (I) is not specifically limited as long as it is a pharmacologically acceptable salt. Such salt include, for example, a salt with an inorganic base, a salt with an organic base and the like. Examples of a salt with an inorganic base include an alkali metal salt and an alkaline earth metal salt, such as a lithium salt, a sodium salt, a potassium salt, a magnesium salt, a calcium salt, and a barium salt. Examples of a salt with an organic base include a triethylamine salt, a pyridine salt, an ethanolamine salt, a cyclohexylamine salt, a dicyclohexylamine salt, a dibenzylethanolamine salt, a benzylamine salt, a 2-methylbenzylamine salt, an α-methylbenzylamine salt, a brucine salt, a quinine salt, a quinidine salt, a cinchonine salt, a cinchonidine salt, an arginine salt and the like.

Next, the method for producing the compound represented by formula (I) is described. This compound can be produced by various methods. For example, the compound can be efficiently produced based on the production method shown below.

Specific examples of the "protecting group" used in the following production method include, as that of a hydroxyl group or a carboxyl group, a tert-butyl group, a benzyl group, an o-methylbenzyl group, a p-nitrobenzyl group, a p-methoxybenzyl group, an o-chlorobenzyl group, a 2,4-dichlorobenzyl group, a p-bromobenzyl group, an allyl group, a tert-butoxycarbonyl group, a benzyloxycarbonyl group, an o-methylbenzyloxycarbonyl group, a p-nitrobenzyloxycarbonyl group, a p-methoxybenzyloxycarbonyl group, an o-chlorobenzyloxycarbonyl group, a 2,4-dichlorobenzyloxycarbonyl group, a p-bromobenzyloxycarbonyl group, an allyloxycarbonyl group, a methoxymethyl group, a tetrahydropyranyl group and the like. As that of a carbonyl protecting group, examples include a protecting group derived from ethanediol, propanediol, mercaptoethanol, mercaptopropanol, ethanedithiol, propanedithiol and the like.

The compounds represented by formula (I) can be produced based on the method shown in the following scheme 1 (step 1 and step 2). 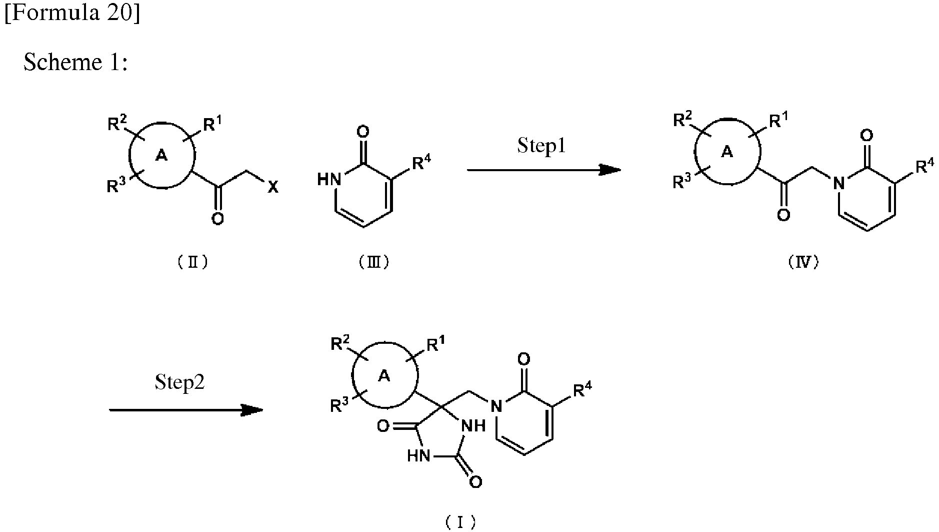 (wherein A, R¹, R², R³, and R⁴ are the same as defined above; and X represents a chlorine atom, a bromine atom, or an iodine atom)

### <Step 1>

In step 1, a compound represented by formula (II) and a compound represented by formula (III) are reacted in the presence of a base to produce a compound represented by formula (IV). Instead of the compound represented by formula (III), a compound represented by formula (V), 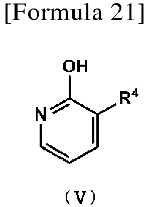 which is a tautomer of the compound represented by formula (III), may be used.. Examples of preferred bases include potassium carbonate, sodium carbonate, cesium carbonate, sodium hydride and the like. In addition, to promote this reaction, an additive may be added Examples of such additives include potassium iodide, sodium iodide, tetrabutylammonium iodide, potassium bromide, sodium bromide, tetrabutylammonium bromide and the like. The reaction solvent is not specifically limited as long as it does not significantly inhibit the reaction. Examples of preferred reaction solvents include N,N-dimethylformamide, dimethyl sulfoxide, acetone, acetonitrile, methanol, ethanol, tetrahydrofuran, 1,4-dioxane, 2-methoxyethanol, a mixed solvent thereof and the like. Further, water can be added to the reaction solvent. In the case of adding water, although the added amount of water is not especially limited, it is preferably 10% or less, for example, based in the reaction solvent. Although the reaction temperature is not especially limited, for example, from room temperature to 60°C is preferred. The reaction time is preferably from 1 hour to 2 days.

### <Step 2>

In step 2, compound (I) is produced by reacting the compound represented by formula (IV) (hereinafter sometimes referred to as compound (IV); the compounds represented by other formula are also denoted in a similar manner) with a cyanide in the presence of a salt. Examples of preferred salts include ammonium carbonate, ammonium bicarbonate and the like. Examples of preferred cyanides include potassium cyanide, sodium cyanide and the like. The reaction solvent is not specifically limited as long as it does not significantly inhibit the reaction. Examples of preferred reaction solvents include water, ammonia water, methanol, ethanol, tetrahydrofuran, 1,4-dioxane, N,N-dimethylformamide, a mixed solvent thereof and the like. The reaction temperature is not specifically limited. For example, 50°C to 120°C is preferred. The reaction time is preferably from 1 hour to 10 days. The compound represented by formula (I) that is obtained in this step can also be obtained in the form of its salt depending on the work-up procedure of this reaction.

Compound (IV) can also be produced based on the method shown in the following scheme 2 (step 3 to step 7). 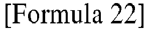 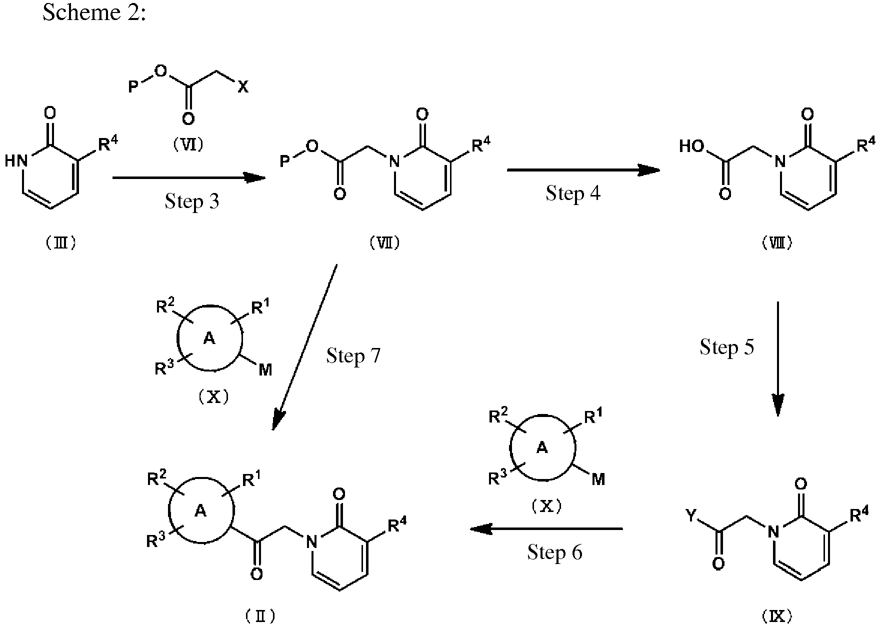 (wherein A, R¹, R², R³, and R⁴ are the same as defined above; X represents a chlorine atom, a bromine atom, or an iodine atom; Y represents an amine derivative group such as 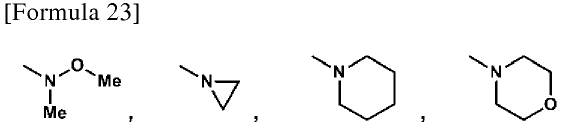 ; P represents a protecting group; and M represents MgBr, MgCl, Li, ZnBr, or ZnCl)

### <Step 3>

In step 3, a compound represented by formula (III) and a compound represented by formula (VI) are reacted in the presence of a base to produce a compound represented by formula (VII). Instead of the compound represented by formula (III), a compound represented by formula (V), 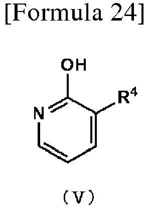 which is a tautomer of the compound represented by formula (III), may be used.. Examples of preferred bases include potassium carbonate, sodium carbonate, cesium carbonate, sodium hydride and the like. In addition, to promote this reaction, an additive may be added Examples of such additives include potassium iodide, sodium iodide, tetrabutylammonium iodide, potassium bromide, sodium bromide, tetrabutylammonium bromide and the like. The reaction solvent is not specifically limited as long as it does not significantly inhibit the reaction. Examples of preferred reaction solvents include N,N-dimethylformamide, dimethyl sulfoxide, acetone, acetonitrile, methanol, ethanol, tetrahydrofuran, 1,4-dioxane, 2-methoxyethanol, a mixed solvent thereof and the like. Although the reaction temperature is not specifically limited, for example, from room temperature to 60°C is preferred. The reaction time is preferably from 1 hour to 2 days.

### <Step 4>

In step 4, compound represented by formula (VII) is hydrolysed in an aqueous inorganic base to produce compound (VIII). Examples of preferred aqueous inorganic bases include aqueous sodium hydroxide, aqueous potassium hydroxide, aqueous lithium hydroxide and the like. The reaction solvent is not specifically limited as long as it does not significantly inhibit the reaction. Examples of preferred reaction solvents include water, methanol, ethanol, tetrahydrofuran, 1,4-dioxane, a mixed solvent thereof and the like. Although the reaction temperature is not specifically limited, for example, from room temperature to 60°C is preferred. The reaction time is preferably from 1 hour to 96 hours. The form of the compound represented by formula (VIII) that is obtained in this step is a carboxylic acid, a sodium carboxylate, a potassium carboxylate, a lithium carboxylate, a mixture of a carboxylic acid with an inorganic salt (sodium chloride, lithium chloride or potassium chloride) or the like.

### <Step 5>

In step 5, the compound (VIII) obtained in step 4 is converted to an activated carboxylic acid derivative, and then reacted with an amine or a salt thereof to produce a compound represented by formula (IX). Examples of the activated carboxylic acid derivative include an acid halide obtained by treating the compound (VIII) with thionyl chloride, phosphorus oxychloride, phosphorus pentachloride, oxalyl chloride, thionyl bromide or the like; an active ester obtained by condensation reaction of the compound (VIII) with a condensing agent such as 1-ethyl-3'-(3'-dimethylaminopropyl)carbodiimide hydrochloride or dicyclohexylcarbodiimide; and a mixed anhydride obtained by reacting the compound (VIII) with ethyl chlorocarbonate, pivaloyl chloride, isobutyl chlorocarboxylate or the like. Further, in this reaction, a base may be added as necessary. Examples of such base include an organic amine, such as triethylamine, tert-butylamine, pyridine, and N-methylmorpholine. Triethylamine, pyridine, or N-methylmorpholine is preferred. The reaction solvent is not specifically limited as long as it does not significantly inhibit the reaction. Examples of preferred reaction solvents include N,N-dimethylformamide, tetrahydrofuran, dichloromethane, chloroform and the like. Although the reaction temperature is not specifically limited, for example, from 0°C to 60°C is preferred. The reaction time is preferably from 1 hour to 96 hours.

### <Step 6>

In step 6, compound (IV) is produced by reacting the compound (IX) obtained in step 5 with a compound represented by formula (X). Examples of compound (X) include a lithium reagent prepared by, using a compound represented by formula (XI), 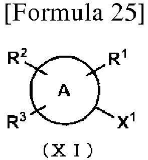 (wherein X¹ represents a chlorine atom, a bromine atom, or an iodine atom)
as a raw material, halogen-metal exchange using a base such as n-butyllithium, sec-butyllithium, and tert-butyl lithium; a Grignard reagent prepared by treatment using magnesium, isopropyl magnesium bromide, or isopropyl magnesium chloride; and a zinc reagent prepared by treatment using activated zinc, zinc bromide, zinc chloride; and the like. The reaction solvent is not specifically limited as long as it does not significantly inhibit the reaction. Examples of preferred reaction solvents include tetrahydrofuran, diethyl ether, 1,4-dioxane, dimethoxyethane and the like. Although the reaction temperature is not specifically limited, for example, from - 100°C to room temperature is preferred. The reaction time is preferably from 1 hour to 24 hours.

### <Step 7>

In step 7, compound (IV) is produced by reacting a compound represented by formula (VII) and a compound represented by formula (X) in the same manner as in step 6.

Compound (II) can also be produced based on the method shown in the following scheme 3 (step 8 to step 10). 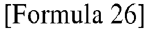 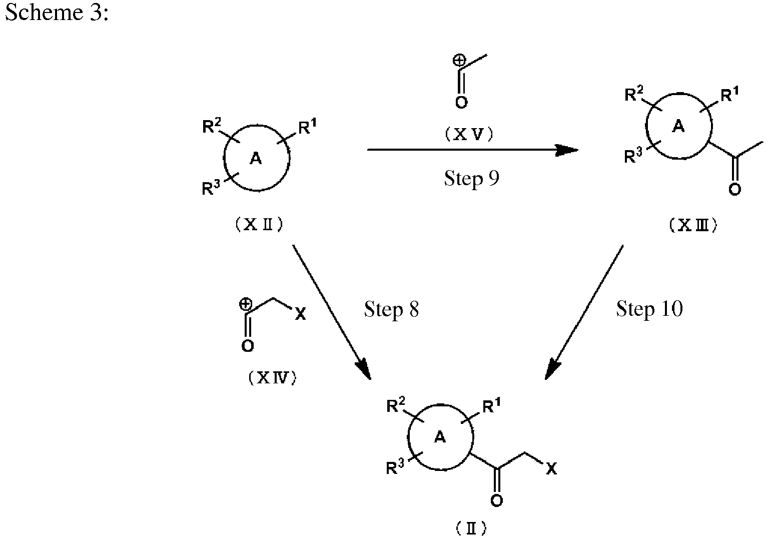 (wherein A, R¹, R², R³, and R⁴ are the same as defined above; and X represents a chlorine atom, a bromine atom, or an iodine atom)

### <Step 8>

In step 8, compound (II) is produced by reacting a compound represented by formula (XII) with an intermediate represented by formula (XIV). Examples of the intermediate (XIV) include an active intermediate obtained from an acid halide and a Lewis acid; an active intermediate obtained from an acid anhydride and a Lewis acid; and an active intermediate obtained from a carboxylic acid and a dehydrating agent. Examples of the acid halide include chloroacetyl chloride, chloroacetyl bromide, bromoacetyl bromide, bromoacetyl chloride, iodoacetyl chloride and the like. Examples of the acid anhydride include chloroacetic anhydride, bromoacetic anhydride, iodoacetic anhydride and the like. Examples of the carboxylic acid include chloroacetic acid, bromoacetic acid, and iodoacetic acid. Examples of the Lewis acid include aluminum chloride, zinc chloride and the like. Examples of the dehydrating agent include phosphorus pentoxide and the like. The reaction solvent is not specifically limited as long as it does not significantly inhibit the reaction. Examples of preferred reaction solvents include dichloromethane, dichloroethane and the like. Further, the reaction solvent may not be used. Although the reaction temperature is not specifically limited, for example, from 0°C to 100°C is preferred. The reaction time is preferably from 1 hour to 24 hours.

### <Step 9>

In step 9, compound (XIII) is produced by reacting a compound represented by formula (XII) with an intermediate represented by formula (XV). Examples of the intermediate (XV) include an active intermediate obtained from an acetyl halide and a Lewis acid; an active intermediate obtained from an acetic anhydride and a Lewis acid; and an active intermediate obtained from acetic acid and a dehydrating agent. Examples of the acetyl halide include acetyl chloride, acetyl bromide, and acetyl iodide. Examples of the Lewis acid include aluminum chloride, zinc chloride and the like. Examples of the dehydrating agent include phosphorus pentoxide and the like. The reaction solvent is not specifically limited as long as it does not significantly inhibit the reaction. Examples of preferred reaction solvents include dichloromethane, dichloroethane and the like. Further, the reaction solvent may not be used. Although the reaction temperature is not specifically limited, for example, from 0°C to 100°C is preferred. The reaction time is preferably from 1 hour to 24 hours.

### <Step 10>

In step 10, compound (II) is produced by reacting a compound represented by formula (XIII) with a halogenating agent. Examples of the halogenating agent include N-chlorosuccinimide, N-bromosuccinimide, N-iodosuccinimide, benzyltrimethylammonium tribromide and the like. This reaction can be accelerated by using a suitable acid. The reaction solvent is not specifically limited as long as it does not significantly inhibit the reaction. Examples of preferred reaction solvents include tetrahydrofuran, dichloromethane, dichloroethane and the like. Although the reaction temperature is not specifically limited, for example, from 0°C to 100°C is preferred. The reaction time is preferably from 1 hour to 72 hours.

The compound produced based on the above-described methods is isolated and purified as a free compound, a salt thereof, a hydrate thereof, a various solvate thereof such as ethanol solvate, polymorphilic crystalline products or the like. A pharmaceutically acceptable salt of the inventive compound can be prepared by a conventional salt-forming reaction. The isolation and purification can be carried out employing chemical operations such as fractional extraction, crystallization, and chromatography for fraction.

An optically-active substance can also be obtained as a stereochemically pure isomer by conventional optical resolution of the racemic compound based on the method shown in the following scheme 4 (step 11). 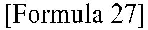 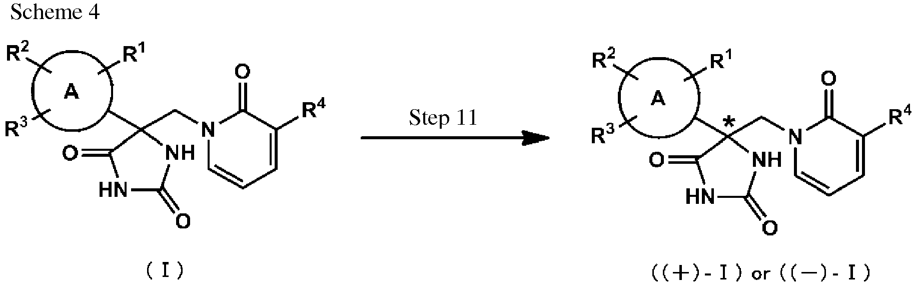 (wherein A, R¹, R², R³, and R⁴ are the same as defined above; and an asterisk (*) indicates an optically pure form)

### <Step 11>

In step 11, the compound I) produced in racemic form is optically resolved using a chiral column into the (+) or (-) isomer. The optical resolution using a chiral column may be carried out based on a method known to the person skilled in the art (e.g., "Resolution of Optical Isomers" (see Kikan Kagaku Sosetsu (Quarterly Chemistry Review) No. 6, 1989, ed. Chemical Society of Japan, Gakkai Shuppan Center) etc.). Further, as the chiral column to be used, any of various commercially-available products may be selected as appropriate.

The composition according to the present invention is a composition for maintaining a function of platelets, the composition including, as an active ingredient, the above-described compound or a salt thereof (hereinafter also referred to as "the compound represented by formula (I) or the like"). In the present invention, the phrase "maintaining a function of platelets" mainly means essentially maintaining a function related to blood coagulation such as hemostasis and thrombus formation by platelets. The composition according to the present invention is capable of achieving such a function by suppressing ADAM 17-mediated GPIbα shedding (release by cleavage).

"Maintaining a function of platelets" can be evaluated, for example, by employing a method in which the adhesion ability of platelets to VWF (von Willebrand factor) is evaluated using a flow chamber system as described in WO2009/119105 and WO2012/036257, a method in which the in vivo lifespan of platelets is evaluated using thrombocytopenic mouse, a method in which the shape of platelets is observed on a fibrinogen-coated cover glass in the presence of thrombin, a biomolecular imaging technique in which a thrombus formation model animal is prepared using laser irradiation together with hematoporphyrin and the like to observe the kinetics of the platelets at an individual level in the model animal, or the like.

ADAM 17 (a disintegrin and metallopeptidase domain 17) is a protein having a structure containing a disintegrin domain and that has a metalloproteinase activity. ADAM 17 is also called TACE (TNF-alpha converting enzyme) because ADAM 17 sheds, other than GPIbα (glycoprotein Ib alpha), membrane-bound TNF-α (tumor necrosis factor-alpha) to produce soluble TNF-α. Typically, human-derived ADAM17 is a protein (gene) specified under ACCESSION No. NP_003174.3 (No. NM_003183.4).

Further, GPIbα is a membrane protein of platelets, and functions as a receptor for VWF. In addition, since GPIbα is expressed only on platelets in a human body, GPIbα is also called CD42b antigen and used as a surface marker for platelets. Typically, human-derived GPIbα is a protein (gene) specified under ACCESSION No. NP_000164.5 (No. NM_000173.5).

Although various reference sequences registered in GenBank are exemplified as typical examples of ADAM17 and GPIbα, the amino acid sequences of the proteins may be mutated naturally (i.e., non-artificially). Thus, it should be understood that ADAM17 and GPIbα involved in the action mechanism of the composition according to the present invention include such naturally-occurring mutants.

In the present invention, the phrase "suppressing ADAM 17-mediated GPIbα shedding" includes both complete suppression (inhibition) and partial suppression of shedding by ADAM17. Further, as described in Test Example 3 below, the composition according to the present invention may be evaluated as suppressing ADAM17-mediated GPIbα shedding when the percentage of the number of CD42b (GPIbα) (+) platelets to the total number of platelets in the presence of the composition according to the present invention is calculated to be a large value in comparison with the percentage obtained in the absence of the composition according to the present invention. As the degree of GPIbα shedding suppressed by the composition according to the present invention, a higher percentage of the number of CD42b (+) platelets in the presence of the composition according to the present invention is preferred. This percentage is preferably 60% or higher, more preferably 70% or higher, and even more preferably 80% or higher.

Note that when the ability of the composition according to the present invention to suppress GPIbα shedding is evaluated using the method described below in Test Example 1, it is preferred that the 50% inhibitory concentration (IC₅₀) is less than 1,000 nmol/L, more preferred is less than 500 nmol/L, and even more preferred is less than 100 nmol/L.

Examples of the compound represented by formula (I) as an active ingredient of the composition according to the present invention include, but are not especially limited to: 5-[(3-methyl-2-oxopyridin-1(2H)-yl)methyl]-5-phenylimidazolidine-2,4-dione, 5-[(3-methyl-2-oxopyridin-1(2H)-yl)methyl]-5-(4-phenoxyphenyl)imidazolidine-2,4-dione, 5-(3,4-difluorophenyl)-5-[(3-methyl-2-oxopyridin-1(2H)-yl)methyl]imidazolidine-2,4-dione, 5-(4-benzylphenyl)-5-[(3-methyl-2-oxopyridin-1(2H)-yl)methyl]imidazolidine-2,4-dione, (+)-5-(3,4-difluorophenyl)-5-[(3-methyl-2-oxopyridin-1(2H)-yl)methyl]imidazolidine-2,4-dione, and the like.

Among those compounds, (+)-5-(3,4-difluorophenyl)-5-[(3-methyl-2-oxopyridin-1(2H)-yl)methyl]imidazolidine-2,4-dione is the " (+)-I-10" or "S-108470" in the working examples and test examples described below.

Examples of the form of the composition according to the present invention include a medicine additive and a reagent to be used for the purpose of research and development (e.g., in vitro and in vivo research and development).

The composition according to the present invention has an action of maintaining a function of platelets by suppressing ADAM17-mediated GPIbα shedding. Accordingly, the composition of the present invention can be suitably used as a reagent for maintaining a function of platelets (e.g., a reagent used in a culture system for differentiating megakaryocytes from cells capable of differentiating into megakaryocytes and then producing platelets from the megakaryocytes, or a reagent for maintaining a function of produced platelets by adding the regent) or as a medicine additive added to a blood product containing platelets for transfusion. When used as the above-described additive or reagents, the composition according to the present invention may include, in addition to the compound represented by formula (I) or the like as the active ingredient, added components, such as a stabilizer, a solvent, and the like, for example. Further, based on the intended purpose, the composition according to the present invention may be the compound represented by formula (I) per se.

A product (e.g., reagent, medicine additive) of the composition according to the present invention or a protocol thereof may be labeled in order to indicate that the product (protocol thereof) is to be used to maintain a function of platelets. Herein, the phrase "a product or a protocol is labeled" means that the body of the product, a container or a package therefor, or the like, is labeled, or that a protocol, an attached document, an advertisement, other printed matter, or the like, disclosing information on the product is labeled. The label indicating that the product or protocol thereof is to be used to maintain a function of platelets may include information on a mechanism of how the compound represented by formula (I) or the like as the active ingredient of the composition according to the present invention demonstrates an effect of maintaining a function of platelets. An example of the information on the mechanism includes information that a function of platelets is maintained by suppressing ADAM17-mediated GPIbα shedding. Moreover, the label indicating that the product or protocol thereof is to be used to maintain a function of platelets may include information that the product or protocol thereof is to be used to produce or store platelets or for other purposes.

The compound represented by formula (I) or the like acts as an active ingredient for producing the composition according to the present invention. Thus, the present invention also provides the compound represented by the above-described formula (I) or the like, the compound being used to produce the composition according to the present invention, and a method for producing the composition according to the present invention, the method including using the compound represented by formula (I) or the like.

Further, the present invention provides a culture obtained by adding the compound represented by formula (I) or the like to a culture system for differentiating megakaryocytes from cells capable of differentiating into megakaryocytes and for producing platelets from the megakaryocytes. In addition, the present invention provides a method for preparing platelets, the method including adding the compound represented by formula (I) or the like to a culture system for differentiating megakaryocytes from cells capable of differentiating into megakaryocytes and producing platelets from the megakaryocytes.

The term "megakaryocytes", which are also called platelet progenitor cells or megakaryocytic cells, as used in the present invention refers to cell that produce platelets by dividing the cytoplasm.

In the present invention, the "cells capable of differentiating into megakaryocytes" are not particularly limited, as long as the cells are capable of differentiating into megakaryocytes. Examples thereof include fertilized eggs, pluripotent stem cells such as embryonic stem cells (ES cells), induced pluripotent stem cells (iPS cells), and embryonic germ cells (EG cells), hematopoietic stem cells, hematopoietic progenitor cells, megakaryoblasts, and adipocytes. The hematopoietic stem cells and the hematopoietic progenitor cells may be collected from bone marrow, umbilical cord blood, or the like. Further, among these, iPS cells are preferable from the viewpoint that such cells do not involve any ethical problems because an embryo is not destroyed, and the viewpoint that it is easy to match the human leukocyte antigen (HLA) type of a patient transfused with platelets prepared according to the present invention.

Further, the animal species from which the above-described cells to be used in the method according to the present invention are derived is not particularly limited. Examples thereof include human, mice, rats, dogs, cats, cattle, horses, sheep, and the like. Preferred are mice, rats, and human, more preferred are mice and humans, and particularly preferred is human.

Examples of the "culture system for differentiating megakaryocytes from cells capable of differentiating into megakaryocytes and producing platelets from the megakaryocytes" in the present invention include a culture system for forming embryoid bodies (cell population containing differentiation-induced, undifferentiated mesodermal cells) in the middle of platelet production (Eto et al., Proc. Natl. Acad. Sci. USA, 2002, vol. 99, pp. 12819-12824 , etc.), a culture system for forming a net-like structure (sac structure) enclosing hematopoietic progenitor cells (see Patent Literature 1 to 3 etc.), a culture system for producing platelets from umbilical cord blood-derived hematopoietic progenitor cells (see Robert et al., "Glycoprotein Ibalpha receptor instability is associated with loss of quality of platelets produced in culture. ", Stem Cells and Development, published online on May 26, 2010, etc.), and the like (other examples include Fujimoto et al., Blood, 2003, vol. 102, pp. 4044-4051 : Hiroyama et al., Exp. Hematol., 2006, vol. 34, pp. 760-769 : Gaur et al. , J Thromb Haemost., 2005, vol. 4, pp. 436-442, etc.). Among these, a culture system for forming a net-like structure is preferable because hematopoietic progenitor cells are condensed in the net-like structure, enabling efficient ex vivo production of megakaryocytes, platelets, and the like.

The culture conditions of the culture system according to the present invention are not particularly limited, as long as the conditions are suitable for megakaryocytes culturing and platelet production.

Further, when the "cells capable of differentiating into megakaryocytes" according to the present invention are cultured (particularly, in the process of inducing differentiation to megakaryocytes), the cells are preferably co-cultured with feeder cells. The "feeder cells" used here are not particularly limited, as long as the feeder cells contribute to the differentiation induction of the "cells capable of differentiating into megakaryocytes." For example, it is possible to use mouse embryonic fibroblasts, preferably a 10T1/2 cell line, OP9 cells, and the like. An example of cells other than immortalized cell lines includes human bone marrow-derived mesenchymal stem cells (cultured cells directly prepared from human bone marrow). Even when mesenchymal stem cells are used as the feeder cells, megakaryocyte maturation and platelet production from human ES cells or iPS cells are possible. Platelets can be induced by culturing on an extracellular substrate such as Matrigel as well. When "feeder cells" are used, it is preferable to suppress the growth of cells through radiation exposure or the like.

The amount (concentration) of the compound represented by formula (I) or the like to be added to the culture system is preferably from 0.01 to 100 µM. If the added amount is equal to or more than the lower limit, cleavage of the GPIbα in the platelets can be preferably suppressed. If the added amount is equal to or less than the upper limit, suppression of cell growth due to the effects of the compound can be preferably avoided.

Hereinafter, the method for preparing platelets according to the present invention is described in more detail by using an example of a method for forming a net-like structure that can be suitably used for the culture system of the present invention.

First, the method for forming the net-like structure (sac structure) is described. The culture conditions suitable for preparing the net-like structure depend on ES cells or iPS cells used. However, for example, Iscove's Modified Dulbecco's Medium (IMDM) supplemented with FBS to a final concentration of 15% may be used as a medium. Even in a case of a serum-free medium, a medium to which a growth factor, a supplement, or the like has been added as appropriate can also be used. Further, in order to form the net-like structure efficiently, a vascular endothelial growth factor (VEGF) should be added to a level of approximately from 0 to 300 ng/ml, and more preferably approximately 20 ng/ml. The culture environment depends on the type of ES cells or iPS cells used. However, the conditions are preferably 5% CO₂ at 35 to 38°C, more preferably 36 to 38°C, and even more preferably 37°C. The culturing period until a net-like structure is formed depends on the type of ES cells or iPS cells, but hematogenic precursor cells can be efficiently obtained at approximately Day 15 (about 14 to 16 days later) after seeding on feeder cells.

The formed net-like structure has a follicular structure. In the structure, hematopoietic progenitor cells, particularly CD34-positive cells, are present in a concentrated state. The hematopoietic progenitor cells present inside the net-like structure can be separated by physical means, for example, by passing the cells through a sterilized sieve-like tool (e.g., cell strainer or the like).

Next, the method for preparing the platelets from the hematopoietic progenitor cells separated from the net-like structure is described. The hematopoietic progenitor cells obtained by separation are seeded on feeder cells, and cultured under conditions suitable for producing megakaryocytes and platelets. Herein, examples of the "conditions suitable for producing megakaryocytes and platelets" include culturing for approximately 7 to 15 days in the presence of thrombopoietin (TPO, approximately 10 to 200 ng/mL, preferably 100 ng/mL), or in the presence of a stem cell factor (SCF, approximately 10 to 200 ng/mL, preferably 50 ng/mL), heparin (approximately 10 to 100 U/mL, preferably 25 U/ml), and TPO (approximately 10 to 200 ng/mL, preferably 100 ng/mL). As the culture environment, the conditions are preferably 5% CO₂ at 35 to 38°C, more preferably 37°C.

Further, in the above-described culture system, CD42b (GPIbα) was expressed over time. Although the expressed amount increased, during this process the expressed amount was uneven among the cells. Therefore, in order to more effectively suppress CD42b (GPIbα) shedding, it is preferred that the compound represented by formula (I) or the like be added to the above-described culture system when reseeding the hematogenic precursor cells on the feeder cells.

In addition, in the method according to the present invention, platelets can be prepared by collecting a culture solution fraction (e.g., in the method for forming a net-like structure, the fraction at approximately Days 22 to 28 after human iPS cells or ES cells are cultured) in which platelets released from megakaryocytes are abundant, and then removing components other than the platelets (i.e., megakaryocytes and other blood cells) using a leukocyte reduction filter (available from, for example, Terumo Corporation, Asahi Kasei Medical Co., Ltd., etc.) and the like.

As described above, the compound represented by formula (I) or the like suppresses ADAM 17-mediated GPIbα shedding and has an action of maintaining a function of platelets. Thus, the present invention also provides a platelet-containing blood product to which the compound represented by formula (I) or the like has been added, and, a method for maintaining a function of platelets in a blood product, the method including adding, to a platelet-containing blood product, the compound represented by formula (I) or the like.

Further, the compound represented by formula (I) or the like serves as an active ingredient for producing a platelet-containing blood product. Thus, the present invention also provides the compound represented by formula (I) or the like as a medicine additive used to produce the blood product according to the present invention.

The platelets contained in the blood product according to the present invention are not particularly limited, and may be platelets obtained by the above-described method for preparing platelets according to the present invention or may be platelets derived from peripheral blood or the like obtained by collecting blood. In the preparation of the blood product, in view of the storage instability of platelets and the like, other ingredients for stabilizing platelets may also be added. The conditions for stabilizing the platelets can be selected from among methods well known to the person with ordinary skill in the art. For example, the product can be prepared by suspending the platelets in a solution required to maintain a function of platelets (e.g., an ACD-A solution (a solution prepared from sodium citrate/citric acid/glucose) and the like; in some cases, frozen plasma or the like may be added as appropriate) at an appropriate concentration (for example, approximately 1×10⁸ to 1×10¹⁰ platelets/mL, preferably approximately 1×10⁹ platelets/mL). Note that, as a container for storing the platelet-containing product, it is preferable to avoid using a material that activates platelets, such as glass. The added amount (concentration) of the compound represented by formula (I) or the like is preferably from 0.01 to 100 µmol/L. If the added amount is equal to or more than the lower limit, the adhesion function of the platelets can be preferably maintained. If the added amount is equal to or less than the upper limit, safety during transfusion can be increased.

### Examples

The present invention is now described in more detail based on the following working examples and test examples. However, the present invention is not limited to the following working examples.

### Method for Producing Test Compounds

Representative production examples of the compound represented by formula (I), which is the active ingredient of the composition according to the present invention, are now illustrated as examples. In the following examples, the materials, its usage amounts and ratios, handling, procedure or the like may be suitably modified as long as such modifications do not go beyond the intent of the invention. Therefore, the scope of the present invention should not be construed as being limited by the specific examples illustrated below.

The ¹H-NMR spectra shown below were measured with a JNM-ECA 400 spectrometer (400 MHz, manufactured by JEOL, Ltd.) using deuterated chloroform (CDCl₃) or deuterated dimethyl sulfoxide (DMSO-d₆) as a solvent and tetramethylsilane (TMS) as an internal standard. In the chemical shift measurement results, the δ value is represented in ppm, and the coupling constant J value is represented in Hz. The abbreviation s stands for singlet, d for doublet, t for triplet, q for quartet, m for multiplet, and br for broad. For the mass spectrum (electrospray ionization: ESI-MS) measurement, Exactive manufactured by Thermo Fisher Scientific was employed. For the specific optical rotation measurement, a polarimeter (SEPA-300, manufactured by Horiba, Ltd., was employed.

### Working Example 1

### Production of 5-[(3-methyl-2-oxopyridin-1(2H)-yl)methyl]-5-phenyl-imidazolidine-2,4-dione (I-1)

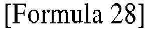

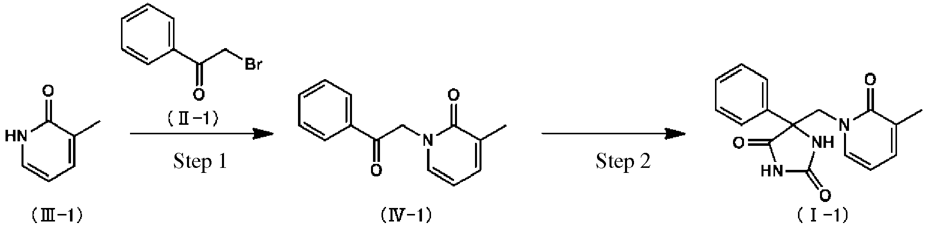

### Step 1

Sodium hydride (60%, 1.8 g, 45.8 mmol) was added to a solution of 3-methyl-2-pyridone (III-1) (5.0 g, 45.8 mmol) in N,N-dimethylformamide (91 mL), and the resultant mixture was stirred for 10 minutes at room temperature. Phenacyl bromide (II-1) (9.1 g, 45.8 mmol) was added to the reaction solution, and the resultant mixture was stirred for 1.5 hours at room temperature. After gradually adding water to the reaction solution, the mixture was extracted with ethyl acetate. The organic layer was washed with water, and then dried over anhydrous sodium sulfate. The solvent was removed under reduced pressure to obtain compound (IV-1) (500 mg, 2.2 mmol) as a yellow solid.

### Step 2

A suspension of this compound (IV-1) (7.0 g, 30.8 mmol), potassium cyanide (2.7 g, 41.5 mmol), and ammonium carbonate (11.8 g, 123 mmol) in ethanol (30 mL) and water (30 mL) was sealed, and stirred for 89 hours at 90°C. After leaving to cool, the reaction solution was diluted with water, and the resultant mixture was extracted with ethyl acetate. The organic layer was successively washed with water and saturated brine, and then dried over sodium sulfate. The solvent was removed under reduced pressure, and then chloroform was added. The mixture was filtered, and the resultant product was washed with chloroform and dried to obtain compound (I-1) (amount 1.14 g, yield 26%) as a yellow solid. The physical properties are shown below. ¹H-NMR (CDCl₃) δ: 2.12 (3H, s), 4.28 (1H, d, J=13.7 Hz), 4.85 (1H, d, J=13.7 Hz), 6.08 (1H, t, J=6.8 Hz), 7.14 (1H, dd, J=1.4, 6.8 Hz), 7.21 (1H, dd, J=1.4, 6.8 Hz), 7.37-7.44 (3H, m), 7.63-7.66 (2H, m), 8.35 (1H, s).
MS (ESI-FTMS) m/z : 298 [M+H]⁺.

### Working Example 2

### Production of 5-(4-methoxyphenyl)-5-[(3-methyl-2-oxopyridin-1(2H)-yl)methyl]imidazolidine-2,4-dione (I-2)

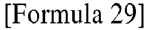

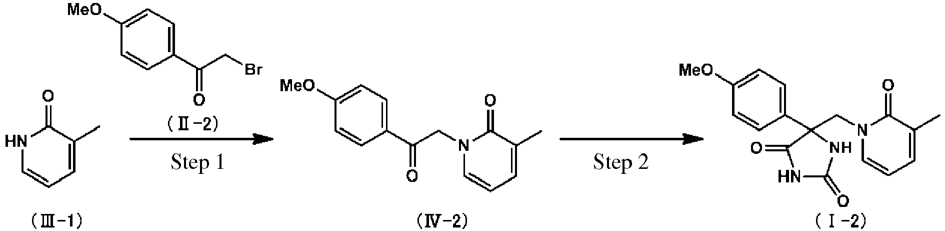

### Step 1

Cesium carbonate (745 mg, 2.3 mmol) and 4'-methoxyphenacyl bromide (II-2) (500 mg, 2.2 mmol) were added to a solution of compound (III-1) (227 mg, 2.1 mmol) in N,N-dimethylformamide (10 mL), and the resultant mixture was stirred for 3.5 hours at room temperature. Water was added under ice cooling to stop the reaction, and then the mixture was extracted with ethyl acetate. The organic layer was washed with brine, and then dried over magnesium sulfate. The solvent was removed under reduced pressure to obtain compound (IV-2) (amount 440 mg, yield 82%) as a light yellow solid.

### Step 2

Water (1.5 mL) was added to a suspension of this compound (IV-2) (436 mg, 1.7 mmol), potassium cyanide (132 mg, 2.0 mmol), and ammonium carbonate (651 mg, 6.8 mmol) in ethanol (1.5 mL). The resultant mixture was sealed, and stirred for 45 hours at 100°C. After leaving to cool, the reaction solution was diluted with water, and the resultant mixture was extracted with ethyl acetate. The organic layer was washed with saturated brine, and then dried over sodium sulfate. The solvent was removed under reduced pressure to obtain compound (I-2) (amount 370 mg, yield 67%) as a pale yellow solid. The physical properties are shown below. ¹H-NMR (DMSO-d₆) δ: 2.00 (3H, s), 3.76 (3H, s), 4.44 (1H, d, J=13.7 Hz), 4.55 (1H, d, J=13.7 Hz), 6.11 (1H, t, J=6.9 Hz), 6.98 (2H, td, J=2.5, 9.2 Hz), 7.20-7.31 (2H, m), 7.53 (2H, td, J=2.5, 9.2 Hz), 8.42 (1H, s), 10.80 (1H, s).
MS (ESI-FTMS) m/z 328 [M+H]⁺.

### Working Example 3

### Production of 4-{4-[(3-methyl-2-oxopyridin-1(2H)-yl)methyl]-2,5-dioxoimidazolidine-4-yl}benzonitrile (I-3)

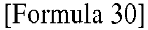

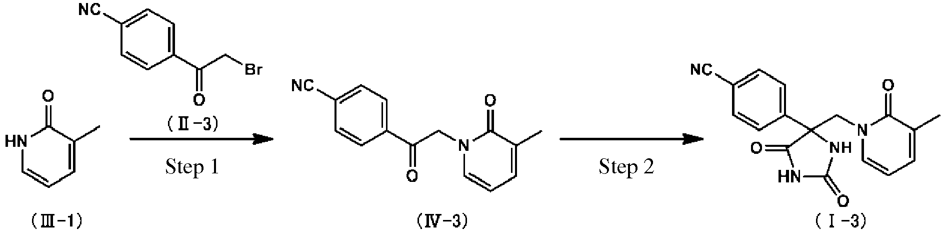

### Step 1

Cesium carbonate (762 mg, 2.4 mmol) and 4'-cyanophenacyl bromide (II-3) (500 mg, 2.2 mmol) were added to a solution of compound (III-1) (232 mg, 2.1 mmol) in N,N-dimethylformamide (10 mL), and the resultant mixture was stirred for 3 hours at room temperature. Water was added under ice cooling to stop the reaction, and then the mixture was extracted with ethyl acetate. The organic layer was washed with saturated brine, and then dried over magnesium sulfate. The solvent was removed under reduced pressure, and the resultant product was purified by column chromatography (silica gel) to obtain compound (IV-3) (amount 440 mg, yield 82%) as a light yellow solid.

### Step 2

Compound (I-3) (amount 35 mg, yield 10%) was obtained as a pale yellow solid from compound (IV-3) based on the same production method as for compound (I-2). The physical properties are shown below.
¹H-NMR (DMSO-d₆) δ: 1.98 (3H, s), 4.47 (1H, d, J=13.7 Hz), 4.67 (1H, d, J=13.7 Hz), 6.13 (1H, t, J=6.7 Hz), 7.25-7.31 (2H, m), 7.80-7.86 (2H, m), 7.90-7.97 (2H, m), 8.74 (1H, s), 11.01 (1H, s). MS (ESI-FTMS) m/z 323 [M+H]⁺.

### Working Example 4

### Production of 5-(3-methoxyphenyl)-5-[(3-methyl-2-oxopyridin-1(2H)-yl)methyl]imidazolidine-2,4-dione (I-4)

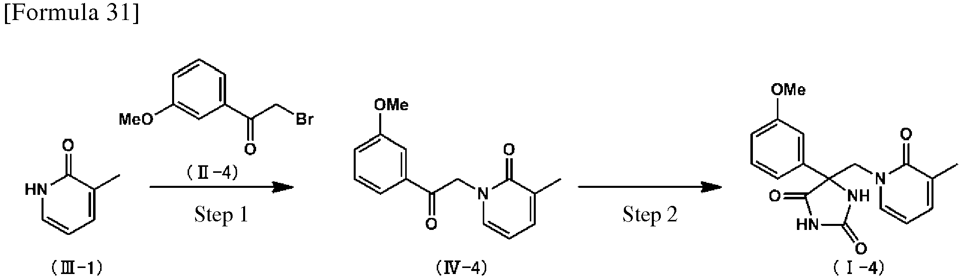

Compound (IV-4) (amount 496 mg, yield 93%) was obtained as a yellow oily substance from compound (III-1) and compound (II-4) based on the same production method as for compound (IV-3).

Compound (I-4) (amount 425 mg, yield 67%) was obtained as a pale yellow solid from compound (IV-4) based on the same production method as for compound (I-2). The physical properties are shown below.
¹H-NMR (DMSO-d₆) δ: 2.00 (3H, s), 3.78 (3H, s), 4.47 (1H, d, J=13.7 Hz), 4.59 (1H, d, J=13.7 Hz), 6.11 (1H, t, J=6.9 Hz), 6.95 (1H, ddd, J=0.9, 2.3, 8.2 Hz), 7.18-7.26 (3H, m), 7.29 (1H, m), 7.35 (1H, t, J=8.0 Hz), 8.58 (1H, s), 10.85 (1H, s).
MS (ESI-FTMS) m/z 328 [M+H]⁺.

### Working Example 5

### Production of 5-[(3-methyl-2-oxopyridin-1(2H)-yl)methyl]-5-(thiophen-3-yl)imidazolidine-2,4-dione (I-5)

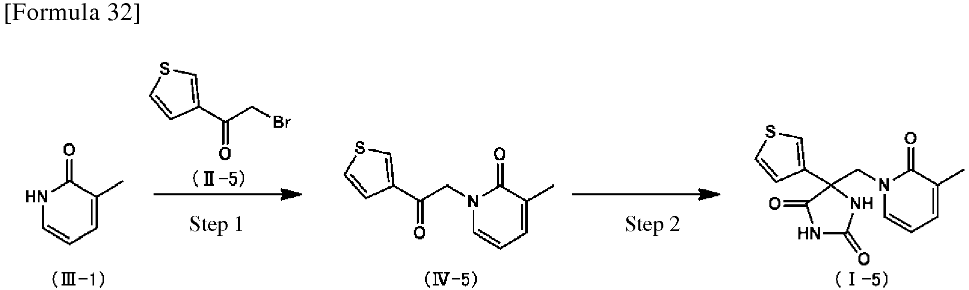

### Step 1

Sodium hydroxide (60%, 102 mg, 2.6 mmol) was added to a solution of compound (III-1) (253 mg, 2.3 mmol) in N,N-dimethylformamide (10 mL). Then, 3-(bromoacetyl)thiophene (II-5) (500 mg, 2.4 mmol) was added, and the resultant mixture was stirred for 16 hours at room temperature. Water was added under ice cooling to stop the reaction, and then the mixture was extracted with ethyl acetate. The organic layer was washed with saturated brine, and then dried over magnesium sulfate. The solvent was removed under reduced pressure to obtain compound (IV-5) (amount 371 mg, yield 69%).

### Step 2

Compound (I-5) (amount 270 mg, yield 56%) was obtained as a pale yellow solid from compound (IV-5) based on the same production method as for compound (I-2). The physical properties are shown below.
¹H-NMR (DMSO-d₆) δ: 2.00 (3H, s), 4.48 (1H, d, J=13.7 Hz), 4.53 (1H, d, J=13.7 Hz), 6.10 (1H, t, J=6.9 Hz), 7.21 (1H, dd, J=1.4, 6.9 Hz), 7.25-7.32 (2H, m), 7.59-7.64 (2H, m), 8.58 (1H, s), 10.87 (1H, s).
MS (ESI-FTMS) m/z 304 [M+H]⁺.

### Working Example 6

### Production of 5-(benzofuran-2-yl)-5-[(3-methyl-2-oxopyridin-1(2H)-yl)methyl]imidazolidine-2,4-dione (I-6)

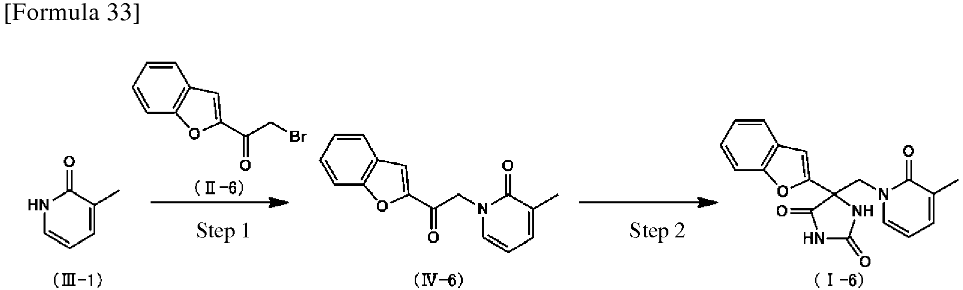

### Step 1

Sodium hydroxide (60%, 88 mg, 2.2 mmol) was added to a solution of compound (III-1) (217 mg, 2.0 mmol) in N,N-dimethylformamide (10 mL). Then, 2-(2-bromoacetyl)benzofuran (II-6) (500 mg, 2.1 mmol) was added, and the resultant mixture was stirred for 16 hours at room temperature. Water was added under ice cooling to stop the reaction, and then the mixture was extracted with ethyl acetate. The organic layer was washed with saturated brine, and then dried over magnesium sulfate. The solvent was removed under reduced pressure, and then the resultant product was purified by column chromatography (silica gel) to obtain compound (IV-6) (amount 115 mg, yield 22%).

### Step 2

Compound (I-6) (amount 58 mg, yield 40%) was obtained as a pale yellow solid from compound (IV-6) based on the same production method as for compound (I-2). The physical properties are shown below.
¹H-NMR (DMSO-d₆) δ: 1.99 (3H, s), 4.71 (1H, d, J=13.7 Hz), 4.79 (1H, d, J=13.7 Hz), 6.14 (1H, t, J=6.9 Hz), 7.14 (1H, d, J=0.9 Hz), 7.26-7.33 (3H, m), 7.36 (1H, dt, J=1.4, 7.3 Hz), 7.62 (1H, d, J=8.2 Hz), 7.68 (1H, d, J=7.8 Hz), 8.65 (1H, s), 11.10 (1H, s).
MS (ESI-FTMS) m/z 338 [M+H]⁺.

### Working Example 7

### Production of 5-[(3-methyl-2-oxopyridin-1(2H)-yl)methyl]-5-(pyridin-2-yl)imidazolidine-2,4-dione (I-7)

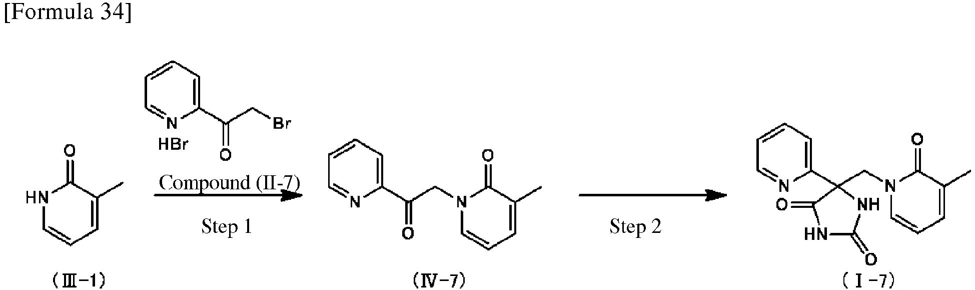

### Step 1

Sodium hydroxide (60%, 339 mg, 8.5 mmol) was added to a solution of compound (III-1) (370 mg, 3.4 mmol) in N,N-dimethylformamide (10 mL). Then, 2-(bromoacetyl)pyridine hydrogen bromide (II-7) (1.0 g, 3.6 mmol) was added, and the resultant mixture was stirred for 4.5 hours at room temperature. Water was added under ice cooling to stop the reaction, and then the mixture was extracted with ethyl acetate. The organic layer was washed with saturated brine, and then dried over magnesium sulfate. The solvent was removed under reduced pressure, and then the resultant product was purified by column chromatography (silica gel) to obtain compound (IV-7) (amount 70 mg, yield 9.1%).

### Step 2

Water (0.3 mL) was added to a suspension of this compound (IV-7) (70 mg, 0.31 mmol), potassium cyanide (24 mg, 0.37 mmol), and ammonium carbonate (118 mg, 1.22 mmol) in ethanol (0.3 mL). The resultant mixture was sealed, and stirred for 65 hours at 100°C. After leaving to cool, the solvent was removed under reduced pressure, methanol was added, and the precipitated solid was removed by filtration. The filtrate solvent was removed under reduced pressure to obtain compound (I-7) (amount 35 mg, yield 38%) as a colorless solid. The physical properties are shown below.
¹H-NMR (DMSO-d₆) δ: 1.99 (3H, s), 4.73 (1H, d, J=13.7 Hz), 4.83 (1H, d, J=13.7 Hz), 6.11 (1H, t, J=6.9 Hz), 7.25-7.34 (2H, m), 7.43 (1H, ddd, J=0.9, 5.0, 7.8 Hz), 7.53 (1H, d, J=7.8 Hz), 7.90 (1H, dt, J=1.8, 7.8 Hz), 8.40 (1H, s), 8.65 (1H, m), 10.88 (1H, s).
MS (ESI-FTMS) m/z 299 [M+H]⁺.

### Working Example 8

### Production of 5-[(3-methyl-2-oxopyridin-1(2H)-yl)methyl]-5-(pyridin-3-yl)imidazolidine-2,4-dione (I-8)

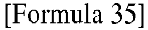

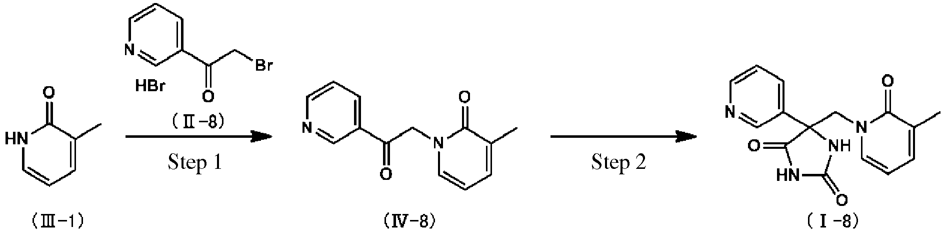

### Step 1

Compound (IV-8) (amount 469 mg, yield 61%) was obtained from compound (III-1) and a 3-(bromoacetyl)pyridine hydrogen bromide (II-8) based on the same production method as for compound (IV-7).

### Step 2

Water (1 mL) was added to a suspension of this compound (IV-8) (469 mg, 2.1 mmol), potassium cyanide (160 mg, 2.5 mmol), and ammonium carbonate (789 mg, 8.2 mmol) in ethanol (1 mL). The resultant mixture was sealed, and stirred for 65 hours at 100°C. After leaving to cool, the solvent was removed under reduced pressure, methanol was added, and the precipitated solid was removed by filtration. The filtrate solvent was removed under reduced pressure, the resultant product was dissolved in a small amount of chloroform. Hexane was added, the precipitated solid was filtered, and the filtered solid was washed with chloroform to obtain compound (I-8) (amount 295 mg, yield 48%) as a colorless solid. The physical properties are shown below.
¹H-NMR (DMSO-d₆) δ: 1.98 (3H, s), 4.52 (1H, d, J=13.7 Hz), 4.66 (1H, d, J=13.7 Hz), 6.13 (1H, t, J=6.9 Hz), 7.29 (2H, dd, J=0.9, 6.4 Hz), 7.46 (1H, m), 8.00 (1H, m), 8.58 (1H, dd, J=1.6, 4.8 Hz), 8.81 (1H, s), 8.82 (1H, d, J=1.8 Hz), 11.07 (1H, s).
MS (ESI-FTMS) m/z 299 [M+H]⁺.

### Working Example 9

### Production of 5-[(3-methyl-2-oxopyridin-1(2H)-yl)methyl]-5-(4-phenoxyphenyl)imidazolidine-2,4-dione (I-9)

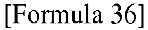

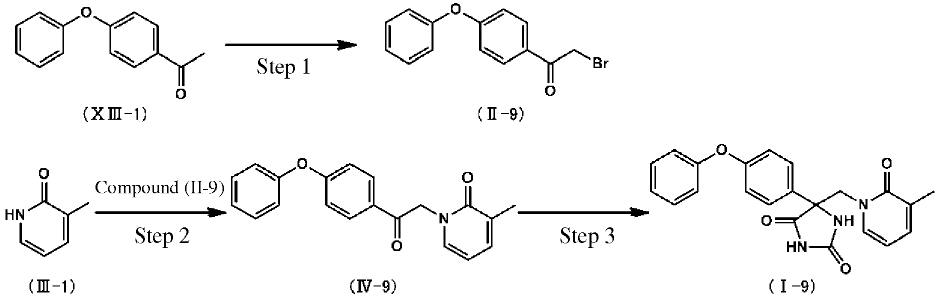

### Step 1

Phenyltrimethylammonium tribromide (1.8 g, 4.7 mmol) was added to a solution of 4'-phenoxyacetophenone (XIII-1) (1.0 g, 4.7 mmol) in tetrahydrofuran (5 mL), and the resultant mixture was stirred for 14 hours at room temperature, then heated under reflux for 8 hours. After leaving to cool, the reaction solution was diluted with water, and the resultant mixture was extracted with ethyl acetate. The organic layer was washed with saturated brine, and then dried over magnesium sulfate. The solvent was removed under reduced pressure, and then the resultant product was purified by column chromatography (silica gel) to obtain compound (II-9) (amount 1.0 g, yield 69%).

### Step 2

Compound (IV-9) (amount 218 mg, yield 42%) was obtained from compound (III-1) and compound (II-9) based on the same production method as for compound (IV-3).

### Step 3

Water (0.7 mL) was added to a suspension of this compound (IV-9) (218 mg, 0.68 mmol), potassium cyanide (53 mg, 0.82 mmol), and ammonium carbonate (262 mg, 2.73 mmol) in ethanol (0.7 mL). The resultant mixture was sealed, and stirred for 66 hours at 100°C. After leaving to cool, the reaction solution was diluted with water. The precipitated solid was collected by filtration, washed with water, and then purified by column chromatography (silica gel) to obtain compound (I-9) (amount 160 mg, yield 58%) as a colorless solid. The physical properties are shown below.
¹H-NMR (DMSO-d₆) δ: 2.00 (3H, s), 4.46 (1H, d, J=13.7 Hz), 4.61 (1H, d, J=13.7 Hz), 6.12 (1H, t, J=6.6 Hz), 6.99-7.09 (4H, m), 7.17 (1H, m), 7.23-7.32 (2H, m), 7.37-7.44 (2H, m), 7.63 (2H, td, J=2.5, 9.2 Hz), 8.60 (1H, s), 10.87 (1H, s).
MS (ESI-FTMS) m/z 390 [M+H]⁺.

### Working Example 10

### Production of 5-(3,4-difluorophenyl)-5-[(3-methyl-2-oxopyridin-1(2H)-yl)methyl]imidazolidine-2,4-dione (I-10)

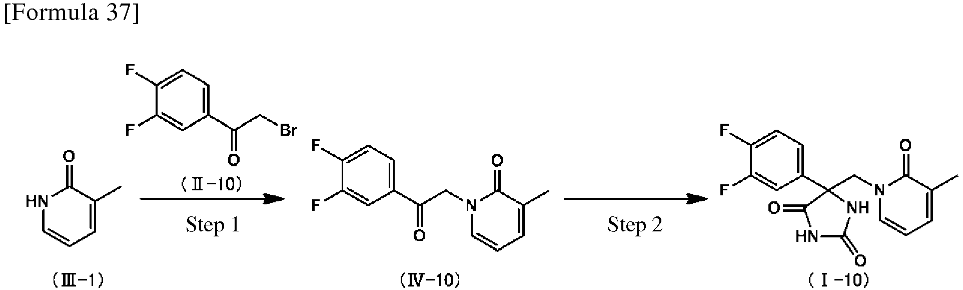

### Step 1

Cesium carbonate (436 mg, 1.3 mmol) and 3',4'-difluorophenacyl bromide (II-10) (300 mg, 1.3 mmol) were added to a solution of compound (III-1) (133 mg, 1.2 mmol) in N,N-dimethylformamide (10 mL), and the resultant mixture was stirred for 3 hours at room temperature. The reaction solution was diluted with water. The precipitated solid was collected by filtration and washed with water to obtain compound (IV-10) (amount 206 mg, yield 64%) as a yellow solid.

### Step 2

Water (0.8 mL) was added to a suspension of this compound (IV-10) (206 mg, 0.78 mmol), potassium cyanide (61 mg, 0.94 mmol), and ammonium carbonate (301 mg, 3.13 mmol) in ethanol (0.8 mL). The resultant mixture was sealed, and stirred for 67 hours at 100°C. After leaving to cool, the reaction solution was diluted with water, and the mixture was extracted with ethyl acetate. The resultant product was dried over anhydrous sodium sulfate, and the solvent was removed under reduced pressure. Chloroform was added to the residue, and the precipitated solid was collected by filtration to obtain compound (I-10) (amount 119 mg, yield 46%) as a colorless solid. The physical properties are shown below.
¹H-NMR (DMSO-d₆) δ: 1.98 (3H, s), 4.46 (1H, d, J=13.7 Hz), 4.61 (1H, d, J=13.7 Hz), 6.13 (1H, t, J=6.9 Hz), 7.22-7.33 (2H, m), 7.45-7.58 (2H, m), 7.69 (1H, ddd, J=2.3, 7.8, 12.4 Hz), 8.66 (1H, s), 10.99 (1H, s).
MS (ESI-FTMS) m/z 334 [M+H]⁺.

### Working Example 11

### Production of 5-(4-bromophenyl)-5-[(3-methyl-2-oxopyridin-1(2H)-yl)methyl]imidazolidine-2,4-dione (I-11)

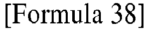

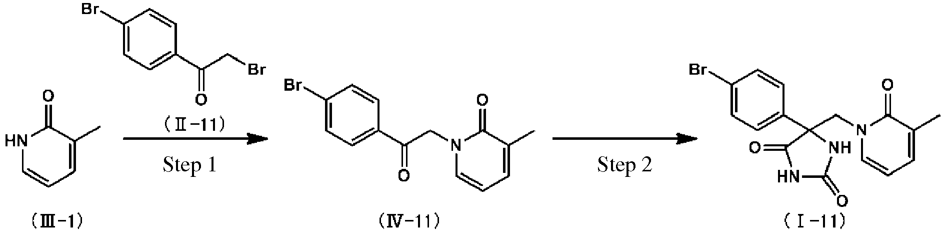

### Step 1

Potassium carbonate (791 mg, 5.7 mmol) and 4'-bromophenacyl bromide (II-11) (636 mg, 2.3 mmol) were added to a solution of compound (III-1) (250 mg, 2.3 mmol) in dimethyl sulfoxide (4.6 mL), and the resultant mixture was stirred at room temperature. After confirming the completion of the reaction by TLC, the reaction solution was diluted with water, and the mixture was extracted with ethyl acetate. The organic layer was washed with saturated brine, and then dried over sodium sulfate. The solvent was removed under reduced pressure, and then the resultant product was purified by column chromatography (silica gel) to obtain compound (IV-11) (amount 553 mg, yield 79%).

### Step 2

Water (0.98 mL) was added to a suspension of this compound (IV-11) (300 mg, 0.98 mmol), potassium cyanide (77 mg, 1.18 mmol), and ammonium carbonate (377 mg, 3.92 mmol) in ethanol (0.98 mL). The resultant mixture was sealed, and stirred for 48 hours at 100°C. After leaving to cool, water was added to the reaction solution. The precipitated solid was collected by filtration and washed with chloroform to obtain compound (I-11) (amount 230 mg, yield 62%). The physical properties are shown below.
¹H-NMR (DMSO-d₆) δ: 1.99 (3H, s), 4.45 (1H, d, J=13.7 Hz), 4.62 (1H, d, J=13.7 Hz), 6.12 (1H, t, J=6.7 Hz), 7.27 (2H, dd, J=6.9, 13.7 Hz), 7.58 (2H, d, J=8.7 Hz), 7.65 (2H, d, J=8.7 Hz), 8.65 (1H, s), 10.92 (1H, s).
MS (ESI-FTMS) m/z 376, 378 [M+H]⁺.

### Working Example 12

### Production of 5-(4-fluoro-3-methoxyphenyl)-5-[(3-methyl-2-oxopyridin-1(2H)-yl)methyl]imidazolidine-2,4-dione (I-12)

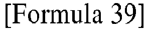

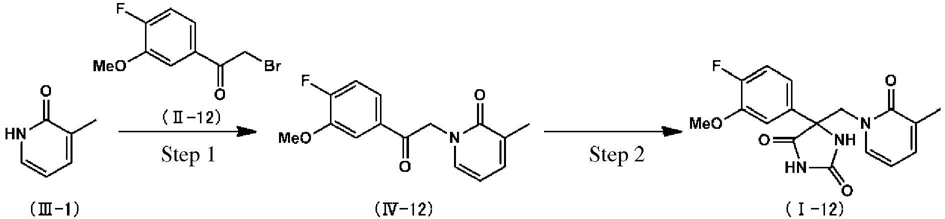

### Step 1

Compound (IV-12) (amount 153 mg, yield 48%) was obtained from compound (III-1) and 2-bromo-4'-fluoro-3'-methoxyacetophenone (II-12) based on the same production method as for compound (IV-10).

### Step 2

Water (1.0 mL) was added to a suspension of this compound (IV-12) (153 mg, 0.56 mmol), potassium cyanide (43 mg, 0.67 mmol), and ammonium carbonate (213 mg, 2.22 mmol) in ethanol (1.0 mL). The resultant mixture was sealed, and stirred for 66 hours at 100°C. After leaving to cool, water was added to the reaction solution. The precipitated solid was collected by filtration and washed with water to obtain compound (I-12) (amount 129 mg, yield 67%) as a colorless solid. The physical properties are shown below.
¹H-NMR (DMSO-d₆) δ: 1.99 (3H, s), 3.85 (3H, s), 4.45 (1H, d, J=13.7 Hz), 4.57 (1H, d, J=13.7 Hz), 6.12 (1H, t, J=6.9 Hz), 7.20-7.26 (2H, m), 7.29 (1H, m), 7.39 (1H, m), 7.48 (1H, dd, J=2.3, 12.8 Hz), 8.59 (1H, s), 10.90 (1H, s).
MS (ESI-FTMS) m/z 346 [M+H]⁺.

### Working Example 13

### Production of 5-[4-(methoxymethoxy)phenyl]-5-[(3-methyl-2-oxopyridin-1(2H)-yl)methyl]imidazolidine-2,4-dione (I-13)

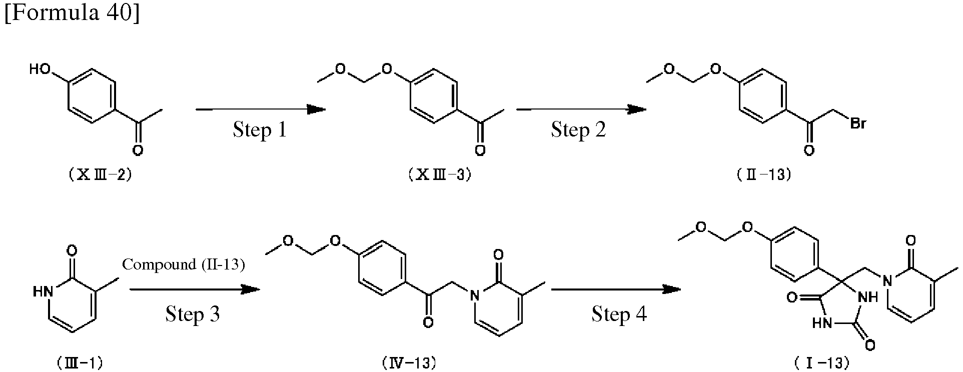

### Step 1

Under ice cooling, methoxymethyl chloride (1.3 mL, 17.6 mmol) was added dropwise to a solution of 4'-hydroxyacetophenone (XIII-2) (2.0 g, 14.6 mmol) and diisopropylethylamine (5.1 mL, 29.4 mmol) in dichloromethane, and the resultant mixture was stirred for 14 hours at room temperature. The solvent was removed under reduced pressure, and the resultant product was purified by column chromatography (silica gel) to obtain compound (XIII-3) (amount 2.7 g, yield 99%) as a colorless oily substance.

### Step 2

Under ice cooling, phenyltrimethylammonium tribromide (5.6 g, 15.0 mmol) was added to a solution of this compound (XIII-3) (2.7 g, 15.0 mmol) in tetrahydrofuran (30 mL), and the resultant mixture was stirred for 1 hour at room temperature. The reaction solution was diluted with water, and the resultant mixture was extracted with ethyl acetate. The organic layer was washed with saturated brine, and then dried over magnesium sulfate. The solvent was removed under reduced pressure, and then the resultant product was purified by column chromatography (silica gel) to obtain compound (II-13) (amount 312 mg, yield 8.0%).

### Step 3

Compound (IV-13) (amount 147 mg, yield 45%) was obtained from compound (III-1) and compound (II-13) based on the same production method as for compound (II-3).

### Step 4

Compound (I-13) (amount 38 mg, yield 26%) was obtained as a yellow solid from compound (IV-13) based on the same production method as for compound (I-12).
¹H-NMR (DMSO-d₆) δ: 2.00 (3H, s), 3.37 (3H, s), 4.45 (1H, d, J= 13.7 Hz), 4.58 (1H, d, J=13.7 Hz), 5.21 (2H, s), 6.11 (1H, t, J=6.9 Hz), 7.07 (2H, td, J=2.5, 8.7 Hz), 7.22-7.31 (2H, m), 7.54 (2H, td, J=2.5, 8.7 Hz), 8.52 (1H, s), 10.81 (1H, s).
MS (ESI-FTMS) m/z 358 [M+H]⁺.

### Working Example 14

### Production of 5-(3-fluoro-4-methoxyphenyl)-5-[(3-methyl-2-oxopyridin-1(2H)-yl)methyl]imidazolidine-2,4-dione (I-14)

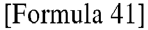

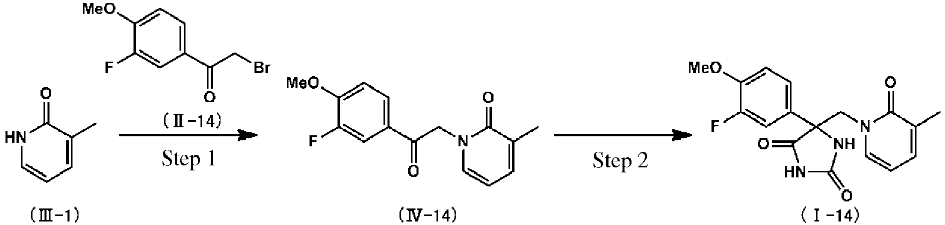

### Step 1

Compound (IV-14) (amount 239 mg, yield 75%) was obtained from compound (III-1) and 2-bromo-3'-fluoro-4'-methoxyacetophenone (II-14) based on the same production method as for compound (IV-5).

### Step 2

An aqueous ammonia solution (28%, 950 µL) was added to a suspension of this compound (IV-14) (237 mg, 0.86 mmol), potassium cyanide (67 mg, 1.03 mmol), and ammonium carbonate (331 mg, 3.44 mmol) in ethanol (950 µL). The resultant mixture was sealed, and stirred for 64 hours at 100°C. After leaving to cool, the reaction solution was diluted with water, and the resultant mixture was extracted with ethyl acetate. The organic layer was washed with saturated brine, and then dried over sodium sulfate. The solvent was removed under reduced pressure, and then the resultant product was purified by column chromatography (silica gel) to obtain compound (I-14) (amount 98 mg, yield 33%) as a yellow solid. The physical properties are shown below.
¹H-NMR (DMSO-d₆) δ: 2.00 (3H, s), 3.86 (3H, s), 4.49 (1H, d, J=13.7 Hz), 4.57 (1H, d, J=13.7 Hz), 6.12 (1H, t, J=6.9 Hz), 7.18-7.31 (4H, m), 7.41 (1H, dd, J=1.8, 8.2 Hz), 8.63 (1H, s), 10.91 (1H, s).
MS (ESI-FTMS) m/z 346 [M+H]⁺.

### Working Example 15

### Production of 5-(4-fluorophenyl)-5-[(3-methyl-2-oxopyridin-1(2H)-yl)methyl]imidazolidine-2,4-dione (I-15)

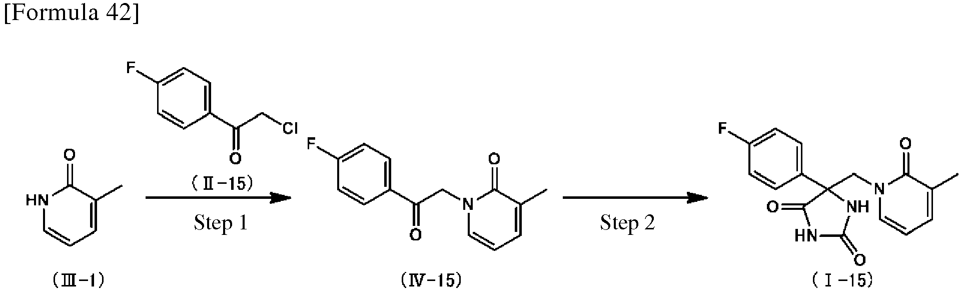

### Step 1

Potassium carbonate (3.1 g, 22.6 mmol) and 4'-fluorophenacyl chloride (II-15) (3.0 g, 17.4 mmol) were added to a solution of compound (III-1) (2.1 g, 19.1 mmol) in acetone (30 mL), and the resultant mixture was heated under reflux for 19 hours. After leaving to cool, the reaction solution was diluted with water, and the resultant mixture was extracted with ethyl acetate. The organic layer was washed with saturated brine, and then dried over magnesium sulfate. The solvent was removed under reduced pressure, and then the resultant product was purified by column chromatography (silica gel) to obtain compound (IV-15) (amount 2.6 g, yield 61%).

### Step 2

An aqueous ammonia solution (28%, 0.8 mL) was added to a suspension of this compound (IV-15) (200 mg, 0.82 mmol), potassium cyanide (64 mg, 0.98 mmol), and ammonium carbonate (313 mg, 3.26 mmol) in ethanol (0.8 mL). The resultant mixture was sealed, and stirred for 64 hours at 100°C. After leaving to cool, the reaction solution was diluted with water. The precipitated solid was collected by filtration and washed with water to obtain compound (I-15) (amount 178 mg, yield 69%) as a colorless solid. The physical properties are shown below.
¹H-NMR (DMSO-d₆) δ: 1.99 (3H, s), 4.45 (1H, d, J=13.7 Hz), 4.61 (1H, d, J=13.7 Hz), 6.11 (1H, t, J=6.9 Hz), 7.23-7.31 (4H, m), 7.64-7.71 (2H, m), 8.62 (1H, s), 10.89 (1H, s).
MS (ESI-FTMS) m/z 316 [M+H]⁺.

### Working Example 16

### Production of 5-(3,4-dimethoxyphenyl)-5-[(3-methyl-2-oxopyridin-1(2H)-yl)methyl]imidazolidine-2,4-dione (I-16)

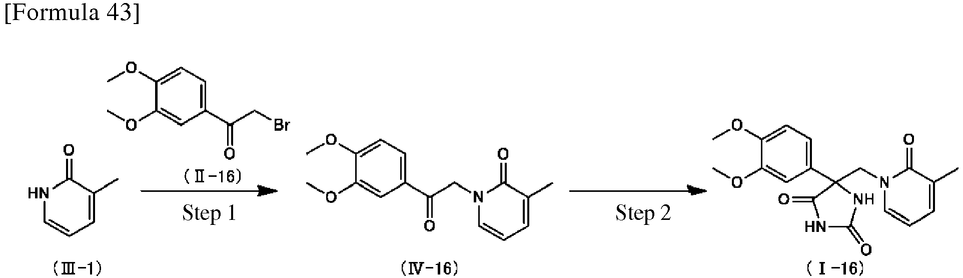

### Step 1

Compound (IV-16) (amount 231 mg, yield 73%) was obtained as a colorless solid from compound (III-1) and 2-bromo-1-(3,4-dimethoxyphenyl)ethanone (II-16) based on the same production method as for compound (III-1).

### Step 2

An aqueous ammonia solution (28%, 0.8 mL) was added to a suspension of this compound (IV-16) (230 mg, 0.80 mmol), potassium cyanide (83 mg, 0.98 mmol), and ammonium carbonate (308 mg, 3.20 mmol) in ethanol (0.8 mL). The resultant mixture was sealed, and stirred for 65 hours at 100°C. After leaving to cool, the solvent was removed under reduced pressure, methanol was added, and the precipitated solid was removed by filtration. The filtrate solvent was removed under reduced pressure, and the resultant product was dissolved in a small amount of chloroform. Hexane was added, the precipitated solid was filtered, and the filtered solid was then washed with chloroform to obtain compound (I-16) (amount 46 mg, yield 16%) as a colorless solid.
¹H-NMR (DMSO-d₆) δ: 2.00 (3H, s), 3.75 (3H, s), 3.78 (3H, s), 4.48 (1H, d, J=13.7 Hz), 4.54 (1H, d, J=13.7 Hz), 6.11 (1H, t, J=6.6 Hz), 6.99 (1H, d, J=8.7 Hz), 7.15 (1H, dd, J=2.1, 8.7 Hz), 7.19-7.32 (3H, m), 8.55 (1H, s), 10.80 (1H, s).
MS (ESI-FTMS) m/z 358 [M+H]⁺.

### Working Example 17

### Production of 5-[4-(tert-butyl)phenyl]-5-[(3-methyl-2-oxopyridin-1(2H)-yl)methyl]imidazolidine-2,4-dione (I-17)

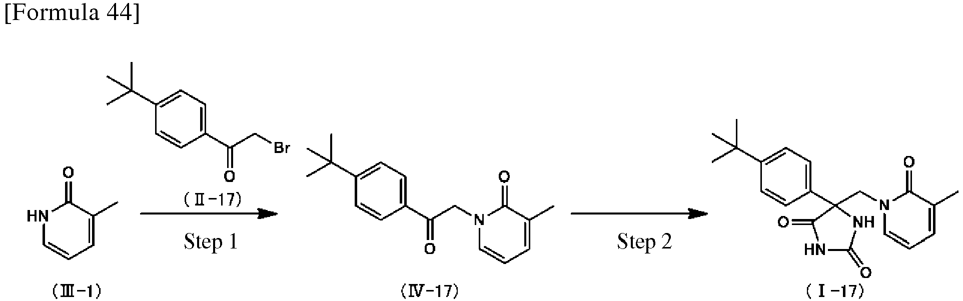

### Step 1

Potassium carbonate (950 mg, 6.9 mmol) and 4'-tert-butylphenacyl chloride (II-17) (300 mg, 2.7 mmol) were added to a solution of compound (III-1) (330 mg, 3.0 mmol) in dimethyl sulfoxide (5.5 mL), and the resultant mixture was stirred at room temperature. After confirming the completion of the reaction by TLC, the reaction solution was diluted with water, and the resultant mixture was extracted with ethyl acetate. The organic layer was washed with saturated brine, and dried over sodium sulfate. The solvent was removed under reduced pressure, and the resultant product was purified by column chromatography (silica gel) to obtain compound (IV-17) (amount 723 mg, yield 93%).

### Step 2

An aqueous ammonia solution (28%, 1.1 mL) was added to a suspension of this compound (IV-17) (300 mg, 1.1 mmol), potassium cyanide (83 mg, 1.3 mmol), and ammonium carbonate (407 mg, 4.2 mmol) in ethanol (1.1 mL). The resultant mixture was sealed, and stirred for 64 hours at 100°C. After leaving to cool, the reaction solution was diluted with water. The precipitated solid was collected by filtration and washed with chloroform to obtain compound (I-17) (amount 301 mg, yield 80%). The physical properties are shown below.
¹H-NMR (DMSO-d₆) δ: 1.28 (9H, s), 2.00 (3H, s), 4.45 (1H, d, J=13.7 Hz), 4.61 (1H, d, J=13.7 Hz), 6.12 (1H, t, J=6.9 Hz), 7.24-7.31 (2H, m), 7.45 (2H, d, J=8.2 Hz), 7.54 (2H, d, J=8.7 Hz), 8.52 (1H, s), 10.79 (1H, s).
MS (ESI-FTMS) m/z 354 [M+H]⁺.

### Working Example 18

### Production of 5-(2,4-dimethoxyphenyl)-5-[(3-methyl-2-oxopyridin-1(2H)-yl)methyl]imidazolidine-2,4-dione (I-18)

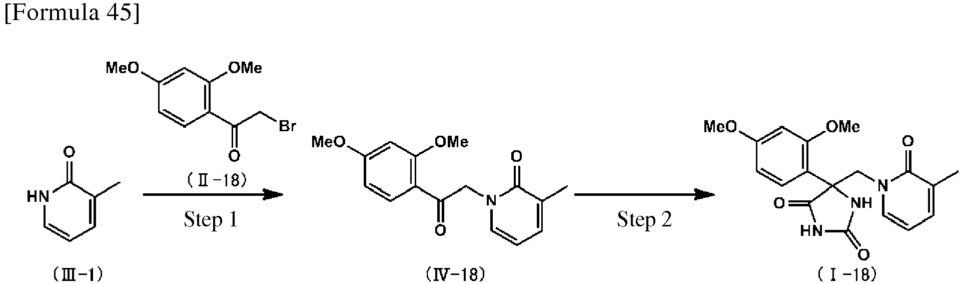

### Step 1

Compound (IV-18) (amount 594 mg, yield 90%) was obtained from compound (III-1) and 2',4'-dimethoxyphenacyl bromide (II-18) based on the same production method as for compound (IV-11).

### Step 2

An aqueous ammonia solution (28%, 1.0 mL) was added to a suspension of this compound (IV-18) (300 mg, 1.0 mmol), potassium cyanide (82 mg, 1.3 mmol), and ammonium carbonate (401 mg, 4.2 mmol) in ethanol (1.0 mL). The resultant mixture was sealed, and stirred for 64.25 hours at 100°C. After leaving to cool, the solvent was removed under reduced pressure. Methanol was added to the residue, and the precipitated solid was removed by filtration. The filtrate solvent was removed under reduced pressure, and the resultant product was then purified by column chromatography (silica gel) to obtain compound (I-18) (amount 285 mg, yield 76%). ¹H-NMR (DMSO-d₆) δ: 1.99 (3H, s), 3.76 (3H, s), 3.78 (3H, s), 4.36 (1H, d, J=13.3 Hz), 4.86 (1H, d, J=13.3 Hz), 6.11 (1H, t, J=6.9 Hz), 6.55 (1H, dd, J=2.3, 8.7 Hz), 6.64 (1H, d, J=2.3 Hz), 7.21 (1H, d, J=6.9 Hz), 7.28 (1H, d, J=6.4 Hz), 7.40 (1H, d, J=8.7 Hz), 7.66 (1H, s), 10.68 (1H, s). MS (ESI-FTMS) m/z 358 [M+H]⁺.

### Working Example 19

### Production of 5-[(3-methyl-2-oxopyridin-1(2H)-yl)methyl]-5-[3-trifluoromethyl)phenyl]imidazolidine-2,4-dione (I-19)

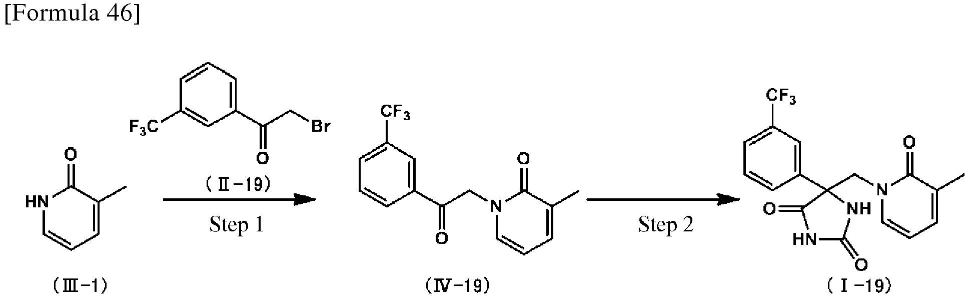

### Step 1

Compound (IV-19) (amount 355 mg, yield 64%) was obtained from compound (III-1) and 3'-(trifluoromethyl)phenacyl bromide (II-19) based on the same production method as for compound (IV-3).

### Step 2

Compound (I-19) (amount 207 mg, yield 56%) was obtained from this compound (IV-19) based on the same production method as for compound (I-18). The physical properties are shown below.
¹H-NMR (DMSO-d₆) δ: 1.97 (3H, s), 4.50 (1H, d, J=13.7 Hz), 4.67 (1H, d, J=13.7 Hz), 6.13 (1H, t, J=6.9 Hz), 7.29 (2H, d, J=6.9 Hz), 7.69 (1H, t, J=8.0 Hz), 7.77 (1H, d, J=7.3 Hz), 7.92-8.02 (2H, m), 8.76 (1H, s), 11.00 (1H, s).
MS (ESI-FTMS) m/z 366 [M+H]⁺.

### Working Example 20

### Production of 5-(2,5-dimethoxyphenyl)-5-[(3-methyl-2-oxopyridin-1(2H)-yl)methyl]imidazolidine-2,4-dione (I-20)

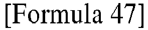

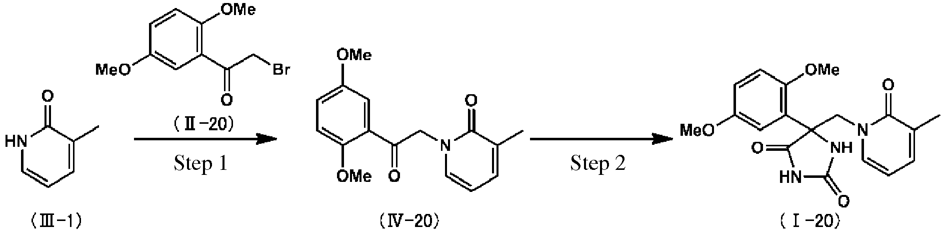

### Step 1

Compound (IV-20) (amount 595 mg, yield 72%) was obtained from compound (III-1) and 2',5'-dimethoxyphenacyl bromide (II-20) based on the same production method as for compound (IV-3).

### Step 2

Compound (I-20) (amount 170 mg, yield 46%) was obtained from this compound (IV-20) based on the same production method as for compound (I-18). The physical properties are shown below.
¹H-NMR (DMSO-d₆) δ: 1.99 (3H, s), 3.73 (3H, s), 3.73 (3H, s), 4.43 (1H, d, J=13.3 Hz), 4.84 (1H, d, J=13.3 Hz), 6.11 (1H, t, J=6.7 Hz), 6.97 (1H, dd, J=3.0, 9.0 Hz), 7.05 (1H, d, J=9.2 Hz), 7.09 (1H, d, J=3.2 Hz), 7.23 (1H, d, J=6.9 Hz), 7.29 (1H, d, J=6.9 Hz), 7.73 (1H, s), 10.74 (1H, s).
MS (ESI-FTMS) m/z 358 [M+H]⁺.

### Working Example 21

### Production of 5-(4-fluoro-2-methoxyphenyl)-5-[(3-methyl-2-oxopyridin-1(2H)-yl)methyl]imidazolidine-2,4-dione (I-21)

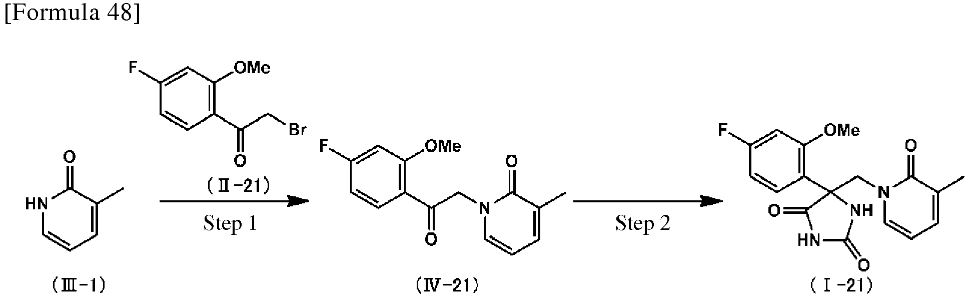

### Step 1

Compound (IV-21) (amount 192 mg, yield 60%) was obtained as a colorless solid from compound (III-1) and 4'-fluoro-2'-methoxyphenacyl bromide (II-21) based on the same production method as for compound (IV-3).

### Step 2

An aqueous ammonia solution (28%, 0.7 mL) was added to a suspension of this compound (IV-21) (192 mg, 0.70 mmol), potassium cyanide (55 mg, 0.84 mmol), and ammonium carbonate (288 mg, 2.80 mmol) in ethanol (0.7 mL). The resultant mixture was sealed, and stirred for 63 hours at 100°C. After leaving to cool, the reaction solution was diluted with water. The precipitated solid was collected by filtration and washed with water and chloroform to obtain compound (I-21) (amount 176 mg, yield 73%) as a colorless solid. The physical properties are shown below.
¹H-NMR (DMSO-d₆) δ: 1.99 (3H, s), 3.79 (3H, s), 4.41 (1H, d, J=13.3 Hz), 4.85 (1H, d, J=13.3 Hz), 6.11 (1H, t, J=6.6 Hz), 6.83 (1H, dt, J=2.7, 8.5 Hz), 7.04 (1H, dd, J=2.7, 11.0 Hz), 7.22 (1H, dd, J=1.4, 6.9 Hz), 7.28 (1H, m), 7.54 (1H, dd, J=6.4, 8.7 Hz), 7.78 (1H, s), 10.76 (1H, s).
MS (ESI-FTMS) m/z 346 [M+H]⁺.

### Working Example 22

### Production of 5-(benzofuran-5-yl)-5-[(3-methyl-2-oxopyridin-1(2H)-yl)methyl]imidazolidine-2,4-dione (I-22)

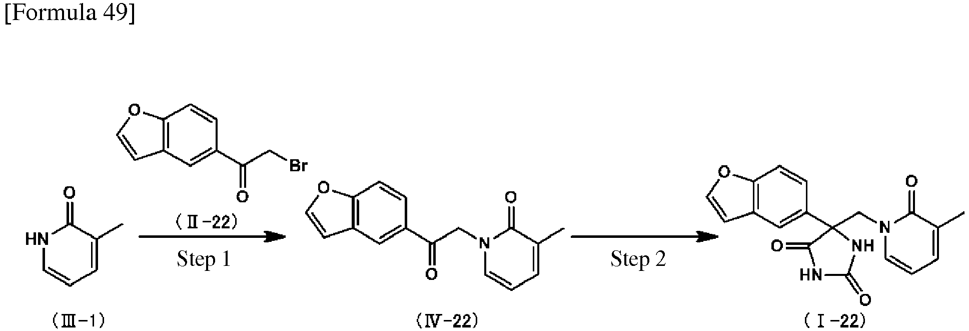

### Step 1

Compound (IV-22) (amount 224 mg, yield 70%) was obtained as a colorless solid from compound (III-1) and 5'-(2-bromoacetyl)benzofuran (II-22) based on the same production method as for compound (IV-3).

### Step 2

Compound (I-22) (amount 219 mg, yield 77%) was obtained as a colorless solid from this compound (IV-22) based on the same production method as for compound (I-15). The physical properties are shown below.
¹H-NMR (DMSO-d₆) δ: 2.00 (3H, s), 4.51 (1H, d, J=13.3 Hz), 4.68 (1H, d, J=13.3 Hz), 6.11 (1H, t, J=6.9 Hz), 7.03 (1H, dd, J=0.9, 2.3 Hz), 7.23-7.32 (2H, m), 7.60 (1H, dd, J=2.3, 8.7 Hz), 7.67 (1H, d, J=8.7 Hz), 7.94 (1H, d, J=1.8 Hz), 8.05 (1H, d, J=2.3 Hz), 8.62 (1H, s), 10.85 (1H, s).
MS (ESI-FTMS) m/z 338 [M+H]⁺.

### Working Example 23

### Production of 5-(benzo[b]thiophen-5-yl)-5-[(3-methyl-2-oxopyridin-1(2H)-yl)methyl]imidazolidine-2,4-dione (I-23)

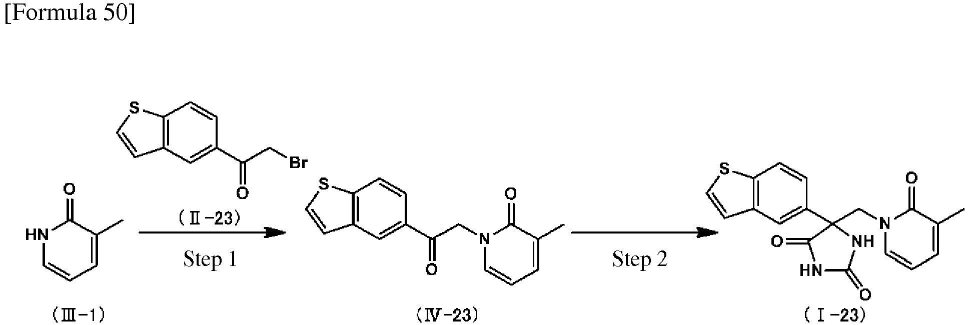

### Step 1

Compound (IV-23) (amount 241 mg, yield 76%) was obtained as a colorless solid from compound (III-1) and 1-(1-benzothiophen-5-yl)-2-bromo-1-ethanone (II-23) based on the same production method as for compound (IV-2).

### Step 2

Compound (I-23) (amount 262 mg, yield 64%) was obtained as a pale yellow solid from this compound (IV-23) based on the same production method as for compound (I-15). The physical properties are shown below.
¹H-NMR (DMSO-d₆) δ: 2.00 (3H, s), 4.54 (1H, d, J=13.7 Hz), 4.72 (1H, d, J=13.7 Hz), 6.12 (1H, t, J=6.7 Hz), 7.29 (2H, dd, J=0.9, 6.9 Hz), 7.52 (1H, d, J=7.5 Hz), 7.64 (1H, dd, J=1.8, 8.7 Hz), 7.83 (1H, d, J=5.5 Hz), 8.08 (1H, d, J=8.2 Hz), 8.30 (1H, d, J=1.8 Hz), 8.69 (1H, s), 10.86 (1H, s).
MS (ESI-FTMS) m/z 354 [M+H]⁺.

### Working Example 24

### Production of 5-[2-methoxy-5-(trifluoromethoxy)phenyl]-5-[(3-methyl-2-oxopyridin-1(2H)-yl)methyl]imidazolidine-2,4-dione (I-24)

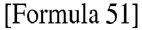

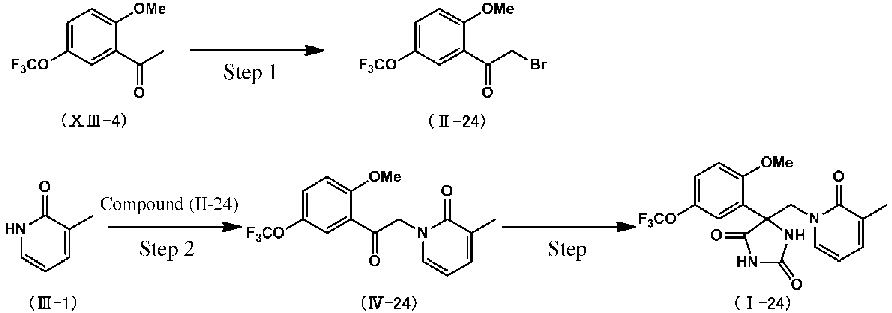

### Step 1

Compound (II-24) (amount 820 mg, yield 93%) was obtained as a colorless solid from 2'-methoxy-5'-(trifluoromethoxy)acetophenone (XIII-4) based on the same production method as for compound (II-13).

### Step 2

Compound (IV-24) (amount 102 mg, yield 33%) was obtained from compound (III-1) and compound (II-24) based on the same production method as for compound (IV-3).

### Step 3

An aqueous ammonia solution (28%, 0.3 mL) was added to a suspension of this compound (IV-24) (102 mg, 0.30 mmol), potassium cyanide (23 mg, 0.36 mmol), and ammonium carbonate (115 mg, 1.20 mmol) in ethanol (0.3 mL). The resultant mixture was sealed, and stirred for 64 hours at 100°C. After leaving to cool, the reaction solution was diluted with water. The precipitated solid was collected by filtration, washed with water, and then purified by column chromatography (silica gel) to obtain compound (I-24) (amount 45 mg, yield 37%) as a colorless solid. The physical properties are shown below.
¹H-NMR (DMSO-d₆) δ: 1.98 (3H, s), 3.81 (3H, s), 4.46 (1H, d, J=13.3 Hz), 4.82 (1H, d, J=13.3 Hz), 6.12 (1H, t, J=6.9 Hz), 7.19-7.32 (3H, m), 7.45 (1H, m), 7.51 (1H, d, J=2.7 Hz), 7.90 (1H, s), 10.85 (1H, s).
MS (ESI-FTMS) m/z 412 [M+H]⁺.

### Working Example 25

### Production of 5-[(3-methyl-2-oxopyridin-1(2H)-yl)methyl]-5-[4-(methylsulfonyl)phenyl]imidazolidine-2,4-dione (I-25)

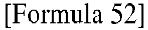

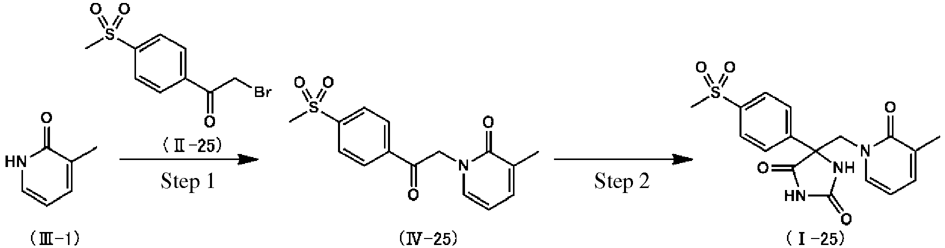

### Step 1

Compound (IV-25) (amount 175 mg, yield 21%) was obtained from compound (III-1) and 4'-(methylsulfonyl)phenacyl bromide (II-25) based on the same production method as for compound (IV-3).

### Step 2

Compound (I-25) (amount 86 mg, yield 63%) was obtained from this compound (IV-25) based on the same production method as for compound (I-17). The physical properties are shown below.
¹H-NMR (DMSO-d₆) δ: 1.99 (3H, s), 3.24 (3H, s), 4.48 (1H, d, J=13.7 Hz), 4.72 (1H, d, J=13.7 Hz), 6.14 (1H, t, J=6.9 Hz), 7.26-7.34 (2H, m), 7.91 (2H, d, J=8.7 Hz), 8.00 (2H, d, J=8.2 Hz), 8.72 (1H, s), 10.99 (1H, s).
MS (ESI-FTMS) m/z 376 [M+H]⁺.

### Working Example 26

### Production of 5-(chroman-6-yl)-5-[(3-methyl-2-oxopyridin-1(2H)-yl)methyl]imidazolidine-2,4-dione (I-26)

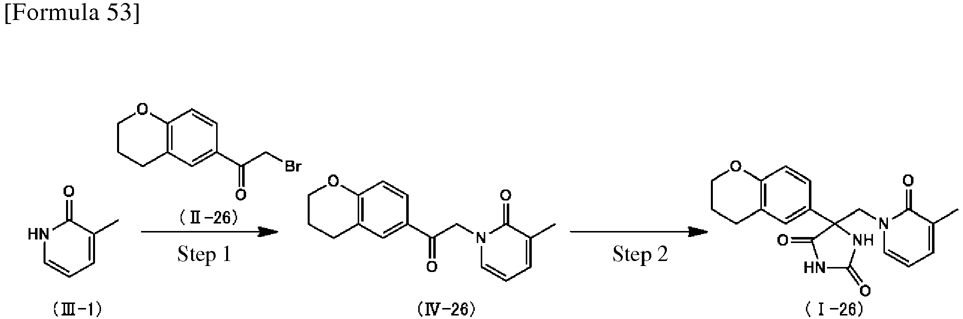

### Step 1

Compound (IV-26) (amount 256 mg, yield 99%) was obtained from compound (III-1) and 2-chloro-1-chroman-6-yl-ethanone (II-26) based on the same production method as for compound (IV-15).

### Step 2

Compound (I-26) (amount 81 mg, yield 25%) was obtained as a colorless solid from this compound (IV-26) based on the same production method as for compound (I-21). The physical properties are shown below.
¹H-NMR (DMSO-d₆) δ: 1.88-1.95 (2H, m), 2.00 (3H, s), 2.70-2.82 (2H, m), 4.06-4.20 (2H, m), 4.42 (1H, d, J=13.7 Hz), 4.54 (1H, d, J=13.3 Hz), 6.11 (1H, t, J=6.9 Hz), 6.77 (1H, d, J=8.7 Hz), 7.22-7.34 (4H, m), 8.43 (1H, s), 10.75 (1H, s).
MS (ESI-FTMS) m/z 354 [M+H]⁺.

### Working Example 27

### Production of 5-(5-chloro-2-methoxyphenyl)-5-[(3-methyl-2-oxopyridin-1(2H)-yl)methyl]imidazolidine-2,4-dione (I-27)

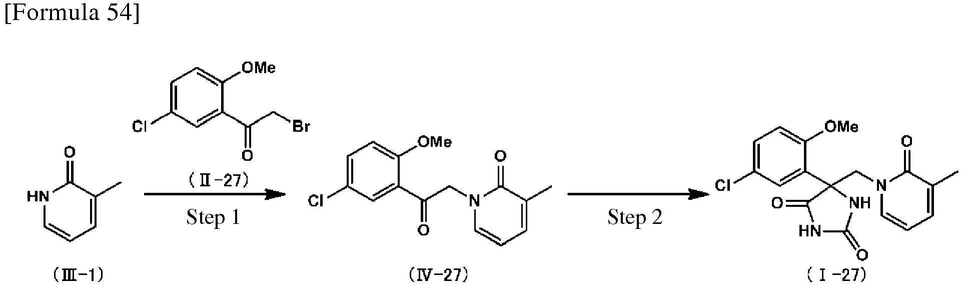

### Step 1

Compound (IV-27) (amount 228 mg, yield 72%) was obtained from compound (III-1) and 2-bromo-5'-chloro-2'-methoxyacetophenone (II-27) based on the same production method as for compound (IV-13).

### Step 2

Compound (I-27) (amount 61 mg, yield 23%) was obtained as a beige solid from this compound (IV-27) based on the same production method as for compound (I-17). The physical properties are shown below.
¹H-NMR (DMSO-d₆) δ: 1.99 (3H, s), 3.78 (3H, s), 4.45 (1H, d, J=13.2 Hz), 4.82 (1H, d, J=13.2 Hz), 6.12 (1H, t, J=6.9 Hz), 7.15 (1H, d, J=8.7 Hz), 7.22 (1H, m), 7.29 (1H, m), 7.47 (1H, dd, J=2.3, 8.7 Hz), 7.54 (1H, d, J=2.3 Hz), 7.83 (1H, br s), 10.82 (1H, br s).
MS (ESI-FTMS) m/z 362, 364 [M+H]⁺.

### Working Example 28

### Production of 5-(3-fluorophenyl)-5-[(3-methyl-2-oxopyridin-1(2H)-yl)methyl]imidazolidine-2,4-dione (I-28)

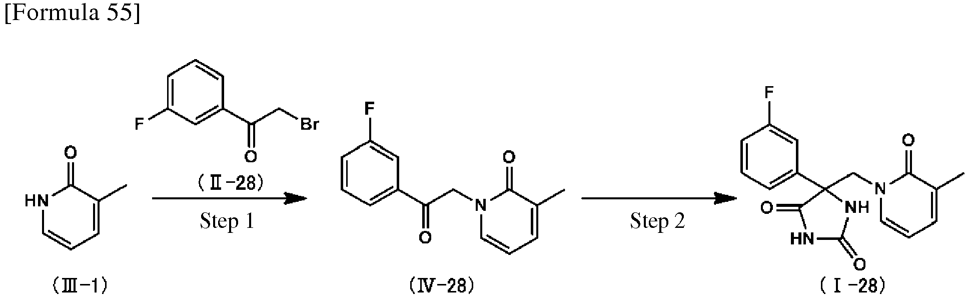

### Step 1

Compound (IV-28) (amount 195 mg, yield 46%) was obtained from compound (III-1) and 3'-fluorophenacyl bromide (II-28) based on the same production method as for compound (IV-3).

### Step 2

An aqueous ammonia solution (28%, 0.9 mL) was added to a suspension of this compound (IV-28) (195 mg, 0.80 mmol), potassium cyanide (78 mg, 1.19 mmol), and ammonium carbonate (306 mg, 3.18 mmol) in ethanol (0.9 mL). The resultant mixture was sealed, and stirred for 64 hours at 100°C. After leaving to cool, the reaction solution was diluted with water, and the resultant mixture was extracted with ethyl acetate. The solvent was removed under reduced pressure. Then, ethyl acetate-hexane (2:1) was added, and the precipitated solid was collected by filtration to obtain compound (I-28) (amount 140 mg, yield 56%). The physical properties are shown below.
¹H-NMR (DMSO-d₆) δ: 1.99 (3H, s), 4.47 (1H, d, J=13.7 Hz), 4.62 (1H, d, J=13.7 Hz), 6.12 (1H, d, J=6.9 Hz), 7.20-7.31 (3H, m), 7.44-7.53 (3H, m), 8.63 (1H, s), 10.93 (1H, br s).
MS (ESI-FTMS) m/z 316 [M+H]⁺.

### Working Example 29

### Production of 5-[(3-methyl-2-oxopyridin-1(2H)-yl)methyl]-5-(2,4,5-trifluorophenyl)imidazolidine-2,4-dione (I-29)

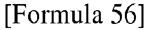

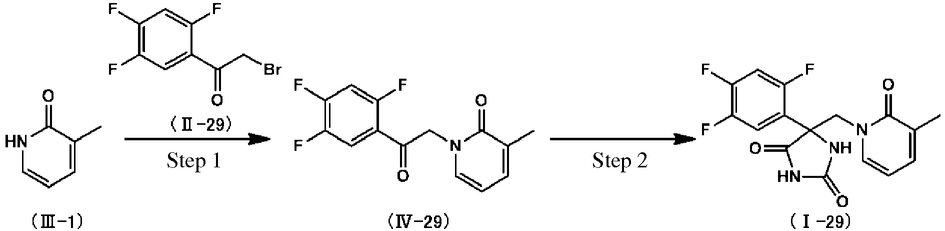

### Step 1

Compound (IV-29) (amount 130 mg, yield 25%) was obtained from compound (III-1) and 2',4',5'-trifluorophenacyl bromide (II-29) based on the same production method as for compound (IV-15).

### Step 2

An aqueous ammonia solution (28%, 0.8 mL) was added to a suspension of this compound (IV-29) (140 mg, 0.50 mmol), potassium cyanide (49 mg, 0.75 mmol), and ammonium carbonate (192 mg, 2.00 mmol) in ethanol (0.8 mL). The resultant mixture was sealed, and stirred for 64 hours at 100°C. After leaving to cool, the reaction solution was diluted with water, and the resultant mixture was extracted with ethyl acetate. The solvent was removed under reduced pressure, and the resultant product was purified by column chromatography (silica gel) to obtain compound (I-29) (amount 56 mg, yield 32%). The physical properties are shown below.
¹H-NMR (CDCl₃) δ: 2.11 (3H, s), 4.20 (1H, d, J=13.7 Hz), 5.09 (1H, d, J=13.7 Hz), 6.12 (1H, d, J=6.9 Hz), 7.04 (1H, m), 7.15 (1H, m), 7.23 (1H, m), 7.30 (1H, s), 7.60 (1H, m).
MS (ESI-FTMS) m/z 352 [M+H]⁺.

### Working Example 30

### Production of 5-(4-fluoro-3-methylphenyl)-5-[(3-methyl-2-oxopyridin-1(2H)-yl)methyl]imidazolidine-2,4-dione (I-30)

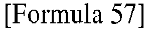

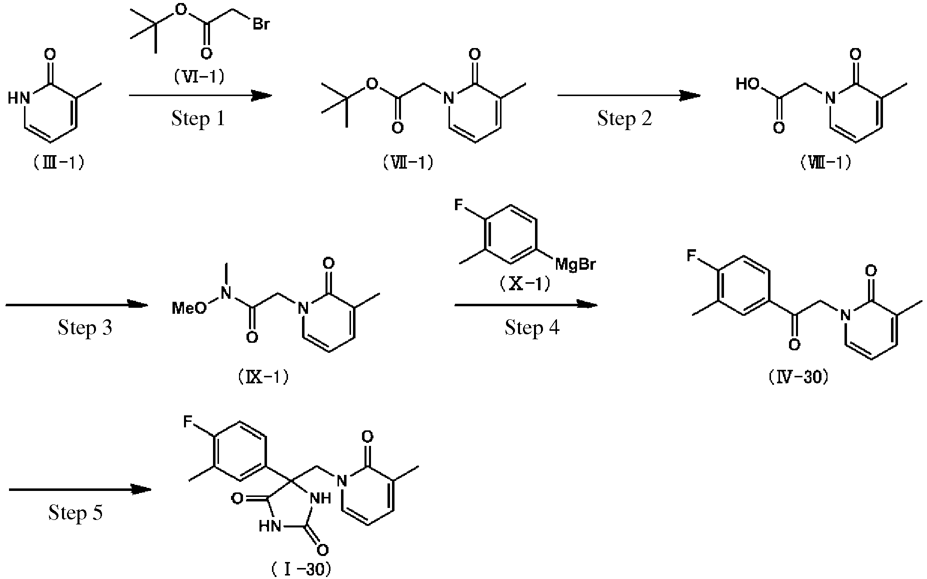

### Step 1

Tert-butyl 2-bromoacetate (VI-1) (4.81 mL, 32.99 mmol) was added to a suspension of compound (III-1) (3 g, 27.49 mmol) and potassium carbonate (9.50 g, 68.73 mmol) in dimethyl sulfoxide (27.5 mL), and the resultant mixture was stirred for 3.25 hours at room temperature. Water was added to the reaction solution, and the resultant mixture was extracted with ethyl acetate. The organic layer was washed with saturated brine, and then dried over anhydrous sodium sulfate. The solvent was removed under reduced pressure to obtain compound (VII-1) (amount 5.76 g, yield 94%).

### Step 2

Trifluoroacetic acid (26 mL) was added to a solution of this compound (VII-1) (5.76 g, 25.8 mmol) in chloroform (26 mL), and the resultant mixture was stirred for 15 hours at room temperature. The solvent was removed under reduced pressure to obtain compound (VIII-1) (amount 4.52 g, yield 99%).

### Step 3

Compound (VIII-1) (4.31 g, 25.8 mmol), N,O-dimethylhydroxylamine hydrochloride (3.02 g, 30.96 mmol), N-methylmorpholine (8.51 mL, 77.40 mmol), and 1-hydroxybenzotriazole monohydrate (4.77 g, 30.96 mmol) were dissolved in N,N-dimethylformamide (25.8 mL). Water-soluble carbodiimide hydrochloride (5.94 g, 30.96 mmol) was then added, and the resultant mixture was stirred for 194.25 hours at room temperature. Water was added to the reaction solution, and the resultant mixture was extracted with chloroform. The organic layer was washed with saturated brine, and then dried over anhydrous sodium sulfate. The solvent was removed under reduced pressure, and the resultant product was purified by column chromatography (silica gel) to obtain compound (IX-1) (amount 4.63 g, yield 85%) as a white solid.

### Step 4

4-Fluoro-3-methylphenylmagnesium bromide (X-1) in 1.0 mol/L tetrahydrofuran solution ( 1.1 mL, 1.1 mmol) was added dropwise to a solution (10 mL) cooled to -78°C of compound (IX-1) in tetrahydrofuran, and the resultant mixture was stirred for 30 minutes at -78°C. To the reaction solution was added 2 mol/L hydrochloric acid, and the resultant mixture was extracted with ethyl acetate. The organic layer was washed with saturated brine, and then dried over anhydrous sodium sulfate, and the solvent was removed under reduced pressure. The resultant product was purified by column chromatography (silica gel) to obtain compound (IV-30) (amount 90 mg, yield 34%) as a colorless oily substance.

### Step 5

Compound (I-30) (amount 30 mg, yield 26%) was obtained as a colorless solid from this compound (IV-30) based on the same production method as for compound (IV-17).
¹H-NMR (CDCl₃) δ: 2.11 (3H, s), 2.29 (3H, d, J=1.4 Hz), 4.22 (1H, d, J=13.7 Hz), 4.82 (1H, d, J=13.7 Hz), 6.10 (1H, t, J=6.9 Hz), 7.02 (1H, t, J=8.7 Hz), 7.14 (1H, m), 7.21 (1H, m), 7.42-7.50 (2H, m), 8.77 (1H, br s).
MS (ESI-FTMS) m/z 330 [M+H]⁺.

### Working Example 31

### Production of 5-[(3-methyl-2-oxopyridin-1(2H)-yl)methyl]-5-[4-(pyridin-4-yl)phenyl]imidazolidine-2,4-dione (I-31)

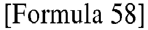

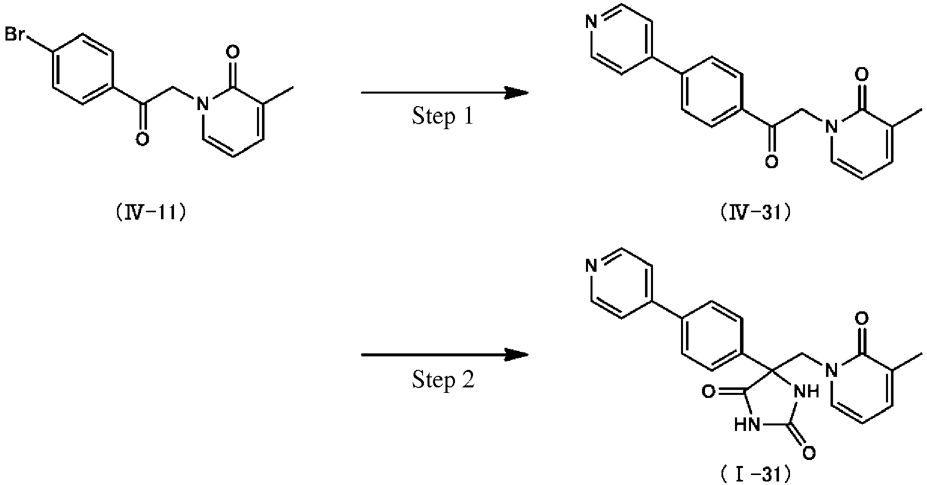

### Step 1

A solution of compound (IV-11) (400 mg, 1.32 mmol), (4-pyridine)cyclic triolborate sodium salt (320 mg, 1.44 mmol), and triphenylphosphine (34 mg, 0.13 mmol) in N,N-dimethylformamide (6.5 mL) was degassed. Then, under an argon atmosphere, palladium acetate (15 mg, 0.065 mmol) and copper iodide (50 mg, 0.26 mmol) were added, and the resultant mixture was heated for 15.5 hours at 90°C. A saturated aqueous solution of ammonium chloride was added to the reaction solution, and the resultant mixture was extracted with ethyl acetate. The organic layer was washed with saturated brine, and then dried over anhydrous sodium sulfate. The solvent was removed under reduced pressure, and the resultant product was purified by silica gel column chromatography to obtain compound (IV-31) (amount 109 mg, yield 22%).

### Step 2

An autoclave was charged with this compound (IV-31) (90 mg, 0.30 mmol), potassium cyanide (23 mg, 0.36 mmol), ammonium carbonate (114 mg, 1.18 mmol), ethanol (0.3 mL), and saturated ammonia water (0.3 mL). The autoclave was sealed, and the mixture was stirred for 63.75 hours at 100°C. The solvent was removed under reduced pressure and the resultant product was then purified by silica gel column chromatography to obtain compound (I-31) (amount 42 mg, yield 38%). The physical properties are shown below.
¹H-NMR (DMSO-d₆) δ: 2.00 (3H, s), 4.51 (1H, d, J=13.3 Hz), 4.68 (1H, d, J=13.7 Hz), 6.14 (1H, t, J=6.9 Hz), 7.27-7.32 (2H, m), 7.75 (2H, dd, J=1.8, 4.6 Hz), 7.78 (2H, d, J=8.7 Hz), 7.90 (2H, d, J=8.7 Hz), 8.66 (2H, d, J=6.0 Hz), 8.69 (1H, s), 10.91 (1H, s).
MS (ESI-FTMS) m/z: 375 [M+H]⁺.

### Working Example 32

### Production of 5-[(3-methyl-2-oxopyridin-1(2H)-yl)methyl]-5-[4-(pyridin-3-yl)phenyl]imidazolidine-2,4-dione (I-32)

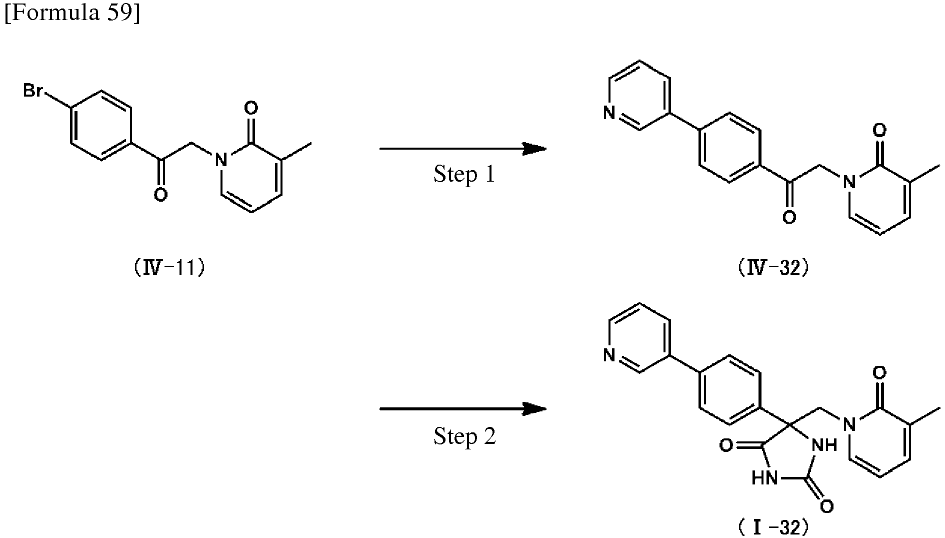

### Step 1

A suspension of compound (IV-11) (500 mg, 1.63 mmol), 3-pyridylboronic acid (221 mg, 1.80 mmol), and tripotassium phosphate (555 mg, 2.61 mmol) in 1,4-dioxane (3.3 mL) was degassed. Then, under an argon atmosphere, tetrakis(triphenylphosphine)palladium (94 mg, 0.082 mmol) was added, and the resultant mixture was heated for 22.5 hours at 90°C. Water was added to the reaction solution, and the resultant mixture was extracted with ethyl acetate. The organic layer was washed with saturated brine, and then dried over anhydrous sodium sulfate. The solvent was removed under reduced pressure, and the resultant product was purified by silica gel column chromatography to obtain compound (IV-32) (amount 232 mg, yield 47%).

An autoclave was charged with this compound (IV-32) (196 mg, 0.64 mmol), potassium cyanide (50 mg, 0.77 mmol), ammonium carbonate (248 mg, 2.58 mmol), ethanol (0.64 mL), and saturated ammonia water (0.64 mL). The autoclave was sealed, and the mixture was stirred for 66 hours at 100°C. Water was added to the reaction solution, and the precipitated solid was collected by filtration to obtain compound (I-32) (amount 184 mg, yield 76%). The physical properties are shown below.
¹H-NMR (DMSO-d₆) δ: 2.01 (3H, s), 4.52 (1H, d, J=13.7 Hz), 4.68 (1H, d, J=13.7 Hz), 6.14 (1H, t, J=6.9 Hz), 7.29 (2H, d, J=6.9 Hz), 7.51 (1H, dd, J=4.8, 8.0 Hz), 7.76 (2H, d, J=8.7 Hz), 7.83 (2H, d, J=8.7 Hz), 8.11 (1H, td, J=2.3, 8.2 Hz), 8.59 (1H, dd, J=1.4, 4.6 Hz), 8.66 (1H, s), 8.93 (1H, d, J=1.8 Hz), 10.89 (1H, s).
MS (ESI-FTMS) m/z: 375 [M+H]⁺.

### Working Example 33

### Production of 5-[4-(3,3-dimethylbut-1-yn-1-yl)phenyl]-5-[(3-methyl-2-oxopyridin-1(2H)-yl)methyl]imidazolidine-2,4-dione (I-33)

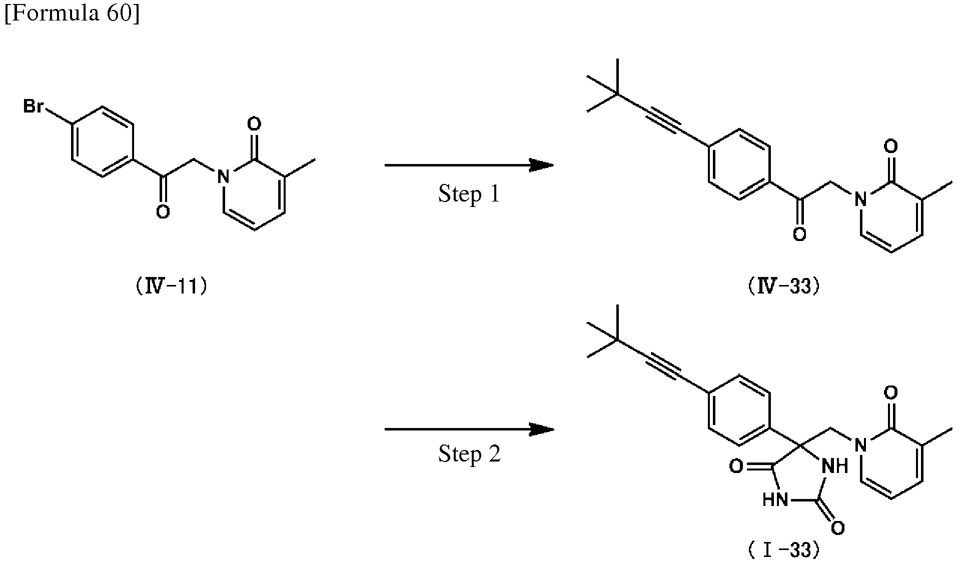

### Step 1

A solution of compound (IV-11) (500 mg, 1.63 mmol), 3,3-dimethylbut-1-yn (222 µL, 1.80 mmol), and triethylamine (250 µL, 1.80 mmol) in tetrahydrofuran (3.3 mL) was degassed. Then, under an argon atmosphere, tetrakis(triphenylphosphine)palladium (94 mg, 0.082 mmol) and copper iodide (31 mg, 0.16 mmol) were added, and the resultant mixture was heated under reflux for 22.5 hours. Water was added to the reaction solution, and the resultant mixture was extracted with ethyl acetate. The organic layer was washed with saturated brine, and then dried over anhydrous sodium sulfate. The solvent was removed under reduced pressure, and the resultant product was purified by silica gel column chromatography to obtain compound (IV-33) (amount 531 mg, quantitative yield).

Compound (I-33) (amount 270 mg, yield 73%) was obtained from compound (IV-33) based on the same production method as for compound (I-32). The physical properties are shown below.
¹H-NMR (DMSO-d₆) δ: 1.29 (9H, s), 1.99 (3H, s), 4.44 (1H, d, J=13.3 Hz), 4.62 (1H, d, J=13.7 Hz), 6.11 (1H, t, J=6.9 Hz), 7.22-7.29 (2H, m), 7.41 (2H, d, J=8.7 Hz), 7.59 (2H, d, J=8.2 Hz), 8.58 (1H, s), 10.88 (1H, s).
MS (ESI-FTMS) m/z: 378 [M+H]⁺.

### Working Example 34

### Production of 5-[(3-methyl-2-oxopyridin-l(2H)-yl)methyl]-5-[4-(p-tolyloxy)phenyl]imidazolidine-2,4-dione (I-34)

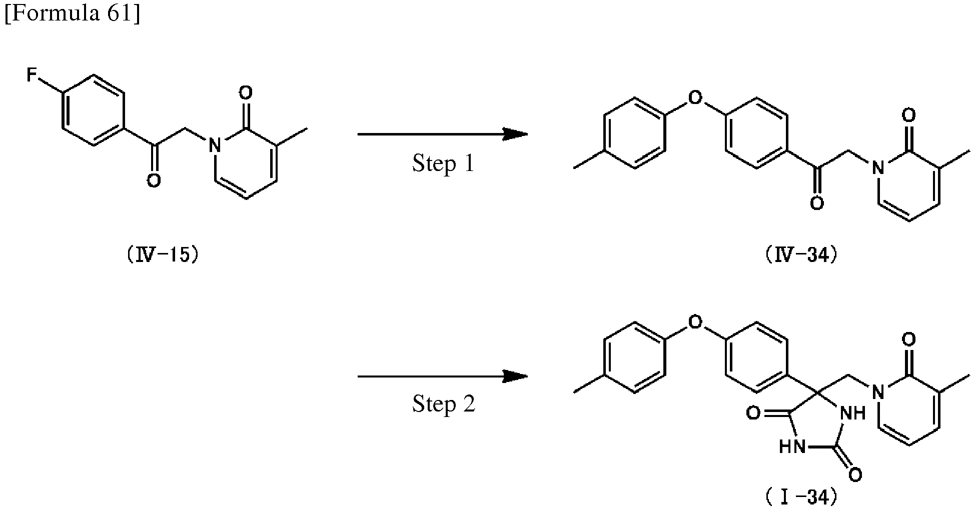

### Step 1

A suspension of compound (IV-15) (150 mg, 0.61 mmol), 4-methylphenol (66 mg, 0.61 mmol), and potassium carbonate (127 mg, 0.92 mmol) in N,N-dimethylacetamide was heated under reflux for 3 hours. After leaving to cool, the reaction solution was diluted with water, and the resultant mixture was extracted with ethyl acetate. The organic layer was washed with saturated brine, and then dried over anhydrous magnesium sulfate. The solvent was removed under reduced pressure, and the resultant product was purified by column chromatography (silica gel) to obtain compound (IV-34) (amount 122 mg, yield 60%).

### Step 2

An aqueous ammonia solution (28%, 0.35 mL) was added to a suspension of this compound (IV-34) (122 mg, 0.37 mmol), potassium cyanide (29 mg, 0.44 mmol), and ammonium carbonate (141 mg, 1.46 mmol) in ethanol (0.35 mL). The resultant mixture was sealed, and stirred for 63 hours at 100°C. After leaving to cool, the solvent was removed under reduced pressure, methanol was added, and the precipitated solid was removed by suction filtration. The filtrate solvent was removed under reduced pressure, and the resultant product was purified by column chromatography (silica gel) to obtain compound (I-34) (amount 56 mg, yield 38%) as a colorless solid. The physical properties are shown below.
¹H-NMR (DMSO-d₆) δ: 1.99 (3H, s), 2.30 (3H, s), 4.45 (1H, d, J=13.3 Hz), 4.60 (1H, d, J=13.3 Hz), 6.12 (1H, t, J=6.9 Hz), 6.93 (2H, td, J=2.5, 8.2 Hz), 7.01 (2H, td, J=2.5, 8.7 Hz), 7.17-7.32 (4H, m), 7.60 (2H, td, J=2.5, 9.2 Hz), 8.57 (1H, d, J=1.4 Hz), 10.85 (1H, s).
MS (ESI-FTMS) m/z 404 [M+H]⁺.

### Working Example 35

### Production of 5-[(3-methyl-2-oxopyridin-l(2H)-yl)methyl]-5-[4-(o-tolyloxy)phenyl]imidazolidine-2,4-dione (I-35)

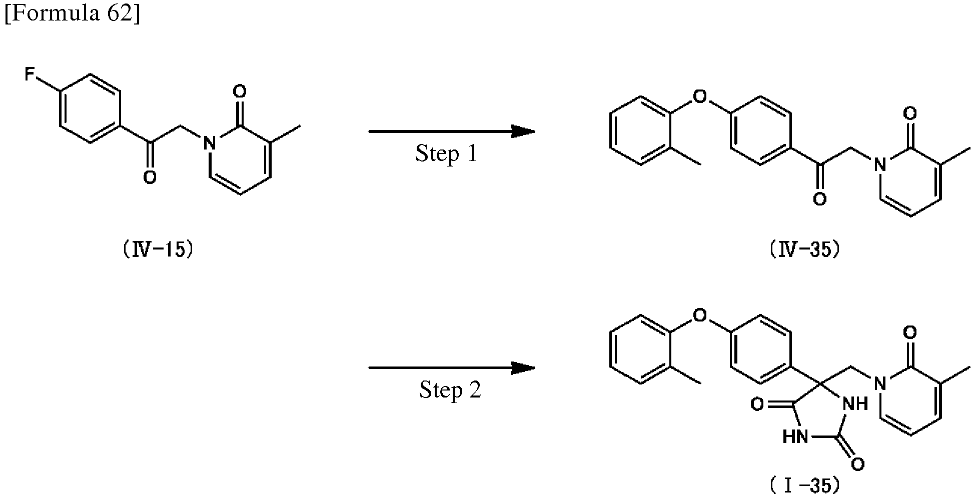

### Step 1

Compound (IV-35) (amount 188 mg, yield 92%) was obtained as a green oily substance from compound (IV-15) based on the same production method as for compound (IV-34).

An aqueous ammonia solution (28%, 0.6 mL) was added to a suspension of this compound (IV-35) (188 mg, 0.56 mmol), potassium cyanide (44 mg, 0.68 mmol), and ammonium carbonate (217 mg, 2.27 mmol) in ethanol (0.6 mL). The resultant mixture was sealed, and stirred for 63 hours at 100°C. After leaving to cool, the reaction solution was diluted with water, and the precipitated solid was collected by filtration. The resultant product was purified by column chromatography (silica gel) to obtain compound (I-35) (amount 101 mg, yield 45%) as a colorless solid. The physical properties are shown below.
¹H-NMR (DMSO-d₆) δ: 1.99 (3H, s), 2.16 (3H, s), 4.45 (1H, d, J=13.3 Hz), 4.60 (1H, d, J=13.3 Hz), 6.11 (1H, t, J=6.4 Hz), 6.89-6.95 (3H, m), 7.14 (1H, dt, J=1.4, 7.2 Hz), 7.20-7.36 (4H, m), 7.59 (2H, td, J=2.5, 8.7 Hz), 8.55 (1H, s), 10.84 (1H, s).
MS (ESI-FTMS) m/z 404 [M+H]⁺.

### Working Example 36

### Production of 5-[4-(cyclohex-2-en-1-yloxy)phenyl]-5-[(3-methyl-2-oxopyridin-1(2H)-yl)methyl]imidazolidine-2,4-dione (I-36)

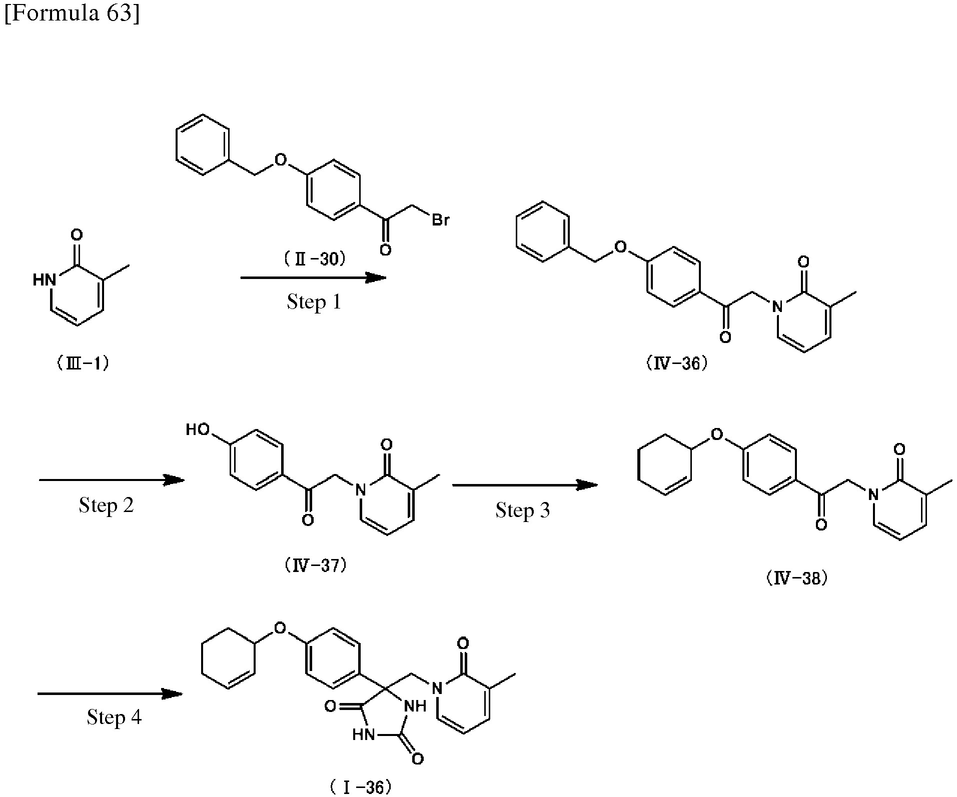

### Step 1

Cesium carbonate (2.2 g, 6.9 mmol) and 1-[4-(benzyloxy)phenyl]-2-bromoethanone (II-30) (2.0 g, 6.6 mmol) were added to a solution of compound (III-1) (681 mg, 6.2 mmol) in N,N-dimethylformamide (20 mL), and the resultant mixture was stirred for 2.5 hours at room temperature. Water was added under ice cooling. The precipitated solid was collected by filtration, and the resultant product was washed with water to obtain compound (IV-36) (amount 1.9 g, yield 89%).

### Step 2

Trifluoroacetic acid (10 mL) was added to a solution of this compound (IV-36) (1.3 g, 3.9 mmol) in chloroform (10 mL), and the resultant mixture was heated under reflux for 10 hours. After leaving to cool, the solvent was removed under reduced pressure and ethyl acetate was added. The precipitated solid was collected by filtration and washed with hexane to obtain compound (IV-37) (amount 813 mg, yield 86%).

### Step 3

3-Bromocyclohexene (50 µL, 0.43 mmol) was added to a suspension of this compound (IV-37) (100 mg, 0.41 mmol) and potassium carbonate (85 mg, 0.62 mmol) in N,N-dimethylformamide (5.0 mL), and the resultant mixture was stirred for 23 hours at room temperature. The reaction solution was diluted with water, and the resultant mixture was extracted with ethyl acetate. The organic layer was washed with saturated brine, and then dried over anhydrous magnesium sulfate. The solvent was removed under reduced pressure, and the resultant product was purified by column chromatography (silica gel) to obtain compound (IV-38) (amount 115 mg, yield 86%) as a yellow amorphous substance.

### Step 4

Compound (I-36) (amount 77 mg, yield 52%) was obtained as a colorless solid from compound (IV-38) based on the same production method as for compound (I-34). The physical properties are shown below.
¹H-NMR (DMSO-d₆) δ: 1.47-1.81 (3H, m), 1.85-2.16 (6H, m), 4.44 (1H, d, J=13.7 Hz), 4.53 (1H, d, J=13.3 Hz), 4.89 (1H, m), 5.79 (1H, m), 5.92 (1H, m), 6.11 (1H, t, J=6.9 Hz), 6.96-7.03 (2H, m), 7.17-7.32 (2H, m), 7.48-7.56 (2H, m), 8.51 (1H, d, J=1.4 Hz), 10.79 (1H, s).
MS (ESI-FTMS) m/z 394 [M+H]⁺.

### Working Example 37

### Production of 5-[4-(cyclohexyloxy)phenyl]-5-[(3-methyl-2-oxopyridin-1(2H)-yl)methyl]imidazolidine-2,4-dione (I-37)

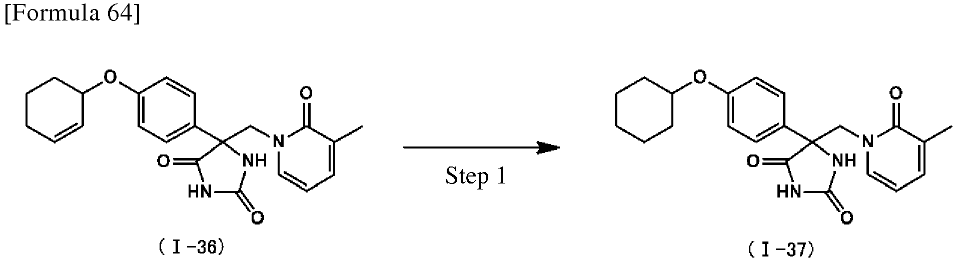

### Step 1

To a solution of compound (I-36) (50 mg, 0.13 mmol) in methanol (2.0 mL) was added 10% palladium carbon (20 mg), and the resultant mixture was stirred for 4 hours under a hydrogen atmosphere. The reaction solution was filtered through a pad of Celite, and the pad was washed with methanol. The solvent was removed under reduced pressure, and the resultant product was purified by column chromatography (silica gel) to obtain compound (I-37) (amount 36 mg, yield 72%) as a colorless solid. The physical properties are shown below.
¹H-NMR (DMSO-d₆) δ: 1.20-1.46 (5H, m), 1.48-1.58 (1H, m), 1.66-1.76 (2H, m), 1.87-1.95 (2H, m), 1.99 (3H, s), 4.32-4.40 (1H, m), 4.43 (1H, d, J=13.7 Hz), 4.56 (1H, d, J=13.7 Hz), 6.11 (1H, t, J=6.9 Hz), 6.97 (2H, d, J=8.7 Hz), 7.20-7.33 (2H, m), 7.50 (2H, d, J=8.7 Hz), 8.50 (1H, s), 10.78 (1H, s).
MS (ESI-FTMS) m/z 396 [M+H]⁺.

### Working Example 38

### Production of 5-[4-(cyclohex-2-en-l-yloxy)-1-methylphenyl]-5-[(3-methyl-2-oxopyridin-1(2H)-yl)methyl]imidazolidine-2,4-dione (I-38)

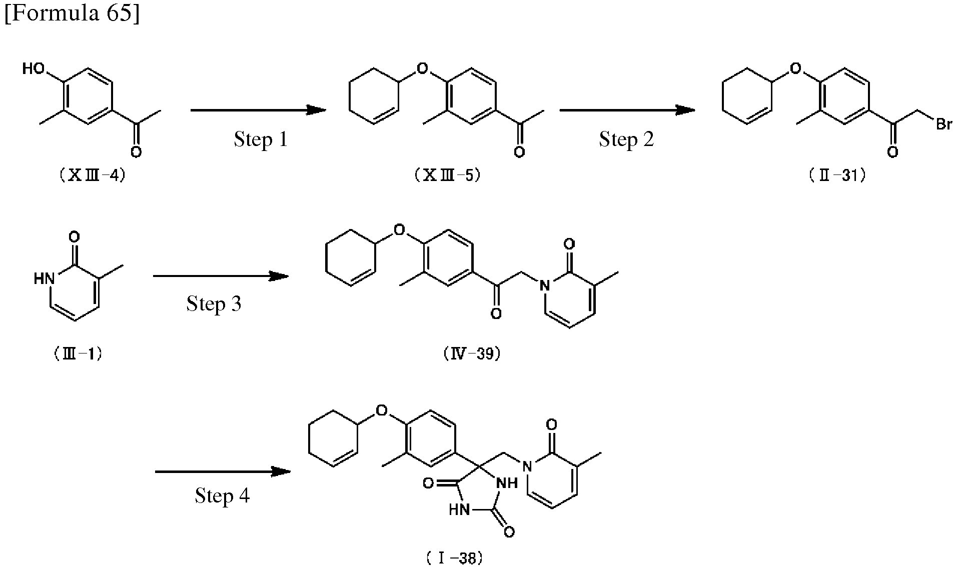

### Step 1

To a solution of 1-(4-hydroxy-3-methylphenyl)ethanone (XIII-4) (1.2 g, 8.0 mmol) in N,N-dimethylformamide (10 mL) was added 60% sodium hydride (384 mg, 9.6 mmol), and the resultant mixture was stirred for 30 minutes at room temperature. 3-Bromocyclohexene (1.5 g, 9.6 mmol) was added, and the resultant mixture was stirred overnight at room temperature. Ice water was added to stop the reaction, and the mixture was extracted with ethyl acetate. The organic layer was washed with water, and then dried over anhydrous sodium sulfate. The solvent was removed under reduced pressure, and the resultant product was purified by column chromatography (silica gel) to obtain compound (XIII-5) (amount 1.71 g, yield 88%).

### Step 2

Phenyltrimethylammonium tribromide (1.7 g, 4.0 mmol) was added to a solution of compound (XIII-5) (1.0 g, 4.1 mmol) in tetrahydrofuran (40 mL), and the resultant mixture was stirred for 2 hours at room temperature, and then heated under reflux for 4 hours. After leaving to cool, the precipitated solid was collected by filtration, and the filtrate solvent was removed to obtain a crude compound (II-31) (amount 1.2 g).

### Step 3

Cesium carbonate (1.5 g, 4.1 mmol) and the crude compound (II-31) (1.2 g) were added to a solution of compound (III-1) (407 mg, 3.7 mmol) in N,N-dimethylformamide (7.5 mL), and the resultant mixture was stirred overnight at room temperature. The reaction solution was diluted with water, and the resultant mixture was extracted with ethyl acetate. The organic layer was washed with saturated brine, and then dried over anhydrous sodium sulfate. The solvent was removed under reduced pressure, and the resultant product was purified by column chromatography (silica gel) to obtain compound (IV-39) (amount 449 mg, two steps, cumulative yield 33%).

### Step 4

An aqueous ammonia solution (28%, 1.5 mL) was added to a suspension of this compound (IV-39) (449 mg, 1.3 mmol), potassium cyanide (130 mg, 2.0 mmol), and ammonium carbonate (511 mg, 5.3 mmol) in ethanol (1.5 mL). The resultant mixture was sealed, and stirred at 100°C. After leaving to cool, water was added to the reaction solution, and the mixture was extracted with ethyl acetate. The organic layer was then washed with water, and dried over anhydrous sodium sulfate. The solvent was removed under reduced pressure to obtain compound (I-38) (amount 410 mg, yield 76%). The physical properties are shown below.
¹H-NMR (CDCl₃) δ: 1.58-1.97 (4H, m), 2.08-2.18 (2H, m), 2.13 (3H, s), 2.23 (3H, s), 4.20 (1H, d, J=13.7Hz), 4.78 (1H, m), 4.85 (1H, d, J=13.7Hz), 5.85 (1H, m), 5.96 (1H, m), 6.09 (1H, t, J=6.9Hz), 6.88 (1H, d, J=8.7Hz), 6.93 (1H, s), 7.14 (1H, d, J=6.9Hz), 7.20 (1H, d, J=6.9Hz), 7.34-7.41 (2H, m).
MS (ESI-FTMS) m/z 408 [M+H]⁺.

### Working Example 39

### Production of 5-[4-(cyclopentyloxy)-3-methylphenyl]-5-[(3-methyl-2-oxopyridin-1(2H)-yl)methyl]imidazolidine-2,4-dione (I-39)

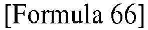

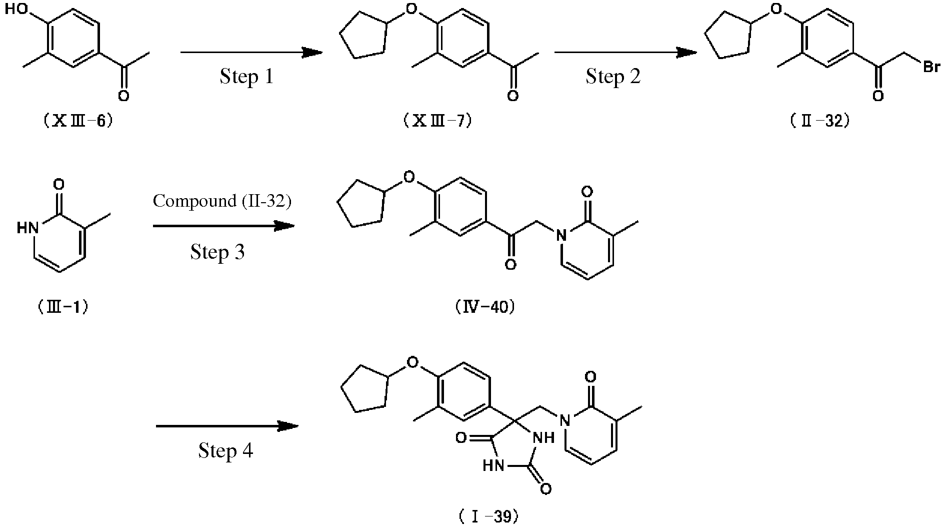

### Step 1

To a solution of compound (XIII-6) (800 mg, 5.3 mmol) in N,N-dimethylformamide (8.0 mL) was added 60% sodium hydride (235 mg, 5.9 mmol), and the resultant mixture was stirred at room temperature. Cyclopentyl iodide (1.2 g, 5.9 mmol) was added, the resultant mixture was stirred for 3 hours at 60°C, and then for 4 hours at 80°C. Ice water was added to stop the reaction, and the mixture was then extracted with ethyl acetate. The organic layer was washed with water, and dried over anhydrous sodium sulfate. The solvent was removed under reduced pressure, and the resultant product was purified by column chromatography (silica gel) to obtain compound (XIII-7) (amount 635 mg, yield 52%).

Compound (I-39) (amount 490 mg, three steps, cumulative yield 45%) was obtained from this compound (XIII-7) in three steps based on the same production method (step 2 to step 4 of Working Example 38) as for compound (I-38) of Working Example 38. The physical properties are shown below.
¹H-NMR (CDCl₃) δ: 1.57-1.94 (8H, m), 2.13 (3H, s), 2.19 (3H, s), 4.20 (1H, d, J=13.7Hz), 4.77 (1H, m), 4.85 (1H, d, J=13.7Hz), 6.08 (1H, t, J=6.9Hz), 6.81 (1H, d J=9.2Hz), 6.90 (1H, br s), 7.14 (1H, d, J=6.9Hz), 7.20 (1H, d, J=6.9Hz), 7.34-7.36 (1H, m), 7.37 (1H, s).
MS (ESI-FTMS) m/z 396 [M+H]⁺.

### Working Example 40

### Production of 5-(4-benzylphenyl)-5-[(3-methyl-2-oxopyridin-1(2H)-yl)methyl]imidazolidine-2,4-dione (I-40)

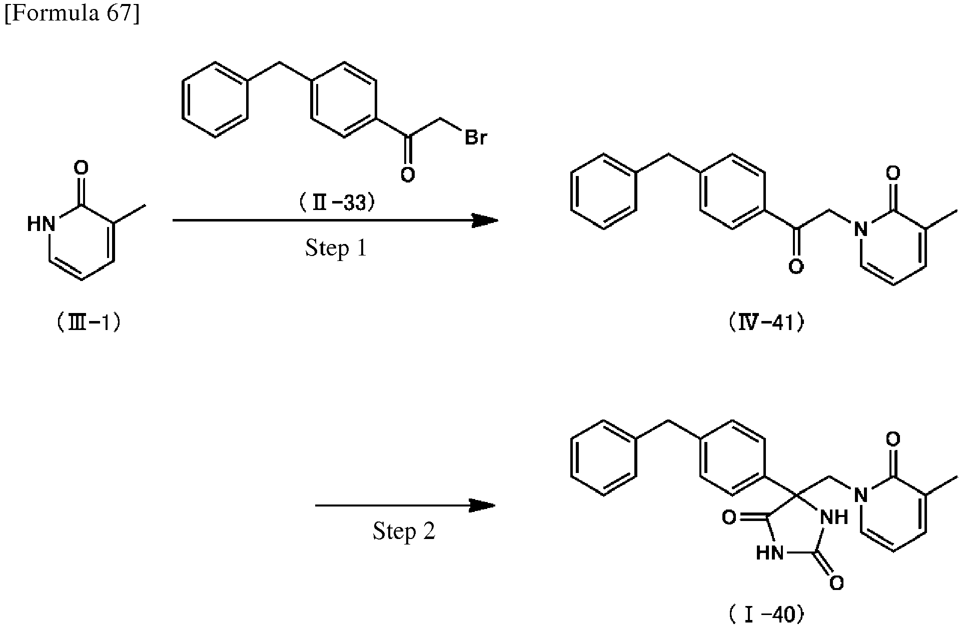

### Step 1

Cesium carbonate (590 mg, 1.8 mmol) and 1-(4-benzylphenyl)-2-bromoethanone (11-33) (500 mg, 1.7 mmol) were added to a solution of compound (III-1) (180 mg, 1.6 mmol) in N,N-dimethylformamide (5.0 mL), and the resultant mixture was stirred for 3 hours at room temperature. Water was added to the reaction solution. The precipitated solid was collected by filtration and washed with water to obtain compound (IV-41) (amount 475 mg, yield 91%).

### Step 2

An aqueous ammonia solution (28%, 1.5 mL) was added to a suspension of this compound (IV-41) (475 mg, 1.5 mmol), potassium cyanide (117 mg, 1.8 mmol), and ammonium carbonate (575 mg, 6.0 mmol) in ethanol (1.5 mL). The resultant mixture was sealed, and stirred for 64 hours at 100°C. After leaving to cool, water was added to the reaction solution, and the mixture was extracted with ethyl acetate. The organic layer was then washed with water, and dried over anhydrous sodium sulfate. The solvent was then removed under reduced pressure, and the resultant product was purified by column chromatography (silica gel) to obtain compound (I-40) (amount 466 mg, yield 80%) as a colorless solid. The physical properties are shown below.
¹H-NMR (DMSO-d₆) δ: 1.99 (3H, s), 3.95 (2H, s), 4.43 (1H, d, J=13.7 Hz), 4.59 (1H, d, J=13.7 Hz), 6.10 (1H, t, J=6.6 Hz), 7.15-7.31 (9H, m), 7.53 (2H, d, J=8.7 Hz), 8.52 (1H, s), 10.81 (1H, s).
MS (ESI-FTMS) m/z 388 [M+H]⁺.

### Working Example 41

### Production of 5-[4-(4-fluorobenzyl)phenyl]-5-[(3-methyl-2-oxopyridin-1(2H)-yl)methyl]imidazolidine-2,4-dione (I-41)

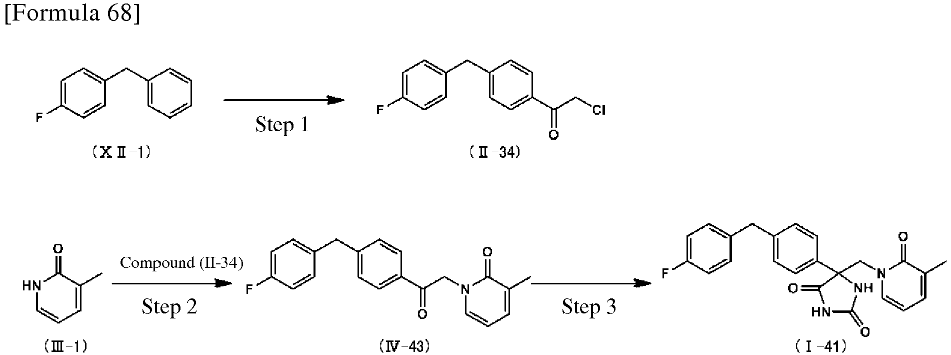

### Step 1

Under ice cooling, chloroacetyl chloride (427 µL, 5.4 mmol) and aluminium chloride (716 mg, 5.4 mmol) were added to a solution of 1-benzyl-4-fluorobenzene (XII-1) (1.0 g, 5.4 mmol) in dichloromethane (5.4 mL), and the resultant mixture was stirred for 15 minutes. After adding water to the reaction solution and extracting with chloroform, the mixture was successively washed with saturated sodium bicarbonate water and saturated brine, and dried over anhydrous sodium sulfate. The solvent was removed under reduced pressure to obtain a crude compound (II-34).

### Step 2

The crude compound (II-34) was dissolved in N,N-dimethylformamide (3.0 mL). After adding potassium carbonate (1.9 g, 13.4 mmol), a solution of compound (III-1) (586 mg, 5.4 mmol) in N,N-dimethylformamide (2.4 mL) was added dropwise, and the resultant mixture was stirred for 2 hours at room temperature. The reaction solution was diluted with water, and the resultant mixture was extracted with ethyl acetate. The organic layer was washed with saturated brine, and dried over sodium sulfate. The solvent was then removed under reduced pressure, and the resultant product was purified by column chromatography (silica gel) to obtain compound (IV-43) (amount 1.5 g, two steps, yield 84%).

### Step 3

Water (4.2 mL) was added to a suspension of this compound (IV-43) (1.4 g, 4.2 mmol), potassium cyanide (326 mg, 5.0 mmol), and ammonium carbonate (1.6 g, 16.7 mmol) in ethanol (4.2 mL). The resultant mixture was sealed, and stirred for 16 hours at 100°C. After leaving to cool, water was added to the reaction solution. The precipitated solid was collected by filtration, and washed with chloroform to obtain compound (I-41) (amount 1.6 g, yield 95%). The physical properties are shown below.
¹H-NMR (DMSO-d₆) δ: 1.99 (3H, s), 3.94 (2H, s), 4.44 (1H, d, J=13.3 Hz), 4.60 (1H, d, J=13.7 Hz), 6.10 (1H, t, J=6.7 Hz), 7.10 (2H, t, J=9.0 Hz), 7.20-7.32 (6H, m), 7.54 (2H, d, J=8.2 Hz), 8.51 (1H, s), 10.81 (1H, s).
MS (ESI-FTMS) m/z 406 [M+H]⁺.

### Working Example 42

### Production of 5-[4-(2H-tetrazol-5-yl)phenyl]-5-[(3-methyl-2-oxopyridin-1(2H)-yl)methyl]imidazolidine-2,4-dione (I-42)

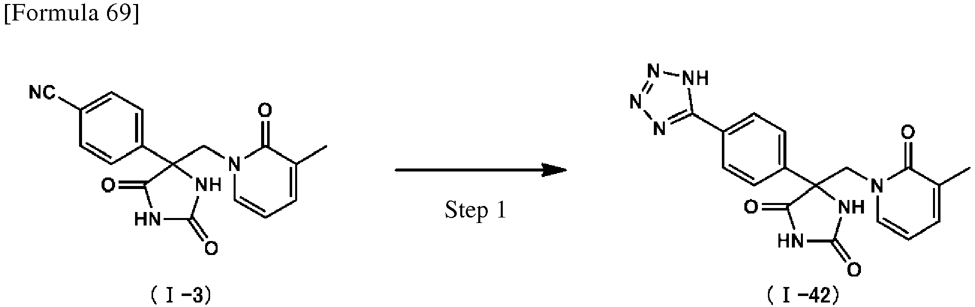

### Step 1

N,N-dimethylformamide (3.4 mL) was added to compound (I-3) (221 mg, 0.69 mmol), ammonium chloride (110 mg, 2.06 mmol), and sodium azide (124 mg, 2.06 mmol), and the resultant mixture was heated and stirred for 1 hour at 80°C, and for 12.5 hours at 110°C. After leaving the reaction solution to cool, 2 mol/L hydrochloric acid was added, and the precipitated solid was collected by filtration. The resultant solid was dissolved, and then purified by silica gel column chromatography to obtain compound (I-42) (amount 101 mg, yield 40%). The physical properties are shown below.
¹H-NMR (DMSO-d₆) δ: 2.00 (3H, s), 4.51 (1H, d, J=13.7 Hz), 4.72 (1H, d, J=13.7 Hz), 6.13 (1H, t, J=6.9 Hz), 7.30 (2H, d, J=6.4 Hz), 7.86 (2H, d, J=8.7 Hz), 8.10 (2H, d, J=8.7 Hz), 8.68 (1H, d, J=1.4 Hz), 10.56 (1H, s).
MS (ESI-FTMS) m/z: 366 [M+H]⁺.

### Working Example 43

### Production of 5-[(3-methyl-2-oxopyridin-1(2H)-yl)methyl]-5-(4-vinylphenyl)imidazolidine-2,4-dione (I-43)

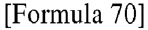

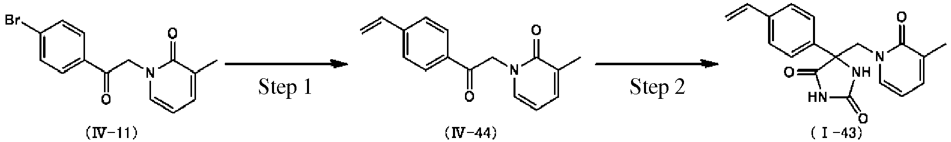

### Step 1

A suspension of compound (IV-11) (400 mg, 1.3 mmol), 4,4,5,5-tetramethyl-2-vinyl-1,3,2-dioxaborolane (242 mg, 1.6 mmol), and tripotassium phosphate (695 mg, 3.3 mmol) in 1,4-dioxane (6.5 mL) was degassed. Then, under an argon atmosphere, tetrakis(triphenylphosphine)palladium (76 mg, 0.066 mmol) was added, and the resultant mixture was heated and stirred at 90°C. After confirming the completion of the reaction by TLC, water was added to the reaction solution, and the resultant mixture was extracted with ethyl acetate. The organic layer was washed with saturated brine, and then dried over anhydrous sodium sulfate. The solvent was removed under reduced pressure, and the resultant residue was purified by column chromatography (silica gel) to obtain compound (IV-44) (amount 253 mg, yield 76%).

### Step 2

Compound (I-43) (amount 182 mg, yield 59%) was obtained from compound (IV-44) based on the same production method as for compound (I-17). The physical properties are shown below.
¹H-NMR (DMSO-d₆) δ: 2.00 (3H, s), 4.47 (1H, d, J=13.3 Hz), 4.62 (1H, d, J=13.7 Hz), 5.31 (1H, d, J=11.4 Hz), 5.89 (1H, d, J=17.9 Hz), 6.11 (1H, t, J=6.9 Hz), 6.75 (1H, dd, J=10.8, 17.7 Hz), 7.27 (2H, dd, J=6.7, 11.7 Hz), 7.53 (2H, d, J=8.7 Hz), 7.60 (2H, d, J=8.7 Hz), 8.58 (1H, s), 10.85 (1H, s).
MS (ESI-FTMS) m/z 324 [M+H]⁺.

### Working Example 44

### Production of 5-[(3-methyl-2-oxopyridin-1(2H)-yl)methyl]-5-(3,4,5-trifluorophenyl)imidazolidine-2,4-dione (I-44)

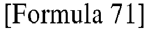

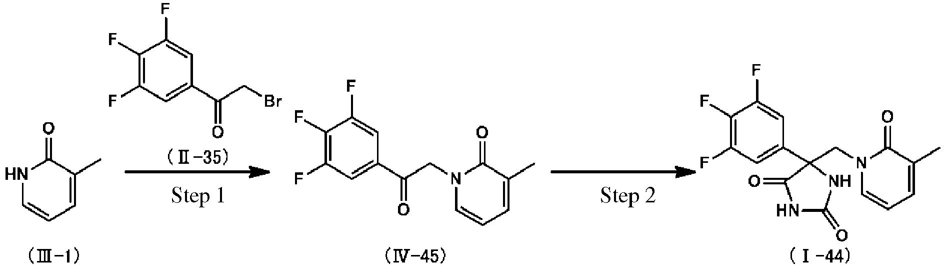

### Step 1

Compound (IV-45) (amount 189 mg, yield 19%) was obtained from compound (III-1) and 2-bromo-3',4',5'-trifluoroacetophenone (II-35) based on the same production method as for compound (IV-3).

### Step 2

Compound (I-44) (amount 158 mg, yield 70%) was obtained as a pale yellow solid from compound (IV-45) based on the same production method as for compound (I-11).
¹H-NMR (DMSO-d₆) δ: 1.98 (3H, s), 4.47 (1H, m), 4.61 (1H, d, J=13.7 Hz), 6.14 (1H, t, J=6.9 Hz), 7.22-7.33 (2H, m), 7.48-7.65 (2H, m), 8.64 (1H, br s), 11.07 (1H, br s).
MS (ESI-FTMS) m/z 352 [M+H]⁺.

### Working Example 45

Production of 5-[(3-methyl-2-oxopyridin-1(2H)-yl)methyl]-5-(4-propylphenyl)imidazolidine-2,4-dione (I-45)

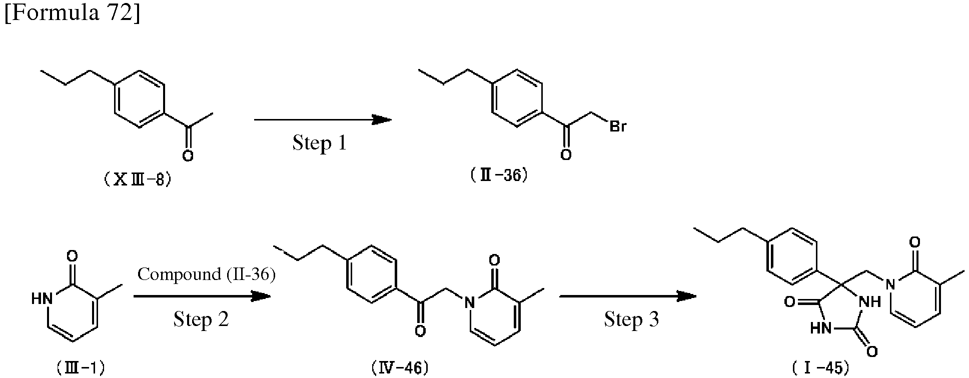

### Step 1

Compound (II-36) (amount 743 mg, yield > 99%) was obtained as a colorless oily substance from 4'-propylacetophenone (XIII-8) based on the same production method as for compound (II-31).

### Step 2

Compound (IV-46) (amount 294 mg, yield 69%) was obtained from compound (III-1) and compound (II-36) based on the same production method as for compound (IV-4).

### Step 3

Compound (I-45) (amount 21 mg, yield 5.8%) was obtained as a colorless solid from compound (IV-46) based on the same production method as for compound (I-24). The physical properties are shown below.
¹H-NMR (DMSO-d₆) δ: 0.88 (3H, t, J=7.3 Hz), 1.58 (2H, sext, J=7.3 Hz), 2.00 (3H, s), 2.56 (2H, t, J=7.6 Hz), 4.45 (1H, d, J=13.7 Hz), 4.60 (1H, d, J=13.7 Hz), 6.11 (1H, t, J=6.6 Hz), 7.18-7.31 (4H, m), 7.52 (2H, d, J=8.7 Hz), 8.52 (1H, s), 10.80 (1H, s).
MS (ESI-FTMS) m/z 340 [M+H]⁺.

### Working Example 46

### Production of 5-[(2-oxopyridin-1(2H)-yl)methyl]-5-phenylimidazolidine-2,4-dione (I-46)

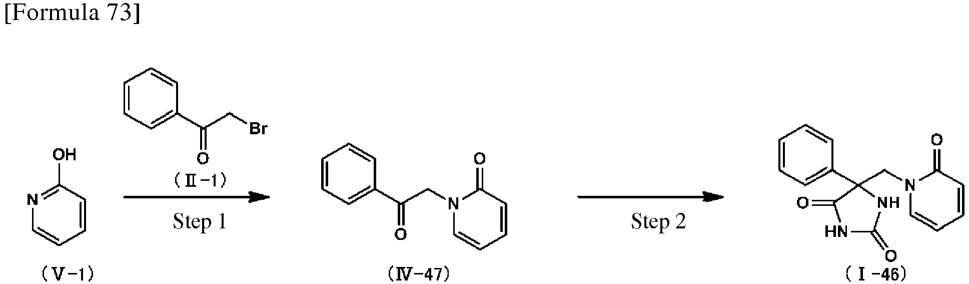

### Step 1

N,N-dimethylformamide (6.3 mL) was added to pyridin-2-ol (V-1) (300 mg, 3.15 mmol) and cesium carbonate (1.13 g, 3.47 mmol), and then compound (II-1) (691 mg, 3.47 mmol) was added, and the resultant mixture was stirred for 1 hour at room temperature. Water was added to the reaction solution, and the resultant mixture was extracted with ethyl acetate. The organic layer was washed with saturated brine, and then dried over anhydrous sodium sulfate. The solvent was removed under reduced pressure, and the resultant product was purified by silica gel column chromatography to obtain compound (IV-47) (amount 602 mg, yield 90%).

### Step 2

An autoclave was charged with this compound (IV-47) (300 mg, 1.41 mmol), potassium cyanide (110 mg, 1.69 mmol), ammonium carbonate (542 mg, 5.64 mmol), ethanol (1.4 mL), and water (1.4 mL). The autoclave was sealed, and the mixture was stirred for 21.5 hours at 100°C. Water was added to the reaction solution, and the precipitated solid was collected by filtration to obtain compound (I-46) (amount 226 mg, yield 57%). The physical properties are shown below. ¹H-NMR (DMSO-d₆) δ: 4.25 (1H, d, J=13.7 Hz), 4.65 (1H, d, J=13.7 Hz), 6.19 (1H, dt, J=1.4, 6.9 Hz), 6.39 (1H, dt, J=1.4, 8.7 Hz), 7.35-7.48 (5H, m), 7.60-7.66 (2H, m), 8.58 (1H, s), 10.85 (1H, s).
MS (ESI-FTMS) m/z: 284 [M+H]⁺.

### Working Example 47

### Production of 5-(4-methoxyphenyl)-5-[(2-oxopyridin-1(2H)-yl)methyl]imidazolidine-2,4-dione (I-47)

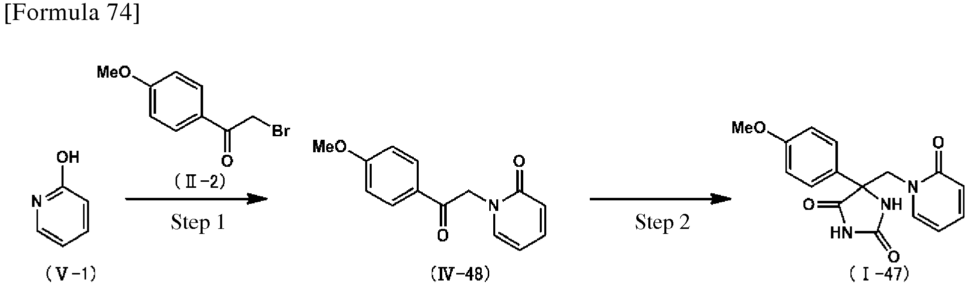

### Step 1

Compound (IV-48) (amount 384 mg, yield 90%) was obtained from compound (V-1) and compound (II-2) based on the same production method as for compound (IV-47).

### Step 2

An autoclave was charged with this compound (IV-48) (300 mg, 1.23 mmol), potassium cyanide (96 mg, 1.48 mmol), ammonium carbonate (474 mg, 4.93 mmol), ethanol (1.2 mL), and saturated ammonia water (1.2 mL). The autoclave was sealed, and the mixture was stirred for 63.75 hours at 100°C. Water was added to the reaction solution, the precipitated solid was collected by filtration, and the collected solid was washed with chloroform to obtain compound (I-47) (amount 112 mg, yield 29%).
¹H-NMR (DMSO-d₆) δ: 3.76 (3H, s), 4.42 (1H, d, J=13.7 Hz), 4.58 (1H, d, J=13.7 Hz), 6.19 (1H, dt, J=1.4, 6.9 Hz), 6.39 (1H, d, J=9.2 Hz), 6.99 (2H, d, J=9.2 Hz), 7.35-7.43 (2H, m), 7.53 (2H, d, J=9.2 Hz), 8.53 (1H, s), 10.81 (1H, s).
MS (ESI-FTMS) m/z: 314 [M+H]⁺.

### Working Example 48

### Production of 5-[(3-fluoro-2-oxopyridin-1(2H)-yl)methyl]-5-(4-fluorophenyl)-imidazolidine-2,4-dione (I-48)

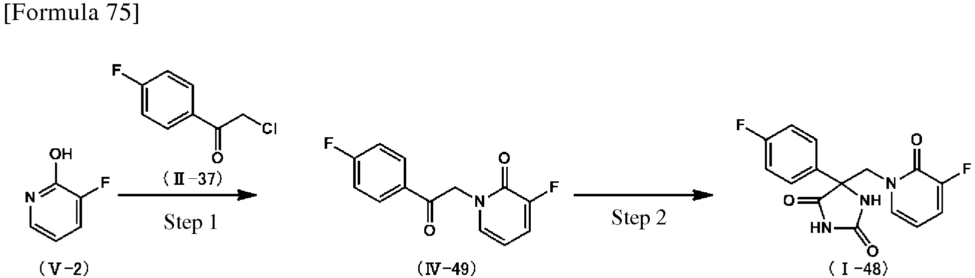

### Step 1

Acetone (6.3 mL) was added to 3-fluoropyridin-2-ol (V-2) (300 mg, 3.15 mmol) and cesium carbonate (1.13 g, 3.47 mmol), and then 2-chloro-1-(4-fluorophenyl)ethanone (II-37) (691 mg, 3.47 mmol) was added, and the resultant mixture was heated under reflux for 1 hour. The reaction solution was filtered, and then the solvent was removed under reduced pressure. The resultant product was purified by silica gel column chromatography to obtain compound (IV-49) (amount 395 mg, yield 90%).

### Step 2

Compound (I-48) (amount 166 mg, yield 43%) was obtained from compound (IV-49) based on the same production method as for compound (I-46). The physical properties are shown below.
¹H-NMR (DMSO-d₆) δ: 4.48 (1H, d, J=13.3 Hz), 4.72 (1H, d, J=13.7 Hz), 6.19 (1H, dt, J=4.6, 7.3 Hz), 7.19-7.24 (1H, m), 7.26-7.34 (2H, m), 7.40 (1H, ddd, J=1.6, 7.6, 9.4 Hz), 7.63-7.70 (2H, m), 8.77 (1H, s), 10.96 (1H, s).
MS (ESI-FTMS) m/z: 320 [M+H]⁺.

### Working Example 49

### Production of 5-([1,1'-biphenyl]-4-yl)-5-[(3-fluoro-2-oxopyridin-1(2H)-yl)methyl]imidazolidine-2,4-dione (I-49)

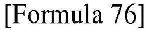

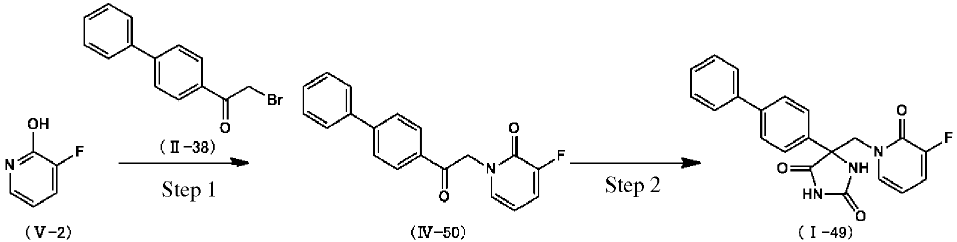

### Step 1

Dimethyl sulfoxide (3.6 mL) was added to compound (V-2) (226 mg, 2.00 mmol) and potassium carbonate (628 mg, 4.54 mmol), and then 1-([1,1'-biphenyl]-4-yl)-2-bromoethanone (II-38) (500 mg, 1.87 mmol) was added, and the resultant mixture was stirred for 1.25 hours at room temperature. Water was added to the reaction solution and the precipitated solid was collected by filtration to obtain compound (IV-50) (amount 665 mg, quantitative yield).

### Step 2

An autoclave was charged with this compound (IV-50) (300 mg, 0.98 mmol), potassium cyanide (76 mg, 1.17 mmol), ammonium carbonate (375 mg, 3.90 mmol), ethanol (0.98 mL), and water (0.98 mL). The autoclave was sealed, and the mixture was stirred for 43 hours at 100°C. Water was added to the reaction solution, the precipitated solid was collected by filtration, and the collected solid was washed with chloroform to obtain compound (I-49) (amount 176 mg, yield 48%).
¹H-NMR (DMSO-d₆) δ: 4.54 (1H, d, J=13.7 Hz), 4.79 (1H, d, J=13.7 Hz), 6.21 (1H, dt, J=4.6, 6.9 Hz), 7.26 (1H, d, J=7.3 Hz), 7.36-7.58 (4H, m), 7.67-7.84 (6H, m), 8.80 (1H, s), 10.96 (1H, s).
MS (ESI-FTMS) m/z: 378 [M+H]⁺.

### Working Example 50

### Production of 5-[(3-chloro-2-oxopyridin-1(2H)-yl)methyl]-5-phenylimidazolidine-2,4-dione (I-50)

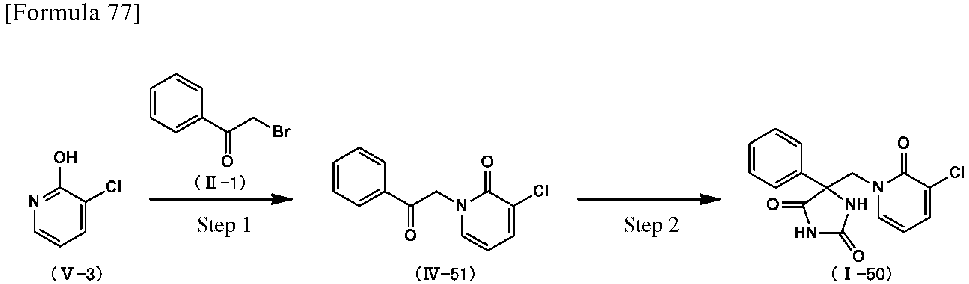

### Step 1

Compound (IV-51) (amount 529 mg, yield 92%) was obtained from 3-chloropyridin-2-ol (V-3) and compound (II-1) based on the same production method as for compound (IV-47).

### Step 2

An autoclave was charged with this compound (IV-51) (300 mg, 1.21 mmol), potassium cyanide (95 mg, 1.45 mmol), ammonium carbonate (465 mg, 4.84 mmol), ethanol (1.2 mL), and water (1.2 mL). The autoclave was sealed, and the mixture was stirred for 62.25 hours at 100°C. The solvent was removed under reduced pressure, and the resultant product was purified by silica gel column chromatography to obtain compound (I-50) (amount 323 mg, yield 84%). The physical properties are shown below.
¹H-NMR (DMSO-d₆) δ: 4.49 (1H, d, J=13.7 Hz), 4.76 (1H, d, J=13.7 Hz), 6.24 (1H, t, J=7.1 Hz), 7.36-7.49 (4H, m), 7.59-7.66 (2H, m), 7.75 (1H, dd, J=1.8, 7.3 Hz), 8.68 (1H, s), 10.91 (1H, s).
MS (ESI-FTMS) m/z: 318, 320 [M+H]⁺.

### Working Example 51

### Production of 5-(benzofuran-2-yl)-5-[(3-chloro-2-oxopyridin-1(2H)-yl)methyl]imidazolidine-2,4-dione (I-51)

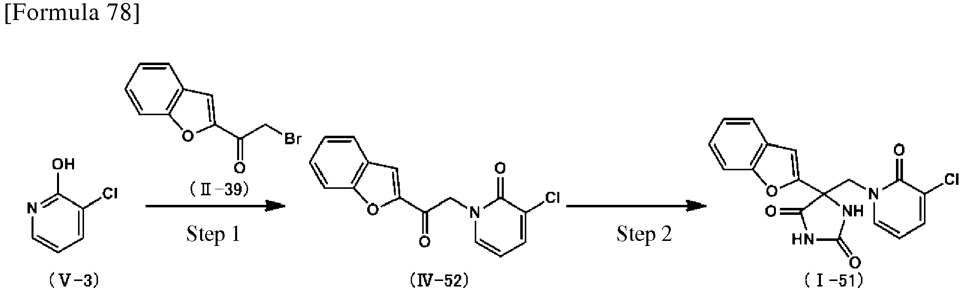

### Step 1

Compound (IV-52) (amount 277 mg, yield 50%) was obtained from compound (V-3) and 1-benzofuran-2-yl-2-bromoethanone (II-39) based on the same production method as for compound (IV-50).

An autoclave was charged with this compound (IV-52) (250 mg, 0.87 mmol), potassium cyanide (68 mg, 1.04 mmol), ammonium carbonate (334 mg, 3.48 mmol), ethanol (0.87 mL), and saturated ammonia water (0.87 mL). The autoclave was sealed, and the mixture was stirred for 64.75 hours at 100°C. The solvent was removed under reduced pressure, and the resultant product was purified by silica gel column chromatography to obtain compound (I-51) (amount 163 mg, yield 52%). The physical properties are shown below.
¹H-NMR (DMSO-d₆) δ: 4.81 (2H, s), 6.26 (1H, t, J=7.1 Hz), 7.14 (1H, s), 7.29 (1H, dt, J=0.9, 7.3 Hz), 7.37 (1H, dt, J=1.3, 7.3 Hz), 7.46 (1H, dd, J=2.1, 7.6 Hz), 7.60-7.72 (2H, m), 7.76 (1H, dd, J=1.8, 7.3 Hz), 8.77 (1H, s), 11.16 (1H, s).
MS (ESI-FTMS) m/z: 358, 360 [M+H]⁺.

### Working Example 52

### Production of 5-[(3-bromo-2-oxopyridin-1(2H)-yl)methyl]-5-(4-methoxyphenyl)imidazolidine-2,4-dione (I-52)

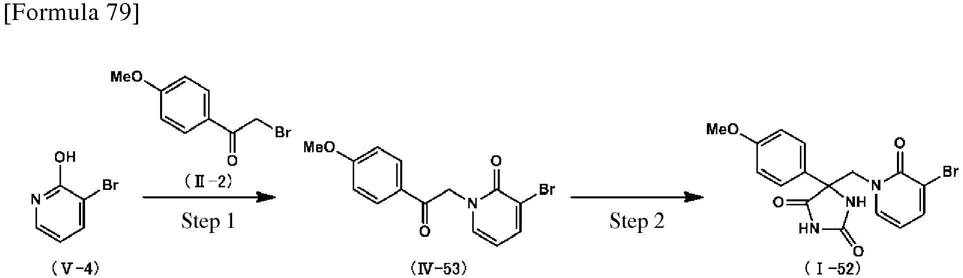

### Step 1

N,N-dimethylformamide (23 mL) was added to 3-bromopyridin-2-ol (V-4) (2.0 g, 11.49 mmol) and cesium carbonate (4.49 g, 13.79 mmol), and then compound (II-2) (2.90 g, 12.64 mmol) was added, and the resultant mixture was stirred for 1.75 hours at room temperature. Water was added to the reaction solution, and the resultant mixture was extracted with ethyl acetate. The organic layer was washed with saturated brine, and then dried over anhydrous sodium sulfate. The solvent was removed under reduced pressure, and the resultant product was purified by silica gel column chromatography to obtain compound (IV-53) (amount 2.09 g, yield 56%).

Compound (I-52) (amount 144 mg, yield 39%) was obtained from compound (IV-53) based on the same production method as for compound (I-47). The physical properties are shown below.
¹H-NMR (DMSO-d₆) δ: 3.76 (3H, s), 4.46 (1H, d, J=13.7 Hz), 4.69 (1H, d, J=13.7 Hz), 6.17 (1H, t, J=6.9 Hz), 6.99 (2H, d, J=9.2 Hz), 7.41 (1H, dd, J=1.6, 6.6 Hz), 7.53 (2H, d, J=8.7 Hz), 7.91 (1H, dd, J=1.8, 7.3 Hz), 8.68 (1H, s), 10.86 (1H, s).
MS (ESI-FTMS) m/z: 392, 394 [M+H]⁺.

### Working Example 53

### Production of 5-[(3-ethyl-2-oxopyridin-1(2H)-yl)methyl]-5-(4-methoxyphenyl)imidazolidine-2,4-dione (I-53)

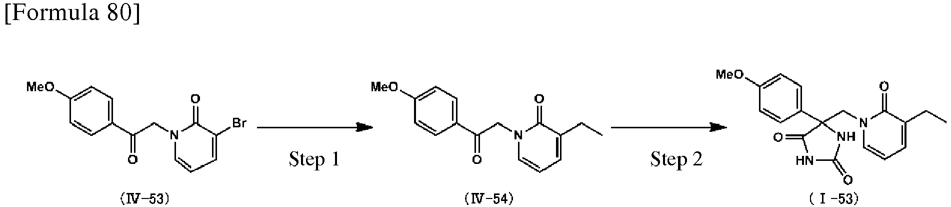

### Step 1

A suspension of compound (IV-53) (500 mg, 1.55 mmol), ethyl boronic acid (126 mg, 1.71 mmol), and tripotassium phosphate (822 mg, 3.88 mmol) in 1,4-dioxane (7.8 mL) was degassed. Under an argon atmosphere, tetrakis(triphenylphosphine)palladium (90 mg, 0.078 mmol) was added, and the resultant mixture was heated and stirred for 17 hours at 90°C. Water was added to the reaction solution, and the resultant mixture was extracted with ethyl acetate. The organic layer was washed with saturated brine, and then dried over anhydrous sodium sulfate. The solvent was removed under reduced pressure, and the resultant product was purified by silica gel column chromatography to obtain compound (IV-54) (amount 286 mg, yield 68%).

### Step 2

Compound (I-53) (amount 113 mg, yield 31%) was obtained from compound (IV-54) based on the same production method as for compound (I-47). The physical properties are shown below.
¹H-NMR (DMSO-d₆) δ: 1.07 (3H, t, J=7.3 Hz), 2.40 (2H, q, J=7.3 Hz), 3.77 (3H, s), 4.46 (1H, d, J=13.7 Hz), 4.56 (1H, d, J=13.7 Hz), 6.14 (1H, t, J=6.9 Hz), 6.95-7.02 (2H, m), 7.20-7.28 (2H, m), 7.50-7.58 (2H, m), 8.49 (1H, s), 10.80 (1H, s).
MS (ESI-FTMS) m/z: 342 [M+H]⁺.

### Working Example 54

### Production of 5-(3-hydroxyphenyl)-5- [(3-methyl- 2-oxopyridin-l (2H)-yl)methyl]imidazolidine-2,4-dione (I-54)

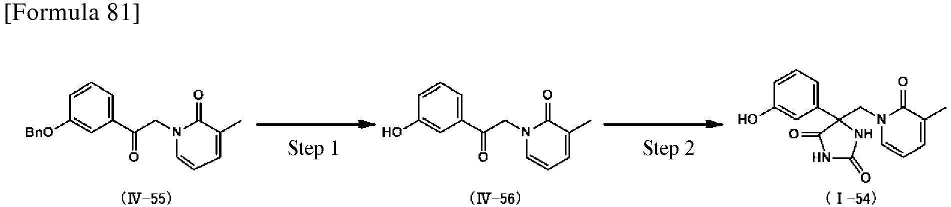

### Step 1

Trifluoroacetic acid (1 mL) was added to a solution of 1-(2-(3-(benzyloxy)phenyl)-2-oxoethyl)-3-methylpyridin-2-(1H)-one (IV-55) (361 mg, 1.08 mmol) in chloroform (1 mL), and the resultant mixture was stirred for 14.5 hours at room temperature, and then heated under reflux for 23.5 hours. A saturated aqueous solution of sodium bicarbonate was added to the reaction solution, and the resultant mixture was extracted with ethyl acetate. The organic layer was washed with saturated brine, and then dried over anhydrous sodium sulfate. The solvent was removed under reduced pressure, chloroform was added, and the precipitated solid was collected by filtration to obtain compound (IV-56) (amount 116 mg, yield 44%).

### Step 2

This compound (IV-56) (100 mg, 0.41 mmol), potassium cyanide (32 mg, 0.49 mmol), ammonium carbonate (158 mg, 1.64 mmol), ethanol (0.41 mL), and saturated ammonia water (0.41 mL) were added together. The resultant mixture was sealed, and stirred for 65 hours at 100°C. The solvent was removed under reduced pressure, and the resultant product was purified by silica gel column chromatography to obtain compound (I-54) (amount 93 mg, yield 72%). The physical properties are shown below.
¹H-NMR (DMSO-d₆) δ: 2.00 (3H, s), 4.42 (1H, d, J=13.3 Hz), 4.59 (1H, d, J=13.3 Hz), 6.11 (1H, t, J=6.9 Hz), 6.76 (1H, dd, J=1.2, 8.0 Hz), 7.00-7.07 (2H, m), 7.18-7.32 (3H, m), 8.49 (1H, s), 9.60 (1H, s), 10.79 (1H, s).
MS (ESI-FTMS) m/z: 378 [M+H]⁺.

Compound I-55 was synthesized based on the same method as for compound I-7. The structure and the physical properties of the compound are shown in Table 1.

Compounds I-145, I-146, I-147, I-148, I-151, I-153, I-154, I-155, I-156, and I-157 were synthesized based on the same method as for compound I-10. The structure and the physical properties of each compound are shown in Table 12 or Table 13.

Compounds I-56 and I-57 were synthesized based on the same method as for compound I-11. The structure and the physical properties of each compound are shown in Table 1.

Compounds I-59 and I-77 were synthesized based on the same method as for compound I-15. The structure and the physical properties of each compound are shown in Table 1 or Table 3.

Compound I-60 was synthesized based on the same method as for compound I-16. The structure and the physical properties of the compound are shown in Table 1.

Compound I-61 was synthesized based on the same method as for compound I-18. The structure and the physical properties of the compound are shown in Table 1.

Compounds I-69 and I-70 were synthesized based on the same method as for compound I-19. The structure and the physical properties of each compound are shown in Table 2.

Compound I-105 was synthesized based on the same method as for compound I-20. The structure and the physical properties of the compound are shown in Table 7.

Compounds I-65 and I-67 were synthesized based on the same method as for compound I-21. The structure and the physical properties of each compound are shown in Table 2.

Compounds I-76 and I-120 were synthesized based on the same method as for compound I-22. The structure and the physical properties of each compound are shown in Table 3 or Table 8.

Compounds I-66 and I-119 were synthesized based on the same method as for compound I-24. The structure and the physical properties of each compound are shown in Table 2 or Table 8.

Compounds I-78, I-79, I-89, I-125, I-126, I-127, and I-128 were synthesized based on the same method as for compound I-25. The structure and the physical properties of each compound are shown in Table 3, Table 5, or Table 9.

Compounds I-92, I-93, I-94, and I-95 were synthesized based on the same method as for compound I-31. The structure and the physical properties of each compound are shown in Table 5.

Compounds I-62, I-72, I-73, I-74, I-75, I-90, I-91, I-96, and I-109 were synthesized based on the same method as for compound I-32. The structure and the physical properties of each compound are shown in Table 1, Table 2, Table 3, Table 5, or Table 7.

Compounds I-58, I-63, I-71, I-106, and I-110 were synthesized based on the same method as for compound I-33. The structure and the physical properties of each compound are shown in Table 1, Table 2, or Table 7.

Compounds I-64, I-68, I-80, I-81, I-86, I-97, I-100, and I-113 were synthesized based on the same method as for compound I-34. The structure and the physical properties of each compound are shown in Table 2, Table 3, Table 4, Table 6, or Table 8.

Compounds I-82, I-83, I-84, I-85, I-87, I-98, I-99, I-101, I-102, I-103, I-104, I-111, I-112, I-114, I-122, and I-123 were synthesized based on the same method as for compound I-35. The structure and the physical properties of each compound are shown in Table 4, Table 6, Table 7, Table 8, or Table 9.

Compound I-88 was synthesized based on the same method as for compound I-36. The structure and the physical properties of the compound are shown in Table 4.

Compounds I-107, I-115, I-116, I-117, I-118, and I-131 were synthesized based on the same method as for compound I-37. The structure and the physical properties of each compound are shown in Table 7, Table 8, or Table 10.

Compounds I-124 and I-129 were synthesized based on the same method as for compound I-39. The structure and the physical properties of each compound are shown in Table 9 or Table 10.

Compounds I-149, I-150, and I-152 were synthesized based on the same method as for compound I-40. The structure and the physical properties of each compound are shown in Table 13.

Compound I-130 was synthesized based on the same method as for compound I-41. The structure and the physical properties of the compound are shown in Table 10.

Compound I-108 was synthesized based on the same method as for compound I-43. The structure and the physical properties of the compound are shown in Table 7.

Compound I-121 was synthesized based on the same method as for compound I-45. The structure and the physical properties of the compound are shown in Table 9.

Compounds I-132, I-133, I-134, I-137, I-138, I-139, and I-140 were synthesized based on the same method as for compound I-46. The structure and the physical properties of each compound are shown in Table 11 or Table 12.

Compounds I-141 and I-142 were synthesized based on the same method as for compound I-47. The structure and the physical properties of each compound are shown in Table 12.

Compound I-135 was synthesized based on the same method as for compound I-49. The structure and the physical properties of the compound are shown in Table 11.

Compounds I-136 and I-143 were synthesized based on the same method as for compound I-50. The structure and the physical properties of each compound are shown in Table 11 or Table 12.

Compound I-144 was synthesized based on the same method as for compound I-53. The structure and the physical properties of the compound are shown in Table 12.

[Table 1]

**Table 1**

| Compound | Structure | ¹H-NMR | MS (M+H)⁺ |
|---|---|---|---|
| I-55 | 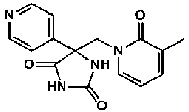 | (DMSO-d₆) δ: 1.98 (3H, s), 4.47 (1H, d, J=13.7 Hz), 4.66 (1H, d, J=13.7 Hz), 6.13 (1H, t, J=6.9 Hz), 7.24-7.32 (2H, m), 7.60-7.64 (2H, m), 8.60-8.66 (2H, m), 8.73 (1H, d, J=1.4 Hz), 11.01 (1H, s). | 299 |
| I-56 | 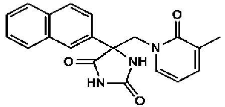 | (DMSO-d₆) δ: 2.00 (3H, s), 4.57 (1H, d, J=13.3 Hz), 4.78 (1H, d, J=13.7 Hz), 6.13 (1H, t, J=6.9 Hz), 7.30 (2H, t, J=6.4 Hz), 7.52-7.60 (2H, m), 7.79 (1H, dd, J=1.8, 8.7 Hz), 7.91-8.01 (3H, m), 8.16 (1H, s), 8.69 (1H, s), 10.89 (1H, s). | 348 |
| I-57 | 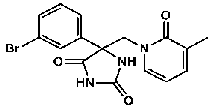 | (DMSO-d₆) δ: 1.99 (3H, s), 4.46 (1H, d, J=13.7 Hz), 4.62 (1H, d, J=13.7 Hz), 6.13 (1H, t, J=6.9 Hz), 7.27 (1H, d, J=7.8 Hz), 7.29 (1H, d, J=7.8 Hz), 7.41 (1H, t, J=7.8 Hz), 7.58-7.68 (2H, m), 7.83 (1H, t, J=1.8 Hz), 8.67 (1H, s), 10.96 (1 H, s). | 376 378 |
| I-58 | 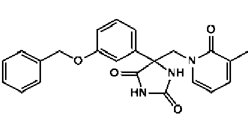 | (DMSO-d₆) δ: 2.00 (3H, s), 4.47 (1H, d, J=13.3 Hz), 4.60 (1H, d, J=13.7 Hz), 5.12 (2H, s), 6.12 (1H, t, J=6.7 Hz), 7.03 (1H, dd, J=2.0, 8.0 Hz), 7.18-7.54 (10H, m), 8.59 (1H, s), 10.87 (1H, s). | 404 |
| I-59 | 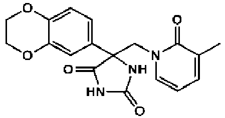 | (DMSO-d₆) δ : 2.00 (3H, s), 4.25 (4H, s), 4.43 (1H, d, J=13.7 Hz), 4.52 (1H, d, J=13.7 Hz), 6.11 (1H, t, J=6.9 Hz), 6.90 (1H, d, J=8.7 Hz), 7.07 (1H, dd, J=2.3, 8.7 Hz), 7.13 (1H, d, J=2.3 Hz), 7.19-7.32 (2H, m), 8.50 (1H, s), 10.73 (1H, s). | 356 |
| I-60 | 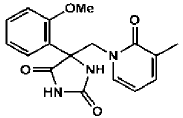 | (DMSO-d₆) δ : 1.99 (3H, s), 3.78 (3H, s), 4.42 (1H, d, J=13.3 Hz), 4.89 (1H, d, J=13.3 Hz), 6.12 (1H, t, J=6.9 Hz), 7.00 (1H, dt, J=1.4, 7.3 Hz), 7.11 (1H, m), 7.23 (1H, dd, J=1.4, 6.9 Hz), 7.29 (1H, m), 7.40 (1H, m), 7.51 (1H, dd, J=1.4, 7.8 Hz), 7.73 (1H, s), 10.72 (1H, s). | 328 |
| I-61 | 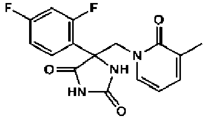 | (DMSO-d₆) δ: 1.99 (3H, s), 4.68 (1H, d, J=13.3 Hz), 4.78 (1H, d, J=13.3 Hz), 6.14 (1H, t, J=6.9 Hz), 7.18 (1H, dt, J=2.7, 8.7 Hz), 7.27 (1H, d, J=6.9 Hz), 7.31 (1H, d, J=6.4 Hz), 7.38 (1H, ddd, J=2.7, 9.2, 11.9 Hz), 7.64-7.72 (1H, m), 8.26 (1H, s), 10.93 (1H, s). | 334 |
| I-62 | 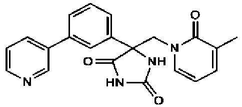 | (DMSO-d₆ δ: 2.00 (3H, s), 4.59 (1H, d, J=13.7 Hz), 4.67 (1H, d, J=13.7 Hz), 6.13 (1H, t, J=6.9 Hz), 7.30 (2H, d, J=6.9 Hz), 7.51-7.60 (2H, m), 7.71 (1H, d, J=7.8 Hz), 7.76 (1H, d, J=7.8 Hz), 7.99 (1H, t, J=1.6 Hz), 8.10-8.14 (1H, m), 8.61 (1H, dd, J=1.6, 4.8 Hz), 8.68 (1H, s), 8.95 (1H, dd, J=0.9, 2.3 Hz), 10.92 (1H, s). | 375 |
| I-63 | 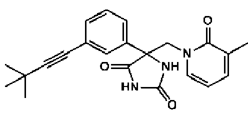 | (DMSO-d₆) δ: 1.31 (9H, s), 1.99 (3H, s), 4.42 (1H, d, J=13.7 Hz), 4.63 (1H, d, J=13.7 Hz), 6.12 (1H, t, J=6.7 Hz), 7.23-7.31 (2H, m), 7.32-7.44 (2H, m), 7.56-7.74 (2H, m), 8.60 (1H, s), 10.89 (1H, s). | 378 |

[Table 2]

**Table 2**

| Compound | Structure | ¹H-NMR | MS (M+H)⁺ |
|---|---|---|---|
| I-64 | 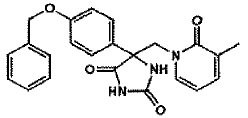 | (DMSO-d₆) δ : 2.00 (3H, s), 4.36 (1H, d, J=13.7 Hz), 4.57 (1H, d, J=13.7 Hz), 5.12 (2H, s), 6.11 (1H, t, J=6.9 Hz), 7.07 (2H, d, J=9.2 Hz), 7.24 (1H, d, J=6.9 Hz), 7.28 (1H, d, J=6.0 Hz), 7.33 (1H, t, J=6.9 Hz), 7.39 (2H, t, J=7.3 Hz), 7.42-7.48 (2H, m), 7.53 (2H, d, J=8.7 Hz), 8.52 (1H, s), 10.80 (1H, s). | 404 |
| I-65 | 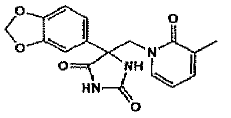 | (DMSO-d₆) δ 2.00 (3H, s), 4.44 (1H, d, J=13.7 Hz), 4.58 (1H, d, J=13.7 Hz), 6.04 (2H, s), 6.11 (1H, t, J=6.6 Hz), 6.96 (1H, d, J=8.2 Hz), 7.10 (1H, dd, J=2.3, 8.2 Hz), 7.18-7.31 (3H, m), 8.51 (1H, s), 10.83 (1H, s). | 342 |
| I-66 | 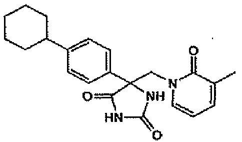 | (DMSO-d₆) δ : 1.17-1.47 (5H, m), 1.66-1.84 (6H, m), 2.00 (3H, s), 4.45 (1H, d, J=13.7 Hz), 4.60 (1H, d, J=13.7 Hz), 6.12 (1H, t, J=6.9 Hz), 7.23-7.31 (4H, m), 7.52 (2H, d, J=8.7 Hz), 8.51 (1H, s), 10.79 (1 H, s). | 380 |
| I-67 | 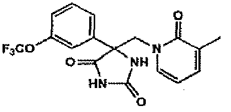 | (DMSO-d₆) δ: 1.98 (3H, s), 4.48 (1H, d, J=13.7 Hz), 4.64 (1H, d, J=13.7 Hz), 6.11 (1H, t, J=6.6 Hz), 7.24-7.33 (2H, m), 7.40 (1H, m), 7.55-7.63 (2H, m), 7.68 (1H, m), 8.70 (1H, s), 10.97 (1H, s). | 382 |
| I-68 | 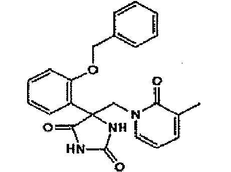 | (DMSO-d₆) δ : 1.99 (3H, s), 4.52 (1H, d, J=12.8 Hz), 4.90 (1H, d, J=13.3 Hz), 5.20 (2H, s), 6.04 (1H,t,J=13.7 Hz), 7.00 (1H,m), 7.09 (1H, m), 7.16 (1H, dd, J=1.4, 6.9 Hz), 7.24-7.40 (5H, m), 7.48-7.52 (2H, m), 7.55 (1H, dd, J=1.4, 7.8 Hz), 7.70 (1H, s), 10.66 (1H, s). | 404 |
| I-89 | 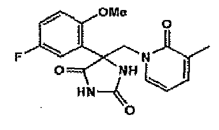 | (DMSO-d₆) δ: 1.99 (3H, s), 3.76 (3H, s), 4.44 (1H, d, J=13.3 Hz), 4.83 (1H, d, J=13.3 Hz), 6.12 (1H, t, J=6.9 Hz), 7.13 (1H, dd, J=4.6, 9.2 Hz), 7.19-7.32 (3H, m), 7.39 (1H, dd, J=2.7, 10.1 Hz), 7.83 (1H, s), 10.79 (1H, s). | 346 |
| I-70 | 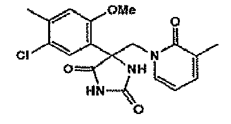 | (DMSO-d₆) δ : 1.99 (3H, s), 2.34 (3H, s), 3.78 (3H, s), 4.41 (1H, d, J=13.3 Hz), 4.81 (1H, d, J=13.3 Hz), 6.12 (1H, t, J=6.9 Hz), 7.14 (1H, s), 7.21 (1H, d, J=6.9 Hz), 7.29 (1H, d, J=6.0 Hz), 7.49 (1H, s), 7.79 (1H,s), 10.80 (1H,s). | 376 |
| I-71 | 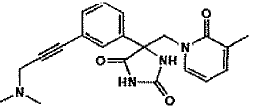 | (DMSO-d₆) δ: 1.99 (3H, s), 2.26 (6H, s), 3.47 (2H, s), 4.46 (1H, d, J=13.3 Hz), 4.63 (1H, d, J=13.7 Hz), 6.12 (1H, t, J=6.9 Hz), 7.24-7.31 (2H, m), 7.42-7.48 (2H, m), 7.61-7.67 (1H, m), 7.71 (1H, d, J=0.9 Hz), 8.63 (1H, s), 10.91 (1H, s). | 379 |
| I-72 | 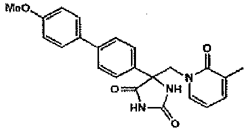 | (DMSO-d₆) δ: 2.01 (3H, s), 3.80 (3H, s), 4.51 (1H, d, J=13.7 Hz), 4.66 (1H, d, J=13.7 Hz), 6.13 (1H, t, J=6.9 Hz), 7.04 (2H, d, J=9.2 Hz), 7.26-7.32 (2H, m), 7.64 (2H, d, J=8.7 Hz), 7.69 (4H, s), 8.62 (1H, s), 10.82 (1H, s). | 404 |

[Table 3]

**Table 3**

| Compound | Structure | ¹H-NMR | MS (M+H)⁺ |
|---|---|---|---|
| I-73 | 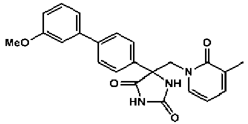 | (DMSO-d₆) δ: 2.01 (3H, s), 3.83 (3H, s), 4.51 (1H, d, J=13.7 Hz), 4.67 (1H, d, J=13.7 Hz), 6.14 (1H, t, J=6.7 Hz), 6.96 (1H, dd, J=2.3, 8.2 Hz), 7.19-7.33 (5H, m), 7.71 (2H, d, J=8.7 Hz), 7.75 (2H, d, J=8.7 Hz), 8.64 (1H, s), 10.87 (1H, s). | 404 |
| I-74 | 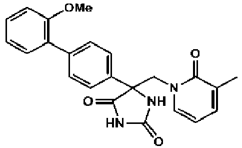 | (DMSO-d₆) δ: 2.01 (3H, s), 3.77 (3H, s), 4.52 (1H, d, J=13.7 Hz), 4.67 (1H, d, J=13.3 Hz), 6.14 (1H, t, J=6.7 Hz), 7.04 (1H, t, J=7.3 Hz), 7.12 (1H, d, J=7.8 Hz), 7.27-7.40 (4H, m), 7.54 (2H, d, J=8.7 Hz), 7.66 (2H, d, J=8.2 Hz), 8.60 (1H, s), 10.84 (1H, s). | 404 |
| I-75 | 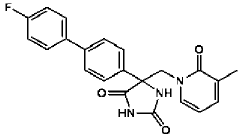 | (DMSO-d₆) δ: 2.01 (3H, s), 4.51 (1H, d, J=13.3 Hz), 4.66 (1H, d, J=13.3 Hz), 6.13 (1H, t, J=6.9 Hz), 7.27-7.35 (4H, m), 7.69-7.78 (6H, m), 8.63 (1H, s), 10.84 (1H, s). | 392 |
| I-76 | 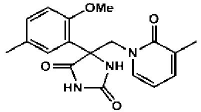 | (DMSO-d₆) δ: 1.99 (3H, s), 2.27 (3H, s), 3.74 (3H, s), 4.41 (1H, d, J=13.3 Hz), 4.87 (1H, d, J=13.3 Hz), 6.12 (1H, t, J=6.4 Hz), 7.00 (1H, d, J=8.7 Hz), 7.16-7.25 (2H, m), 7.29 (1H, m), 7.32 (1H, d, J=1.8 Hz), 7.69 (1H, s), 10.71 (1H, s). | 342 |
| I-77 | 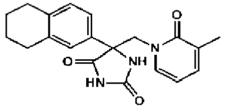 | (DMSO-d₆) δ: 1.68-1.79 (4H, m), 2.00 (3H, s), 2.68-2.76 (4H, m), 4.43 (1H, d, J=13.7 Hz), 4.57 (1H, d, J=13.7 Hz), 6.12 (1H, t, J=6.9 Hz), 7.10 (1H, d, J=8.7 Hz), 7.23-7.34 (4H, m), 8.46 (1H, s), 10.7 (1H, s). | 352 |
| I-78 | 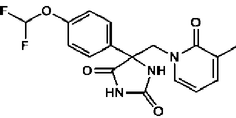 | (DMSO-d₆) δ: 1.99 (3H, s), 4.46 (1H, d, J=13.7 Hz), 4.62 (1H, d, J=13.7 Hz), 6.12 (1H, t, J=6.9 Hz), ,7.22-7.31 (4H, m), 7.27 (1 H, t, J=74.0 Hz), 7.68 (2H, d, J=8.7 Hz), 8.60 (1H, s), 10.88 (1H, s). | 364 |
| I-79 | 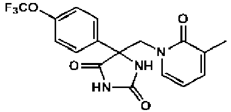 | (DMSO-d₆) δ: 1.99 (3H, s), 4.46 (1H, d, J=13.3 Hz), 4.65 (1H, d, J=13.7 Hz), 6.12 (1H, t, J=6.7 Hz), 7.24-7.32 (2H, m), 7.46 (2H, d, J=7.8 Hz), 7.76 (2H, d, J=9.2 Hz), 8.65 (1H, s), 10.93 (1H, s). | 382 |
| I-80 | 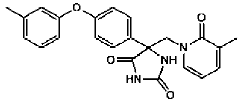 | (DMSO-d₆) δ: 2.00 (3H, s), 2.29 (3H, s), 4.46 (1H, d, J=13.7 Hz), 4.61 (1H, d, =13.7 Hz), 6.12 (1H, t, J=6.9 Hz), 6.80 (1H, dd, J=2.5, 8.2 Hz), 6.85 (1H, s), 6.98 (1H, m), 7.04 (2H, td, J=2.5, 8.7 Hz), 7.23-7.31 (3H, m), 7.62 (2H, td, J=2.5, 9.2 Hz), 8.57 (1H, d, J=1.4 Hz), 10.86 (1H, s). | 404 |

[Table 4]

**Table 4**

| Compound | Structure | ¹H-NMR | MS (M+H)⁺ |
|---|---|---|---|
| I-81 | 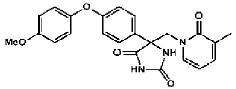 | (DMSO-d₆) δ: 1.99 (3H, s), 3.76 (3H, s), 4.46 (1H, d, J=13.7 Hz), 4.59 (1H, d, J=13.7 Hz), 6.12 (1H, t, J=6.9 Hz), 6.93-7.04 (6H, m), 7.23-7.31 (2H, m), 7.59 (2H, td, J=2.5, 9.2 Hz), 8.55 (1H, s), 10.83 (1H, s). | 420 |
| I-82 | 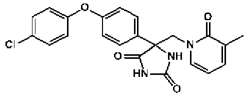 | (DMSO-d₆) δ: 1.99 (3H, s), 4.46 (1H, d, J=13.7 Hz), 4.61 (1H, d, J=13.7 Hz), 6.12 (1H, t, J=6.4 Hz), 7.04 (2H, d, J=8.7 Hz), 7.10 (2H, d, J=8.7 Hz), 7.26 (1H, d, J=6.9 Hz), 7.29 (1H, d, J=6.4 Hz), 7.44 (2H, d, J=9.2 Hz), 7.65 (2H, d, J=8.7 Hz), 8.60 (1H, s), 10.87 (1H, s). | 424 |
| | | | 426 |
| I-83 | 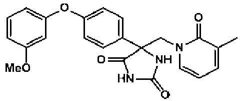 | (DMSO-d₆) δ: 1.99 (3H, s), 3.74 (3H, s), 4.46 (1H, d, J=13.7 Hz), 4.61 (1H, d, J=13.7 Hz), 6.12 (1H, t, J=6.9 Hz), 6.55 (1H, ddd, J=0.9, 2.3, 8.2 Hz), 6.61 (1H, t, J=2.3 Hz), 6.74 (1H, m), 7.07 (2H, td, J=2.5, 8.7 Hz), 7.23-7.32 (3H, m), 7.63 (2H, td, J=2.5, 9.2 Hz), 8.58 (1H, d, J=1.4 Hz), 10.86 (1H, s). | 420 |
| I-84 | 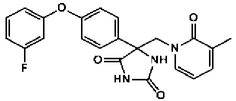 | (DMSO-d₆) δ: 1.99 (3H, s), 4.48 (1H, d, J=13.7 Hz), 4.62 (1H, d, J=13.7 Hz), 6.12 (1H, t, J=6.9 Hz), 6.83 (1H, dd, J=2.3, 8.7 Hz), 6.89 (1H, td, J=2.3, 10.5 Hz), 6.99 (1H, dt, J=1.8, 7.8 Hz), 7.13 (2H, td, J=2.5, 8.7 Hz), 7.24-7.31 (2H, m), 7.42 (1H, m), 7.66 (2H, td, J=2.5, 8.7 Hz), 8.61 (1H, s), 10.88 (1H, s). | 408 |
| I-85 | 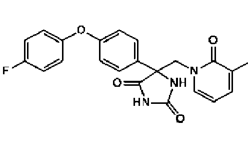 | (DMSO-d₆) δ: 1.99 (3H, s), 4.45 (1H, d, J=13.7 Hz), 4.60 (1H, d, J=13.7 Hz), 6.12 (1H, t, J=6.9 Hz), 7.04 (2H, td, J=2.5, 9.2 Hz), 7.06-7.11 (2H, m), 7.20-7.31 (4H, m), 7.62 (2H, td, J=2.5, 8.7 Hz), 8.58 (1H, s), 10.86 (1H, s). | 408 |
| I-86 | 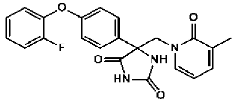 | (DMSO-d₆) δ: 1.99 (3H, s), 4.45 (1H, d, J=13.3 Hz), 4.60 (1H, d, J=13.3 Hz), 6.12 (1H, t, J=6.9 Hz), 7.02 (2H, d, J=9.2 Hz), 7.16-7.31 (5H, m), 7.41 (1H, m), 7.62 (2H, td, J=2.5, 8.7 Hz), 8.55 (1H, s), 10.86 (1H, s). | 408 |
| I-87 | 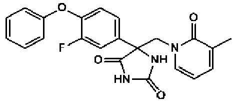 | (DMSO-d₆) δ: 1.99 (3H, s), 4.50 (1H, d, J=13.7 Hz), 4.61 (1H, d, J=13.7 Hz), 6.13 (1H, t, J=6.9 Hz), 6.95-7.10 (2H, m), 7.14 (1H, m), 7.22 (1H, t, J=8.7 Hz), 7.25-7.32 (2H, m), 7.35-7.43 (2H, m), 7.47 (1H, m), 7.64 (1H, dd, J=2.3, 12.3 Hz), 8.66 (1H, s), 10.96 (1H, s). | 408 |
| I-88 | 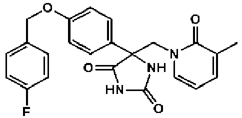 | (DMSO-d₆) δ: 1.99 (3H, s), 4.44 (1H, d, J=13.7 Hz), 4.57 (1H, d, J=13.7 Hz), 5.10 (2H, s), 6.11 (1H, t, J=6.6 Hz), 7.03-7.10 (2H, m), 7.13-7.36 (5H, m), 7.37-7.78 (3H, m), 8.52 (1H, s), 10.80 (1H, s). | 422 |

[Table 5]

**Table 5**

| Compound | Structure | ¹H-NMR | MS (M+H)⁺ |
|---|---|---|---|
| I-89 | 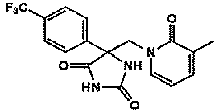 | (DMSO-d₆) δ: 1.97 (3H, s), 4.36 (1H, d, J=13.3 Hz), 4.53 (1H, d, J=13.3 Hz), 6.07 (1H, t, J=6.9 Hz), 7.20 (1H, br s), 7.27 (2H, dd, J=6.9, 11.4 Hz), 7.72 (2H, d, J=8.2 Hz), 7.85 (2H, d, J=8.2 Hz). | 366 |
| I-90 | 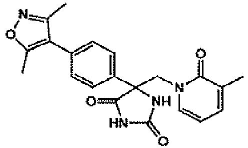 | (DMSO-d₆) δ: 2.00 (3H, s), 2.24 (3H, s), 2.41 (3H, s), 4.51 (1H, d, J=13.3 Hz), 4.66 (1H, d, J=13.7 Hz), 6.13 (1H, t, J=6.7 Hz), 7.29 (2H, t, J=6.4 Hz), 7.47 (2H, d, J=8.7 Hz), 7.73 (2H, d, J=8.7 Hz), 8.66 (1H, s), 10.89 (1H, s). | 393 |
| I-91 | 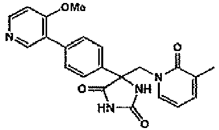 | (DMSO-d₆) δ: 2.01 (3H, s), 3.90 (3H, s), 4.51 (1H, d, J=13.3 Hz), 4.66 (1H, d, J=13.7 Hz), 6.13 (1H, t, J=6.9 Hz), 6.93 (1H, d, J=8.7 Hz), 7.29 (2H, t, J=5.5 Hz), 7.72 (2H, d, J=8.7 Hz), 7.75 (2H, d, J=8.7 Hz), 8.05 (1H, dd, J=2.5, 8.5 Hz), 8.52 (1H, d, J=2.3 Hz), 8.65 (1H, s), 10.88 (1H, s). | 405 |
| I-92 | 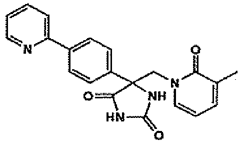 | (DMSO-d₆) δ: 2.00 (3H, s), 4.52 (1H, d, J=13.3 Hz), 4.69 (1H, d, J=13.7 Hz), 6.13 (1H, t, J=6.9 Hz), 7.29 (2H, d, J=6.9 Hz), 7.38 (1H, ddd, J=0.9, 4.6, 5.5 Hz), 7.75 (2H, d, J=8.2 Hz), 7.90 (1 H, dt, J=1.8, 7.3 Hz), 8.01 (1H, d, J=8.2 Hz), 8.15 (2H, d, J=8.2 Hz), 8.64 (1H, s), 8.67-8.70 (1H, m), 10.89 (1H, s). | 375 |
| I-93 | 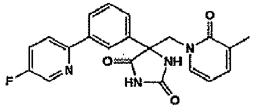 | (DMSO-d6) δ: 2.00 (3H, s), 4.53 (1H, d, J=13.3 Hz), 4.71 (1H, d, J=13.3 Hz), 6.13 (1H, t, J=6.9 Hz), 7.27-7.32 (2H, m), 7.57 (1H, t, J=7.8 Hz), 7.71 (1H, d, J=8.7 Hz), 7.90 (1H, dt, J=3.2, 8.7 Hz), 8.06 (1H, d, J=7.8 Hz), 8.11 (1H, dd, J=4.3, 9.2 Hz), 8.32 (1H, s), 8.69 (1H, s), 8.70 (1H, d, J=3.2 Hz), 10.91 (1H, s). | 393 |
| I-94 | 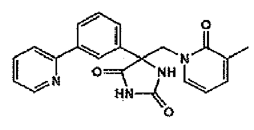 | (DMSO-d₆) δ: 2.00 (3H, s), 4.53 (1H, d, J=13.7 Hz), 4.72 (1H, d, J=13.3 Hz), 6.13 (1H, t, J=6.9 Hz), 7.27-7.34 (2H, m), 7.37-7.43 (1H, m), 7.57 (1H, t, J=7.8 Hz), 7.72 (1H, d, J=8.2 Hz), 7.94 (1H, t, J=7.8 Hz), 8.02 (1H, d, J=7.3 Hz), 8.09 (1H, d, J=7.8 Hz), 8.36 (1H, s), 8.67-8.73 (2H, m), 10.90 (1H, s). | 375 |
| I-95 | 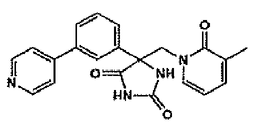 | (DMSO-d₆) δ: 2.00 (3H, s), 4.59 (1H, d, J=13.7 Hz), 4.68 (1H, d, J=13.7 Hz), 6.13 (1H, t, J=6.9 Hz), 7.30 (2H, d, J=6.9 Hz), 7.60 (1H, t, J=7.8 Hz), 7.73-7.79 (3H, m), 7.83 (1H, d, J=7-8 Hz), 8.05 (1H, t, J=1.8 Hz), 8.68 (2H, dd, J=1.4, 4.6 Hz), 8.71 (1H, s), 10.93 (1H, s). | 375 |
| I-96 | 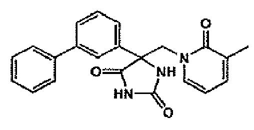 | (DMSO-d₆) δ: 2.00 (3H, s), 4.57 (1H, d, J=13.7 Hz), 4.67 (1H, d, J=13.7 Hz), 6.13 (1H, t, J=6.9 Hz), 7.30 (2H, d, J=6.9 Hz), 7.40 (1H, t, J=7.4 Hz), 7.46-7.76 (7H, m), 7.93 (1H, t, J=1.8 Hz), 8.68 (1H, s), 10.89 (1H, s). | 374 |

[Table 6]

**Table 6**

| Compound | Structure | ¹H-NMR | MS (M+H)⁺ |
|---|---|---|---|
| I-97 | 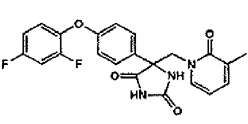 | (DMSO-d₆) δ: 1.99 (3H, s), 4.45 (1H, d, J=13.3 Hz), 4.60 (1H, d, J=13.3 Hz), 6.11 (1H, t, J=6.9 Hz), 7.01 (2H, td, J=2.5, 8.7 Hz), 7.15 (1H, m), 7.23-7.35 (3H, m), 7.50 (1H, m), 7.61 (2H, td, J=2.5, 9.2 Hz), 8.57 (1H, d, J=1.4 Hz), 10.85 (1H, s), | 426 |
| I-98 | 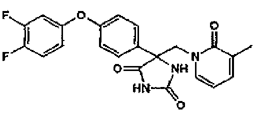 | (DM50-d₆) δ: 1.99 (3H, s), 4.47 (1H, d, J=13.7 Hz), 4.60 (1H, d, J=13.7 Hz), 6.12 (1H, t, J=6.9 Hz), 6.87 (1H, m), 7.10 (2H, td, J=2.5, 9.2 Hz), 7.20-7.32 (3H, m), 7.47 (1H, m), 7.65 (2H, td, J=2.5, 8.7 Hz), 8.60 (1H, s), 10.87 (1H, s). | 426 |
| I-89 | 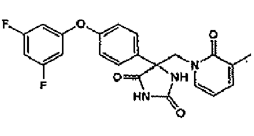 | (DMSO-d₈) δ: 1.99 (3H, s), 4.49 (1H, d, J=13.7 Hz), 4.61 (1H, d, J=13.7 Hz), 6.11 (1H, t, J=6.9 Hz), 6.75 (2H, dd, J=2.3, 8.7 Hz), 7.03 (1H, tt, J=2.3, 9.2 Hz), 7.19 (2H, td, J=2.5, 9.2 Hz), 7.23-7.31 (2H, m), 7.69 (2H, td, J=2.5, 9.2 Hz), 8.64 (1H, d, J=1.4 Hz), 10.90 (1H, s), | 426 |
| I-100 | 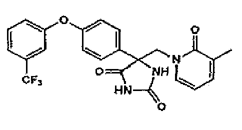 | (DMSO-d₆) δ: 1.99 (3H, s), 4.48 (1H, d, J=13.7 Hz), 4.63 (1H, d, J=13.7 Hz), 6.12 (1H, t, J=6.9 Hz), 7.16 (2H, td, J=2.5, 8.7 Hz), 7.24-7.32 (3H, m), 7.34 (1H, s), 7.52 (1H, d, J=7.8 Hz), 7.63 (1H, t, J=7.9 Hz), 7.68 (2H, td, J=2.5, 8.7 Hz), 8.62 (1H, d, J=0.9 Hz) 10.89 (1H, s). | 458 |
| I-101 | 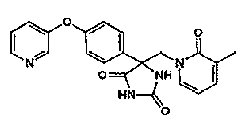 | (DMSO-d₆) δ: 1.99 (3H, s), 4.47 (1H, d, J=13.7 Hz), 4.62 (1H, d, J=13.7 Hz), 6.12 (1H, t, J=6.9 Hz), 7.13 (2H, td, J=2.5, 9.2 Hz), 7.24-7.32 (2H, m), 7.43-7.47 (2H, m), 7.67 (2H, td, J=2.5, 8.7 Hz), 8.38-8.42 (2H, m), 8.61 (1H, d, J=1.4 Hz), 10.88 (1H, s). | 391 |
| I-102 | 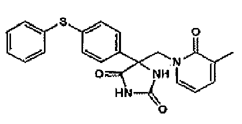 | (DMSO-d₆) δ: 1.99 (3H, s), 4.45 (1H, d, J=13.7 Hz), 4.61 (1H, d, J=13.7 Hz), 6.11 (1H, d, J=6.9 Hz), 7.23-7.31 (2H, m), 7.32-7.43 (7H, m), 7.61 (2H, d, J=8.7 Hz), 8.59 (1H, s), 10.89 (1H, s). | 406 |
| I-103 | 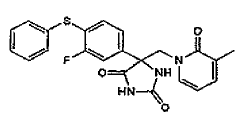 | (DMSO-d₆) δ: 1.98 (3H, s), 4.48 (1H, d, J=13.7 Hz), 4.61 (1H, d, J=13.7 Hz), 6.12 (1H, t, J=6.9 Hz), 7.24-7.43 (8H, m), 7.46 (1H, dd, J=1.8, 8.2 Hz), 7.56 (1H, dd, J=1.8, 11.0 Hz), 8.64 (1H, s), 10.98 (1H, s). | 424 |
| I-104 | 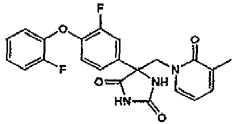 | (DMSO-d₆) δ: 1.99 (3H, s), 4.48 (1H, d, J=13.3 Hz), 4.60 (1H, d, J=13.3 Hz), 6.13 (1H, t, J=6.9 Hz), 7.08-7.16 (2H, m), 7.18-7.32 (4H, m), 7.37-7.47 (2H, m), 7.65 (1H, dd, J=2.3, 12.4 Hz), 8.64 (1H, s), 10.96 (1H, s). | 426 |

[Table 7]

**Table 7**

| Compound | Structure | ¹H-NMR | MS (M+H)⁺ |
|---|---|---|---|
| I-105 | 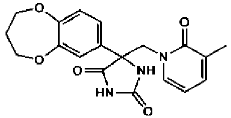 | (DMSO-d₆) δ: 1.99 (3H, s), 2.11 (2H, tt, J=5.5, 5.5 Hz), 4.07-4.19 (4H, m), 4.42 (1H, d, J=13.7 Hz), 4.54 (1H, d, J=13.7 Hz), 6.11 (1H, t, J=6.9 Hz), 7.01 (1H, t, J=8.2 Hz), 7.18 (1H, dd, J=2.3, 8.2 Hz), 7.21-7.25 (1H, m), 7.23 (1H, d, J=2.3 Hz), 7.28 (1H, m), 8.51 (1H, s), 10.76 (1H, m). | 370 |
| I-106 | 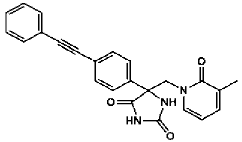 | (DMSO-d₆) δ: 2.00 (3H, s), 4.48 (1H, d, J=13.7 Hz), 4.66 (1H, d, J=13.7 Hz), 6.12 (1H, t, J=6.6 Hz), 7.24-7.31 (2H, m), 7.38-7.46 (3H, m), 7.53-7.60 (2H, m), 7.63 (2H, d, J=1.8 Hz), 7.68 (2H, d, J=1.8 Hz), 8.64 (1H, s), 10.71 (1H, s). | 398 |
| I-107 | 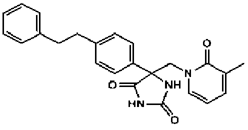 | (DMSO-d₆) δ: 2.00 (3H, s), 2.84-2.92 (4H, m), 4.39 (1H, d, J=13.7 Hz), 4.60 (1H, d, J=13.7 Hz), 6.10 (1H, t, J=6.9 Hz), 7.18 (1H, m), 7.21-7.32 (8H, m), 7.52 (2H, d, J=8.2 Hz), 8.52 (1H, s), 10.81 (1H, s). | 402 |
| I-108 | 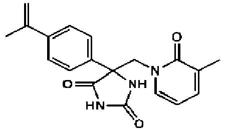 | (DMSO-d₆) δ: 2.00 (3H, s), 2.11 (3H, s), 4.47 (1H, d, J=13.7 Hz), 4.63 (1H, d, J=13.7 Hz), 5.14 (1H, m), 5.47 (1H, s), 6.12 (1H, t, J=6.9 Hz), 7.28 (2H, dd, J=7.1, 9.9 Hz), 7.52-7.67 (4H, m), 8.59 (1H, s), 10.84 (1H, s). | 338 |
| I-109 | 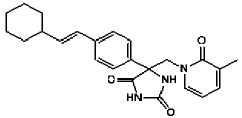 | (DMSO-d₆) δ: 1.12-1.36 (5H, m), 1.60-1.81 (5H, m), 1.99 (3H, s), 2.06-2.20 (1H, m), 4.45 (1H, d, J=13.7 Hz), 4.60 (1H, d, J=13.7 Hz), 6.11 (1H, t, J=6.9 Hz), 6.26-6.40 (2H, m), 7.25 (1H, d, J=7.3 Hz), 7.28 (1H, d, J=6.4 Hz), 7.43 (2H, d, J=8.7 Hz), 7.54 (2H, d, J=8.3 Hz), 8.55 (1H, s), 10.83 (1H, s). | 406 |
| I-110 | 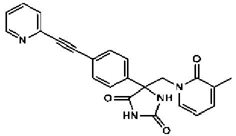 | (DMSO-d₆) δ: 2.00 (3H, s), 4.49 (1H, d, J=13.7 Hz), 4.64 (1H, d, J=13.7 Hz), 6.12 (1H, t, J=6.9 Hz), 7.23-7.32 (2H, m), 7.43 (1H, ddd, J=1.4, 5.0, 7.8 Hz), 7.65-7.74 (5H, m), 7.85 (1H, dt, J=1.8, 7.8 Hz), 8.54 (1H, s), 8.62 (1H, m), 10.94 (1H, s). | 399 |
| I-111 | 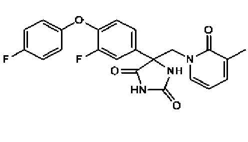 | (DMSO-d₆) δ: 1.99 (3H, s), 4.48 (1H, d, J=13.7 Hz), 4.60 (1H, d, J=13.7 Hz), 6.13 (1H, t, J=6.9 Hz), 7.02-7.09 (2H, m), 7.16-7.32 (5H, m), 7.46 (1H, m), 7.64 (1H, dd, J=2.3, 12.3 Hz), 8.65 (1H, s), 10.97 (1H, s). | 426 |
| I-112 | 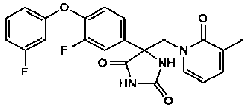 | (DMSO-d₆) δ: 1.98 (3H, s), 4.51 (1H, d, J=13.3 Hz), 4.61 (1H, d, J=13.3 Hz), 6.13 (1H, t, J=6.9 Hz), 6.80 (1H, dd, J=2.3, 8.7 Hz), 6.89 (1H, td, J=2.3, 10.5 Hz), 7.00 (1H, dt, J=2.3, 8.3 Hz), 7.25-7.35 (3H, m), 7.41 (1H, m), 7.49 (1H, dd, J=1.8, 8.2 Hz), 7.66 (1H, dd, J=2.3, 12.4 Hz), 8.68 (1H, s), 10.99 (1H, s). | 426 |

[Table 8]

**Table 8**

| Compound | Structure | ¹H-NMR | MS (M+H)⁺ |
|---|---|---|---|
| I-113 | 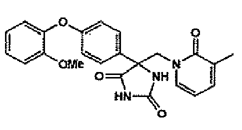 | (DMSO-d₆) δ: 1.99 (3H, s), 3.74 (3H, s), 4.44 (1H, d, J=13.7 Hz), 4.57 (1H, d, J=13.7 Hz), 6.11 (1H, t, J=6.9 Hz), 6.86 (2H, td, J=2.5, 8.7 Hz), 6.99 (1H, dt, J=1.8, 7.8 Hz), 7.06 (1H, dd, J=1.8, 7.8 Hz), 7.16-7.31 (4H, m), 7.54 (2H, td, J=2.5, 9.2 Hz), 8.52 (1H, d, J=1.4 Hz), 10.82 (1H, s). | 420 |
| I-114 | 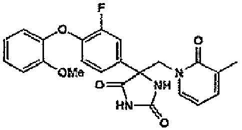 | (DMSO-d₆) δ: 1.99 (3H, s), 3.76 (3H, s), 4.46 (1H, d, J=13.7 Hz), 4.59 (1H, d, J=13.7 Hz), 6.12 (1H, t, J=6.9 Hz), 6.80 (1H, t, J=8.7 Hz), 6.93-7.04 (2H, m), 7.16-7.36 (5H, m), 7.59 (1H, dd, J=2.3, 17.8 Hz), 8.59 (1H, d, J=1.4 Hz), 10.93 (1H, s). | 438 |
| I-115 | 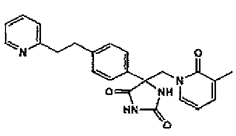 | (DMSO-d₆) δ: 2.00 (3H, s), 2.97-3.07 (4H, m), 4.44 (1H, d, J=13.7 Hz), 4.60 (1H, d, J=13.7 Hz), 6.11 (1H, t, J=6.9 Hz), 7.20-7.32 (6H, m), 7.52 (2H, d, J=8.2 Hz), 7.70 (1H, dt, J=1.8, 7.6 Hz), 8.49-8.54 (2H, m), 10.81 (1H, s). | 403 |
| I-116 | 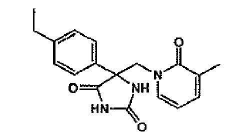 | (DMSO-d₆) δ: 1.17 (3H, t, J=7.8 Hz), 2.00 (3H, s), 2.61 (2H, q, J=7.5 Hz), 4.45 (1H, d, J=13.7 Hz), 4.60 (1H, d, J=13.7 Hz), 6.11 (1H, t, J=6.9 Hz), 7.23-7.31 (4H, m), 7.53 (2H, d, J=8.2 Hz), 8.52 (1H, s), 10.80 (1H, s). | 326 |
| I-117 | 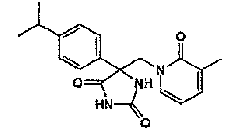 | (DMSO-d₆) δ: 1.20 (6H, d, J=6.9 Hz), 2.00 (3H, s), 2.90 (1H, sept, J=6.9 Hz), 4.45 (1H, d, J=13.7 Hz), 4.61 (1H, d, J=13.7 Hz), 6.12 (1H, t, J=6.9 Hz), 7.23-7.28 (2H, m), 7.31 (2H, d, J=8.3 Hz), 7.54 (2H, d, J=8.7 Hz), 8.52 (1H, d, J=1.4 Hz), 10.79 (1H, s). | 340 |
| I-118 | 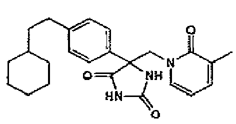 | (DMSO-d₆) δ: 0.84-0.96 (2H, m), 1.09-1.28 (4H, m), 1.40-1.49 (2H, m), 1.57-1.80 (5H, m), 2.00 (3H, s), 2.56-2.61 (2H, m), 4.45 (1H, d, J=13.7 Hz), 4.60 (1H, d, J=13.7 Hz), 6.10 (1H, t, J=6.6 Hz), 7.22-7.30 (4H, m), 7.51 (2H, d, J=8.2 Hz), 8.51 (1H, d, J=1.4 Hz), 10.80 (1H, s). | 408 |
| I-119 | 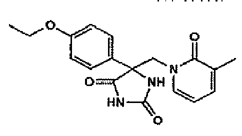 | (DMSO-d₆) δ: 1.32 (3H, t, J=7.1 Hz), 2.00 (3H, s), 4.03 (2H, q, J=6.9 Hz), 4.44 (1H, d, J=13.7 Hz), 4.57 (1H, d, J=13.7 Hz), 6.11 (1H, t, J=6.6 Hz), 6.92-7.02 (2H, m), 7.19-7.32 (2H, m), 7.48-7.56 (2H, m), 8.51 (1H, s), 10.79 (1H, s). | 342 |
| I-120 | 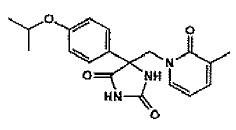 | (DMSO-d₆) δ: 1.26 (6H, d, J=6.0 Hz), 2.00 (3H, s), 4.44 (1H, d, J=13.7 Hz), 4.56 (1H, d, J=13.7 Hz), 4.63 (1H, sept, J=6.0 Hz), 6.11 (1H, t, J=6.6 Hz), 6.92-6.99 (2H, m), 7.21-7.31 (2H, m), 7.51 (2H, td, J=2.5, 8.7 Hz), 8.51 (1H, d, J=1.4 Hz), 10.79 (1H, s). | 356 |

[Table 9]

**Table 9**

| Compound | Structure | ¹H-NMR | MS (M+H)⁺ |
|---|---|---|---|
| I-121 | 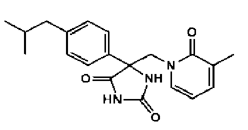 | (DMSO-d₆) δ: 0.85 (6H, d, J=6.9 Hz), 1.83 (1H, m), 1.99 (3H, s), 2.45 (2H, d, J=7.3 Hz), 4.45 (1H, d, J=13.7 Hz), 4.61 (1H, d, J=13.7 Hz), 6.10 (1H, t, J=6.9 Hz), 7.19-7.30 (4H, m), 7.52 (2H, d, J=8.4 Hz), 8.52 (1H, d, J=1.4 Hz), 10.81 (1H, s). | 354 |
| I-122 | 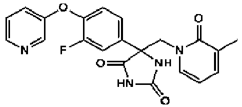 | (CDCl₃) δ: 2.13 (3H, s), 4.30 (1H, d, J=13.7 Hz), 4.80 (1H, d, J=13.7 Hz), 6.13 (1H, t, J=6.9 Hz), 7.11 (1H, t, J=8.2 Hz), 7.21-7.32 (3H, m), 7.39 (1H, s), 7.45 (1H, m), 7.58 (1H, dd, J=2.3, 11.4 Hz), 8.34-8.42 (2H, m), 8.67 (1H, s). | 409 |
| I-123 | 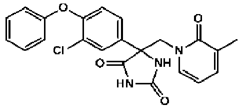 | (DMSO-d₆) δ: 1.99 (3H, s), 4.49 (1H, d, J=13.7 Hz), 4.61 (1H, d, J=13.7 Hz), 6.13 (1H, t, J=6.9 Hz), 6.97 (1H, dd, J=0.9, 8.7 Hz), 7.11-7.19 (2H, m), 7.22-7.32 (3H, m), 7.35-7.43 (2H, m), 7.60 (1H, dd, J=2.3, 8.7 Hz), 7.83 (1H, d, J=2.3 Hz), 8.66 (1H, s), 10.97 (1H, s). | 424 |
| I-124 | 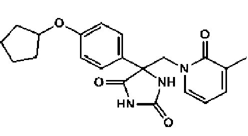 | (CDCl₃) δ: 1.58-1.68 (2H, m), 1.72-1.98 (6H, m), 2.12 (3H, s), 4.22 (1H, d, J=13.7 Hz), 4.75 (1H, m), 4.83 (1H, d, J=13.7 Hz), 6.07 (1H, t, J=13.7 Hz), 6.88 (2H, td, J=2.5, 6.9 Hz), 7.02 (1H, s), 7.13 (1 H, dd, J=1.4, 6.9 Hz), 7.17-7.22 (1H, m), 7.51 (2H, td, J=2.5, 9.2 Hz), 8.10 (1H, s). | 382 |
| I-125 | 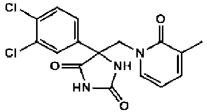 | (DMSO-d₆) δ: 1.98 (3H, s), 4.47 (1H, d, J=13.7 Hz), 4.61 (1H, d, J=13.7 Hz), 6.13 (1H, t, J=6.9 Hz), 7.24-7.31 (2H, m), 7.62 (1H, dd, J=2.3, 8.7 Hz), 7.73 (1H, d, J=8.7 Hz), 7.87 (1H, d, J=2.3 Hz), 8.65 (1H, brs), 10.93 (1H, m). | 366 368 |
| I-126 | 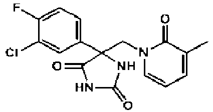 | (DMSO-d6) δ: 1.98 (3H, s), 4.47 (1H, d, J=13.7 Hz), 4.60 (1H, d, J=13.7 Hz), 6.12 (1H, t, J=6.4 Hz), 7.24-7.31 (2H, m), 7.50 (1H, t, J=8.7 Hz), 7.64 (1H, ddd, J=2.7, 4.6, 8.7 Hz), 7.83 (1H, dd, J=2.7, 7.3 Hz), 8.63 (1H, br s), 10.96 (1 H, m). | 350 352 |
| I-127 | 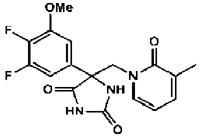 | (DMSO-d₆) δ: 1.99 (3H, s), 3.90 (3H, s), 4.48 (1 H, d, J=13.7 Hz), 4.57 (1H, d, J=13.7 Hz), 6.13 (1H, t, J=6.9 Hz), 7.22-7.32 (4H, m), 8.64 (1H, br s), 11.00 (1H, br s). | 364 |
| I-128 | 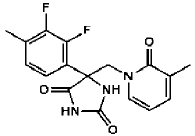 | (DMSO-d₆) δ: 1.99 (3H, s), 2.30 (3H, s), 4.70 (1H, d, J=13.7 Hz), 4.76 (1H, d, J=13.7 Hz), 6.14 (1H, t, J=6.9 Hz), 7.18 (1H, br t, J=7.3 Hz), 7.24-7.34 (3H, m), 8.31 (1H, br s), 10.94 (1H, br s). | 348 |

[Table 10]

**Table 10**

| Compound. | Structure | ¹H-NMR | MS (M+H)⁺ |
|---|---|---|---|
| I-129 | 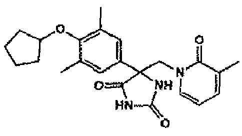 | (CDCl₃) δ: 1.53-1.92 (8H, m), 2.13 (3H, s), 2.28 (6H, s), 4.21 (1H, d, J=13.7Hz), 4.46 (1H, m), 4.84 (1H, d, J=13.7Hz), 6.08 (1H, t, J=6.9Hz), 6.93 (2H, 7.13 (1H, d, J=6.4Hz), 7.20 (1H, d, J=6.4Hz), 7.24 (1H, s). | 410 |
| I-130 | 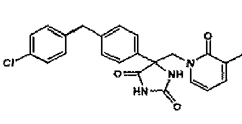 | (DMSO-d₆) δ: 1.99 (3H, s), 3.94 (2H, s), 4.44 (1H, d, J=13.7 Hz), 4.58 (1H, d, J=13.3 Hz), 6.10 (1H, t, J=6.7 Hz), 7.22-7.38 (8H, m), 7.54 (2H, d, J=8.2 Hz), 8.44 (1H, s), 10.76 (1H, s). | 422 424 |
| I-131 | 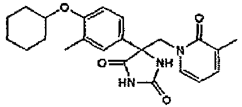 | (CDCl₃) δ: 1.30-1.98 (10H, m), 2.11 (3H, s), 2.22 (3H, s), 4.19 (1H, d, J=13.7 Hz), 4.28 (1H, m), 4.87 (1H, d, J=13.7 Hz), 6.07 (1H, t, J=6.9 Hz), 6,83 (1H, d, J=8.7 Hz), 7.10-7.22 (3H, m), 7.34-7.42 (2H, m), 8.96 (1H, br s). | 410 |

[Table 11]

**Table 11**

| Compound | Structure | ¹H-NMR | MS (M+H)⁺ |
|---|---|---|---|
| I-132 | 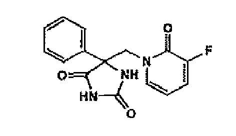 | (DMSO-d₆) δ: 4.49 (1H, d, J=13.7 Hz), 4.75 (1H, d, J=13.7 Hz), 6.18 (1 H, dt, J=4.6, 7.3 Hz), 7.22 (1 H, dt, J=1.4, 6.9 Hz), 7.36-7.48 (4H, m), 7.60-7.65 (2H, m), 8.72 (1H, s), 10.83 (1H, s). | 302 |
| I-133 | 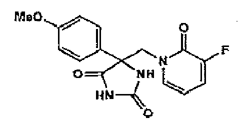 | (DMSO-d₆) δ: 3.76 (3H, s), 4.46 (1H, d, J=13.7 Hz), 4.69 (1H, d, J=13.7 Hz), 6.18 (1H, dt, J=4.6, 7.3 Hz), 7.00 (2H, d, J=8.7 Hz), 7.20 (1H, d, J=6.9 Hz), 7.36-7.43 (1H, m), 7.53 (2H, d, J=8.7 Hz), 8.69 (1H, s), 10.87 (1H, s). | 332 |
| I-134 | 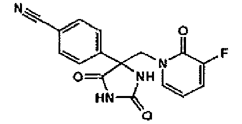 | (DMSO-d₆) δ: 4.51 (1H, d, J=13.7 Hz), 4.78 (1 H, d, J=13.7 Hz), 6.20 (1H, dt, J=4.6, 7.3 Hz), 7.21-7.26 (1H, m), 7.40 (1H, ddd, J=1.8, 7.8, 9.6 Hz), 7.82 (2H, d, J=8.7 Hz), 7.95 (2H, d, J=8.7 Hz), 8.88 (1H, s), 11.08 (1H, s). | 327 |
| I-135 | 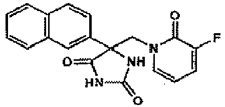 | (DMSO-d₆) δ: 4.60 (1H, d, J=13.7 Hz), 4.89 (1H, d, J=13.7 Hz), 6.20 (1H, dt, J=4.6, 6.9 Hz), 7.28 (1H, d, J=6.9 Hz), 7.41 (1H, ddd, J=1.8, 7.3, 9,2 Hz), 7.54-7.61 (2H, m), 7.78 (1H, dd, J=1.8, 8.7 Hz), 7.92-7.98 (2H, m), 8.01 (1H, d, J=8.7 Hz), 8.15 (1H, d, J=1.8 Hz), 8.84 (1H, s), 10.98 (1H, s). | 352 |
| I-136 | 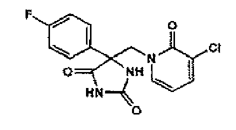 | (DMSO-d₆) δ: 4.48 (1H, d, J=13.3 Hz), 4.73 (1H, d, J=13.7 Hz), 6.24 (1H, t, J=7.1 Hz), 7.26-7.33 (2H, m), 7.39 (1H, dd, J=1.8, 6.9 Hz), 7.63-7.70 (2H, m), 7.75 (1H, dd, J=1.8, 7.3 Hz), 8.78 (1H, s), 10.96 (1H, s). | 336 338 |
| I-137 | 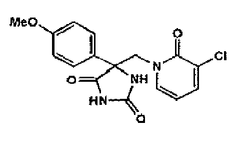 | (DMSO-d₆) δ: 3.77 (3H, s), 4.46 (1H, d, J=13.7 Hz), 4.70 (1H, d, J=13.7 Hz), 6.23 (1H, t, J=7.1 Hz), 7.00 (2H, d, J=9.2 Hz), 7.37 (1H, dd, J=1.8, 6.9 Hz), 7.53 (2H, d, J=9.2 Hz), 7.74 (1H, dd, J=1.8, 7.3 Hz), 8.69 (1H, s), 10.87 (1H, s). | 348 |
| I-138 | 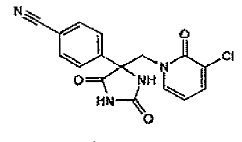 | (DMSO-d₆) δ: 4.51 (1H, d, J=13.3 Hz), 4.75 (1H, d, J=13.7 Hz), 6.26 (1H, t, J=7.1 Hz), 7.41 (1H, dd, J=1.8, 6.9 Hz), 7.75 (1H, dd, J=1.8, 7.3 Hz), 7.82 (2H, d, J=8.7 Hz), 7.95 (2H, d, J=8.7 Hz), 8.86 (1H, s), 10.99 (1H, s). | 343 345 |
| I-139 | 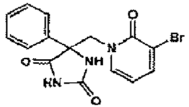 | (DMSO-d₆) δ: 4.49 (1H, d, J=13.3 Hz), 4.75 (1H, d, J=13.7 Hz), 6.18 (1H, t, J=7.1 Hz), 7.36-7.48 (4H, m), 7.60-7.65 (2H, m), 7.92 (1H, dd, J=1.8, 7.3 Hz), 8.74 (1H, s), 10.91 (1H, s). | 362 364 |

[Table 12]

**Table 12**

| | Structure | ¹H-NMR | MS (M+H)+ |
|---|---|---|---|
| I-140 | 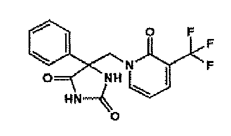 | (DMSO-d₆) 6;4.49 (1H, d, J=13.7 Hz), 4.76 (1H, d, J=13.7 Hz), 6.37 (1H, t, J=6.9 Hz), 7.36-7.48 (3H, m), 7.59-7.66 (2H, m), 7.71 (1H, dd, J=1.8, 6.9 Hz), 7.96 (1H, dd, J=1.2, 7.1 Hz), 8.77 (1H, s), 10.92 (1H, s). | 350 (M-H)⁻ |
| I-141 | 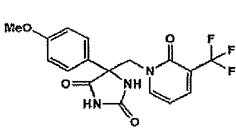 | (DMSO-d₆) δ : 3.76 (3H, s), 4.46 (1H, d, J=13.3 Hz), 4.70 (1H, d, J=13.3 Hz), 6.36 (1H, t, J=6.9 Hz), 6.99 (2H, d, J=9.2 Hz), 7.53 (2H, d, J=8.7 Hz), 7.69 (1H, m), 7.94 (1 H, m), 8.72 (1H, s), 10.87 (1H, s), | 382 |
| I-142 | 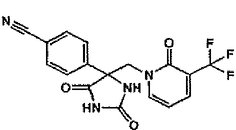 | (DMSO-d₆) δ :4.52 (1H, d, J=13.7 Hz), 4.78 (1H, d, J=13.7 Hz), 6.39 (1H, t, J=7.1 Hz), 7.72-7.77 (1H, m), 7.81 (2H, d, J=8.7 Hz), 7.91-7.98 (3H, m), 8.92 (1H, s), 11.08 (1H, s). | 377 |
| I-143 | 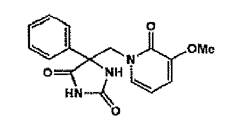 | (DMSO-d₆) δ: 3.69 (3H, s), 4.47 (1H, d, J=13.3 Hz), 4.64 (1H, d, J=13.3 Hz), 6.12 (1H, t, J=7.1 Hz), 6.78 (1H, dd, J=1.6, 7.6 Hz), 6.96 (1H, dd, J=1.8, 6,9 Hz), 7.35-7.48 (3H, m), 7.60-7.66 (2H, m), 8.59 (1H, s), 10.84 (1H, s). | 314 |
| I-144 | 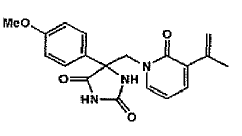 | (DMSO-d₆) δ: 2.01 (3H, s), 3.76 (3H, s), 4.47 (1H, d, J=13.7 Hz), 4.62 (1H, d, J=13.7 Hz), 5.15 (1H,s), 5.83 (1H, d, J=2.3 Hz), 6.22 (1H, t, J=6.9 Hz), 6.99 (2H, d, J=8.7 Hz), 7.38 (2H, ddd, J=1.8, 6.9, 8.7 Hz), 7.55 (2H, d, J=8.7 Hz), 8.53 (1H, s), 10.77 (1H, s). | 354 |
| I-145 | 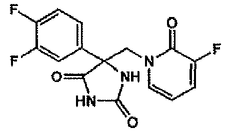 | (DMSO-d₆) δ: 4.49 (1H, d, J=13.7 Hz), 4.71 (1H, d, J=13.7 Hz), 6.20 (1H, dt, J=4.6, 7.3 Hz), 7.22 (1H, d, J=6.9 Hz), 7.36-7.60 (3H, m), 7.69 (1H, ddd, J=2.3, 7.8, 12.4 Hz), 8.78 (1H, s), 11.05 (1H, s). | 338 |
| I-146 | 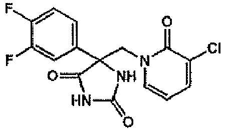 | (DMSO-d₆) δ: 4.44 (1H, d, J=13.7 Hz), 4.55 (1H, d, J=13.7 Hz), 6.25 (1H, t, J=6.9 Hz), 7.39 (1H, dd, J=1.8, 6.9 Hz), 7.43-7.58 (2H, m), 7.69 (1H, ddd, J=2.3, 7.3, 11.9 Hz), 7.75 (1H, dd, J=1.8, 7.3 Hz), 8.71 (1H, s),11.04 (1H, s). | 354 |
| I-147 | 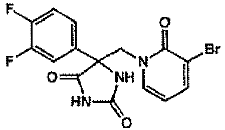 | (DMSO-d₆) δ: 4.49 (1H, d, J=13.7 Hz), 4.70 (1H, d, J=13.7 Hz), 6.19 (1H, t, J=6.9 Hz), 7.39-7.59 (3H, m), 7.68 (1H, ddd, J=2.3, 7.3, 11.9 Hz), 7.92 (1H, dd, J=1.8, 7.3 Hz), 8.74 (1H, s), 11.04 (1H, s). | 398 400 |
| I-148 | 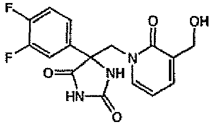 | (DMSO-d₆) δ: 4.29 (2H, d, J=5.0 Hz), 4.47 (1H, d, J=13.7 Hz), 4.61 (1H, d, J=13.7 Hz), 5.11 (1H, t, J=5.0 Hz), 6.25 (1H, t, J=6.9 Hz), 7.30 (1H, dd, J=1.8, 6.9 Hz), 7.40 (1H, dd, J=1.8, 6.9 Hz), 7.45-7.56 (2H, m), 7.66-7.71 (1H, m), 8.64 (1H, s), 10.96 (1 H, s). | 350 |

[Table 13]

**Table 13**

| Compound | Structure | ¹H-NMR | MS (M+H)⁺ |
|---|---|---|---|
| I-149 | 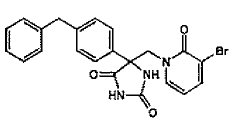 | (DMSO-d₆) δ: 3.95 (2H, s), 4.45 (1H, d, J=13.7 Hz), 4.72 (1H, d, J=13.7 Hz), 6.17 (1H, t, J=7.1 Wiz), 7.18-7.31 (7H, m), 7.42 (1H, dd, J=1.8, 6.9 Hz), 7.53 (2H, d, J=8.2 Hz), 7.91 (1H, dd, J=1.8, 7.3 Hz), 8.69 (1H, s), 10.88 (1 H, s). | 452 |
| | | | 454 |
| I-150 | 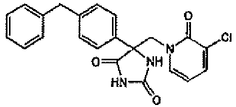 | (DMSO-d₆) δ: 3.95 (2H, s), 4.46 (1H, d, J=13.7 Hz), 4.73 (1H, d, J=13.7 Hz), 6.23 (1H, t, J=7.1 Hz), 7.18-7.31 (7H, m), 7.38 (1N, dd, J=1.8, 6.9 Hz), 7.53 (2H, d, J=8.2 Hz), 7.74 (1H, dd, J=1.8, 7.3 Hz), 8.68 (1H, s), 10.87 (1 H, s). | 408 |
| I-151 | 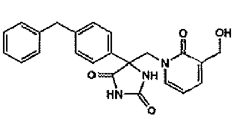 | (DMSO-d₆) δ: 4.30 (2H, d, J=5.5 Hz), 4.44 (1H, d, J=13.7 Hz), 4.61 (1H, d, J=13.7 Hz), 5.12 (1 H, t, J=5.5 Hz), 6.23 (1H, t, J=6.9 Hz), 7.18-7.30 (10H, m), 7.40 (1H, dd, J=1.8, 6.4 Hz), 7.53 (2H, d, J=8.2 Hz), 8.54 (1H, s), 10.82 (1H, s). | 404 |
| I-152 | 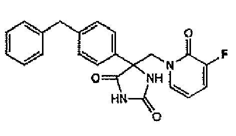 | (DMSO-d₆) δ: 3.95 (2H, s), 4.46 (1H, d, J=13.7 Hz), 4.71 (1H, d, J=13.7 Hz), 6.17 (1H, dt, J=4.6, 7.3 Hz), 7.17-7.23 (4H, m), 7.27-7.31 (4H, m), 7.38 (1H, ddd, J=1.4, 7.3, 10.1 Hz), 7.52 (2H, d, J=8.2 Hz), 8.67 (1H, s), 10.85 (1H, s). | 392 |
| I-153 | 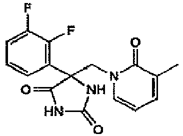 | CDMSO-d₆) δ: 1.99 (3H, s), 4.75 (2H, s), 6.14 (1 H, t, J=6.6 Hz), 7.28-7.33 (3H, m), 7.42-7.45 (1H, m), 7.51-7.57 (1H, m), 8.37 (1H, s), 10.99 (1H, s). | 334 |
| I-154 | 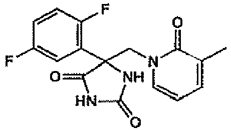 | (DMSO-d₆) δ: 1.99 (3H, s), 4.69 (1H, d, J=13.7 Hz), 4.77 (1H, d, J=13.7 Hz), 6.14 (1H, t, J=6.6 Hz),7.26-7.40 (4H, m), 7.48-7.52 (1H, m), 8.27 (1H, s), 10.96 (1H, s). | 334 |
| I-155 | 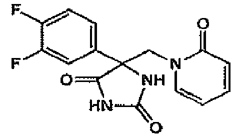 | (DMSO-d₆) δ: 4.45 (1H, d, J=13.7 Hz), 4.61 (1H, d, J=13.7 Hz), 6.21 (1H, dt, J=1.4, 6.4 Hz), 6.40 (1H, m), 7.38-7.43 (2H, m), 7.45-7.58 (2H, m), 7.69 (1H, ddd, J=2.3, 7.8, 12.4 Hz), 8.64 (1H, s), 10.98 (1H, brs). | 320 |
| I -156 | 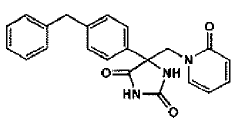 | (DMSO-d₆) δ: 3.94 (2H, s), 4.40 (1H, d, J=13.3 Hz), 4.61 (1H, d, J=13.3 Hz), 6.18 (1H, t, J=6.4 Hz), 6.39 (1H, d, J=9.2 Hz), 7.15-7.32 (7H, m), 7.53 (2H, d, J=8.2 Hz), 8.51 (1H, s), 10.81 (1H, br s). | 374 |
| I-157 | 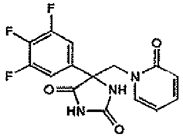 | (DMSO-d₆) δ: 4.45 (1H, d, J=13.3 Hz), 4.60 (1H, d, J=13.7 Hz), 6.21 (1H, m), 6.39 (1H, m), 7.37-7.44 (2H, m), 7.53-7.62 (2H, m), 8.63 (1H, s), 11.08 (1H, br s). | 338 |

### Working Example 55

### Production of (+)-5-(3,4-difluorophenyl)-5-[(3-methyl-2-oxopyridin-1(2H)-yl)methyl]imidazolidine-2,4-dione and (-)-5-(3,4-difluorophenyl)-5-[(3-methyl-2-oxopyridin-1(2H)-yl)methyl] imidazolidine-2,4-dione

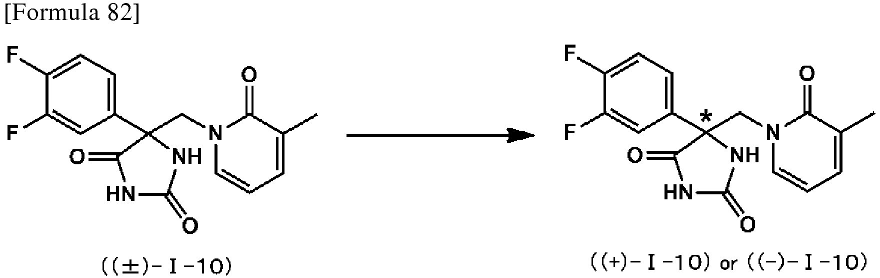

(wherein an asterisk (*) indicates an optically pure form)

A separating column (CHIRALPAK AD) was attached to a high-performance liquid chromatograph (Lachrom Elite, manufactured by Hitachi High-Technologies Corporation). Normal hexane-ethanol (30:70) was passed through the column for 1 hour at a temperature of 40°C and a flow rate of 8 mL/min to equilibrate the column. A solution of the compound produced in Working Example 10 (I-10; racemic form, also referred to as (±)-I-10) (14 mg) in normal hexane-ethanol (30:70) was injected, and then under observation with a UV detector, the first peak (from about after 12.5 minutes to about after 15.5 minutes) and the second peak (from about after 18 minutes to about after 23 minutes) were each collected. After repeating the same operation, the solvent from each collected sample was distilled off under reduced pressure. As the compound exhibiting the first peak, (+)-5-(3,4-difluorophenyl)-5-[(3-methyl-2-oxopyridin-1(2H)-yl)methyl]imidazolidine-2,4-dione ((+)-I-10) (yield 66 mg, optical purity >99.9% ee) was obtained, and as the compound exhibiting the second peak, (-)-5-(3,4-difluorophenyl)-5-[(3-methyl-2-oxopyridin-1(2H)-yl)methyl]imidazolidine-2,4-dione (yield 84 mg, optical purity 99.8% ee) was obtained.

The physical properties of each compound are shown below.

### (+)-5-(3,4-difluorophenyl)-5-[(3-methyl-2-oxopyridin-1(2H)-yl)methyl]imidazolidine-2,4-dione ((+)-I-10)

HPLC storage time 14.4 minutes
(Analysis conditions, column: CHIRALPAK AD-H 4.6 ϕ × 250 mm, mobile phase: *n-*hexane:ethanol = 40:60, flow rate: 0.5 mL/min, column temperature 30°C, detection wavelength: 254 nm)
[α]_{D}^{26.7} = +229.9° (*c* 1.0, CHCl₃)
1H-NMR (400 MHz, DMSO-d₆) δ: 1.98 (3H, s), 4.46 (1H, d, J=13.3 Hz), 4.60 (1H, d, J= 13.3 Hz), 6.12 (1H, t, J=6.9 Hz), 7.25 (1H, d, J= 6.9 Hz), 7.29 (1H, d, J= 6.0 Hz), 7.45-7.58 (2H, m), 7.65-7.73 (1H, m), 8.63 (1H, s), 10.98 (1H, s).
MS (ESI-FTMS) m/z 334 [M+H]⁺.

### (-)-5-(3,4-difluorophenyl)-5-[(3-methyl-2-oxopyridin-1(2H)-yl)methyl]imidazolidine-2,4-dione ((-)-I-10)

HPLC storage time 38.8 minutes
(Analysis conditions, column: CHIRALPAK AD-H 4.6 ϕ × 250 mm, mobile phase: *n-*hexane:ethanol = 40:60, flow rate: 0.5 mL/min, column temperature 30°C, detection wavelength: 254 nm)
[α]_{D}^{27.3} = -196.7° (*c* 1.0, CHCl₃)
1H-NMR (400 MHz, DMSO-d₆) δ: 1.98 (3H, s), 4.46 (1H, d, J=13.7 Hz), 4.60 (1H, d, J=13.7 Hz),
6.12 (1H, t, J=6.7 Hz), 7.25 (1H, d, J= 6.9 Hz), 7.29 (1H, d, J= 6.4 Hz), 7.45-7.58 (2H, m), 7.69 (1H, ddd, J=2.3, 7.8, 12.4 Hz), 8.62 (1H, s), 10.98 (1H, s).

### (Test Example 1) TACE (ADAM17) Inhibition Test

The pyridone derivatives obtained in the above-described working examples were examined to determine whether they had a TACE inhibitory action or not.

The nucleotide sequence of TACE has been reported by Moss et al. (Moss, M. L. et al., Nature 1997, vol. 385, pp. 733-736). Based on this information, the cDNA of TACE was obtained according to the prescribed method from THP-1 cells or the like, and then incorporated the cDNA into an expression vector. Next, this vector was transformed into mammalian cells or insect cells, and TACE protein was expressed. The TACE protein was isolated, and used as an enzyme. Further, a fluorescent synthetic substrate Nma(N-methylanthranilic acid)-Leu-Ala-Gln-Ala-Val-Arg-Ser-Ser-Lys(Dnp(dinitrophenyl))-D-Arg-NH₂ including the TACE-cleaved sequence of a membrane-bound TNF was used as a substrate. The enzyme reaction was carried out by adding 1.8 µL of a solution of the test substance to 90 µL of a fluorescent synthetic substrate prepared with an assay buffer B (50 mM tris-hydrochloric acid buffer (pH 7.5) including 200 mM sodium chloride, 5 mM calcium chloride, 10 µM zinc sulfate, and 0.05% PLURONIC F-68), then mixing 90 µL of a TACE enzyme solution prepared with an assay buffer A (50 mM tris-hydrochloric acid buffer (pH 7.5) including 200 mM sodium chloride, 5 mM calcium chloride, 10 µM zinc sulfate, and 2 mg/mL bovine serum albumin) and reacting at 37°C for 1.5 hours. Enzyme activity was then determined by measuring with a fluorescence intensity meter (Labsystems, Fluoroskan Ascent) at an excitation wavelength of 355 nm and a measurement wavelength of 460 nm. The 50% inhibitory concentration (IC₅₀) of the test substances was calculated by comparing enzyme activity in the presence and the absence of the test substance to determine the inhibition rate. Further, test substances having an IC₅₀ strength of less than 100 nM were evaluated as "A", and test substances having an IC₅₀ strength of 100 nM or more were evaluated as "B".

Tables 14(1) and 14(2), and Tables 15(1) and 15(2), show the results for the compounds I-1 to I-157, which are racemic forms.

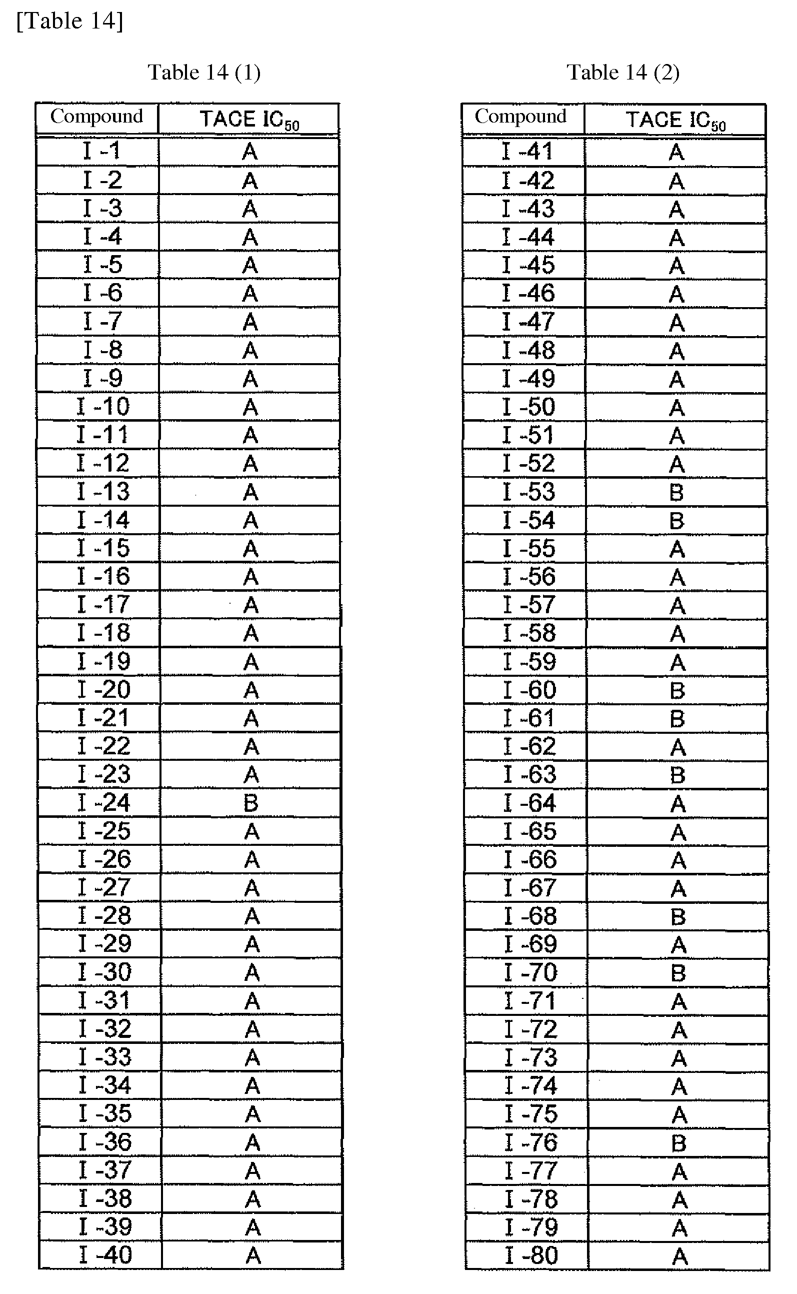

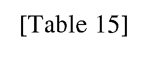

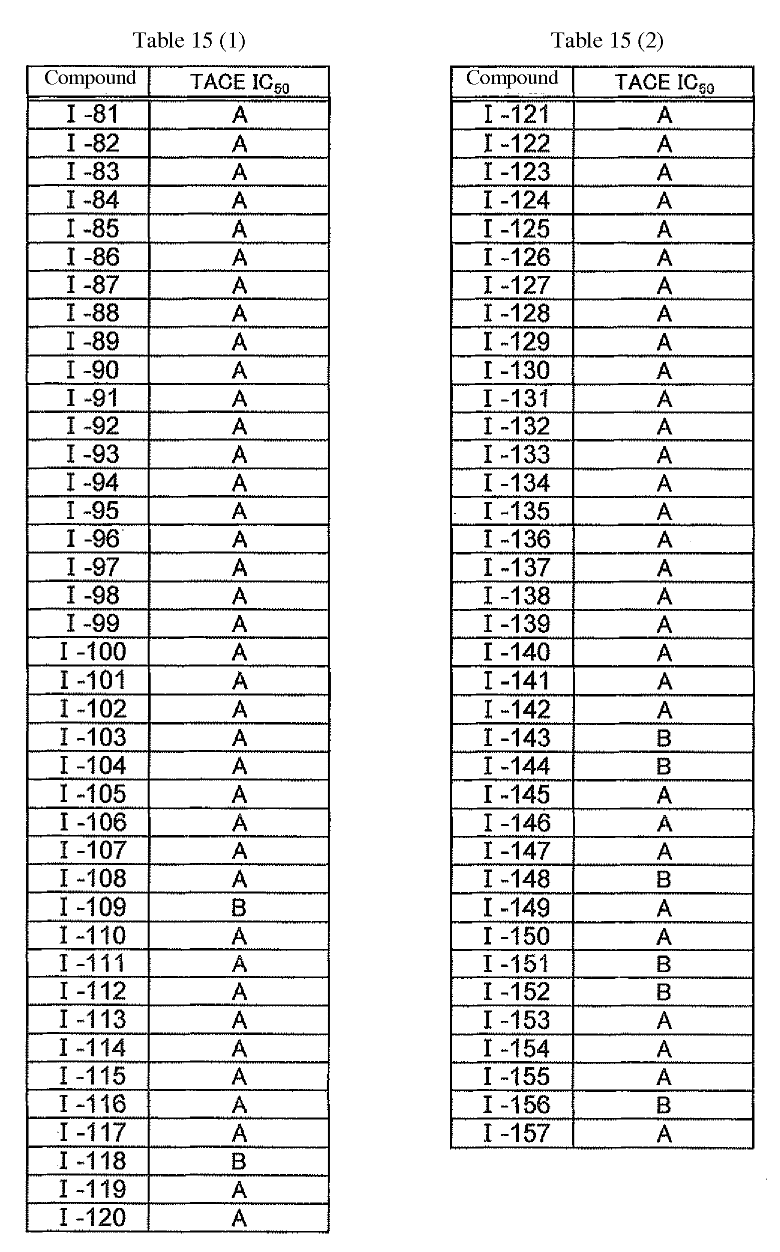

Further, the compound (+)-I-10 (optically-active substance) obtained based on the method described in Working Example 55 exhibited a TACE inhibitory action, having an IC₅₀ value of less than 100 nM. The compound (+)-I-10 had an "A" evaluation.

As can be clearly seen from the above results, it is confirmed that all the compounds (pyridone derivatives) according to the present invention inhibits the enzyme activity of TACE (ADAM17) at a low concentration.

### (Test Example 2) Verification of Function-Maintaining Effect on iPS Cell-Derived Platelets

### <Preparation of Human iPS Cells and Feeder Cells

Human iPS cells (hiPS cells), an iPS cell line (TkDA3-4) established at the University of Tokyo by introducing Oct4, Klf4, Sox2, and c-Myc into human-derived skin cells were cultured on inactivated mouse fibroblasts by treating with 10 µg/mL MitomycinC (manufactured by Wako Pure Chemical Industries Ltd.) in a medium mixture of Dulbecco's modified Eagle's medium and Ham's F-12 medium (manufactured by Sigma-Aldrich Co.) (mixing ratio 1:1) supplemented with 0.1 mM non-essential amino acid (manufactured by Invitrogen Corp.), PSG (100 U/mL penicillin, 100 U/mL streptomycin, and 2 mM L-glutamine) (manufactured by Invitrogen Corp.), 20% knockout serum replacement additive (KSR, manufactured by Invitrogen Corp.), 0.1 mM 2-mercaptoethanol (manufactured by Invitrogen Corp.), and 5 ng/ml basic fibroblast growth factor (bFGF, manufactured by Wako Pure Chemical Industries Ltd.). The cells were subcultured every 3 days, and those cells maintaining an undifferentiated state were used.

In addition, mouse embryo-derived fibroblasts, a C3H10T1/2 cell line (hereinafter also referred to as "10T1/2 cells"), purchased from RIKEN BioResource Center were cultured in basal medium Eagle (BME, manufactured by Invitrogen Corp.) supplemented with 10% fetal bovine serum (FBS) and PSG (100 U/mL penicillin, 100 U/mL streptomycin, and 2 mM L-glutamine) (manufactured by Invitrogen Corp.). Then, the 10T1/2 cells, which had been deactivated in advance by treating with MitomycinC, were seeded so as to give a cell count of 8 x 10⁶/10-cm dish for use as feeder cells.

### <Production and Analysis of Megakaryocytes and Platelets>

The human iPS cells were seeded on the feeder cells such that the cell count was 5 × 10⁴ to 1 × 10⁵/10-cm dish, and then cultured in Iscove's Modified Dulbecco's Medium (IMDM, manufactured by Sigma-Aldrich Co.) supplemented with 15% FBS (manufactured by Invitrogen Corp.), PSG (100 U/mL penicillin, 100 U/mL streptomycin, and 2 mM L-glutamine) (manufactured by Invitrogen Corp.), ITS supplement (10 µg/ml insulin, 5.5 mg/ml human transferrin, 5 ng/ml sodium selenite) (manufactured by Invitrogen Corp.), 50 µg/ml ascorbic acid (manufactured by Sigma-Aldrich Co.), 0.45 mM α-monothioglycerol (MTG, manufactured by Sigma-Aldrich Co.), and 20 ng/ml vascular endothelial growth factor (VEGF, manufactured by R&D Systems, Inc.). This culturing was carried out at 37°C in 5% CO₂ using a CO₂ incubator (product name: HERA CELL 150i, manufactured by Thermo Fisher Scientific K.K.). Further, the medium was replaced every 3 to 4 days, and the culturing was continued under the conditions until Day 14 to 15 when a large number of sac structures (net-like structures) containing hematopoietic progenitor cells therein were confirmed.

Next, the sac structures were detached from the dish, split open, passed through a 40-µm cell strainer (manufactured by BD), and then centrifuged at 1,500 rpm for 8 minutes to collect hematopoietic progenitor cells. The collected hematopoietic progenitor cells were suspended in a 3% FBS-containing phosphate buffer solution (PBS). Then, the number of cells was counted. The hematopoietic progenitor cells were seeded on 10T1/2 cells (6 x 10⁵ cells/plate), which had been treated with MitomycinC in advance, newly prepared in a 6-well plate such that the number of the hematopoietic progenitor cells was 2 to 3 × 10⁴ cells/well. The hematopoietic progenitor cells were further cultured for 8 days in IMDM (manufactured by Sigma-Aldrich Co.) supplemented with 15% FBS (product name: manufactured by Invitrogen Corp.), PSG (100 U/mL penicillin, 100 U/mL streptomycin, and 2 mM L-glutamine) (manufactured by Invitrogen Corp.), ITS supplement (10 µg/ml insulin, 5.5 mg/mL human transferrin, 6.7 ng/mL sodium selenite) (manufactured by Invitrogen Corp.), 50 µg/ml ascorbic acid (manufactured by Invitrogen Corp.), 0. 45 mM MTG (manufactured by Sigma-Aldrich Co.), 100 ng/ml human thrombopoietin (human TPO, manufactured by R&D Systems, Inc.), 50 ng/ml human stem cell factor (human SCF, manufactured by R&D Systems, Inc.), and 25 U/ml heparin. Thereby, megakaryocytes/platelets were induced.

The induced megakaryocytes/platelets were collected with an anticoagulant solution (Acid Citrate Dextrose, ACD), and then 1/25 of the collected megakaryocytes/platelets of each well was stained using antibodies. The used antibodies were an allophycocyanin (APC)-labeled anti-CD41a (integrin αIIb β3 complex, HIP8 clone, manufactured by BioLegend, Inc.) antibody, a PE-modified anti-human CD42b (GPIbα, HIP1 clone, manufactured by eBioscience, Inc.) antibody, and an eFluor (registered trademark) 450-modified anti-human CD42a (GPIX, GR-P clone, manufactured by eBioscience, Inc.). In order to accurately measure the absolute number of cells to be analyzed, using TruCount Tubes (manufactured by BD Biosciences), 1/10 of the number of cells in each tube (namely, 1/250 of the number of cells in each well) were analyzed by flow cytometry. In flow cytometry, a FACS Aria II and a FACS Verse manufactured by Becton Dickinson were used. Note that, although 10 or more kinds of adhesion molecules are present in the platelets, CD41a (GPIIb / IIIa) is expressed in megakaryocytes, platelets, and some hematopoietic progenitor cells, CD42a (GPIX) and CD42b (GPIbα) are locally expressed in megakaryocytes and platelets, and are known as a platelet membrane glycoprotein that is widely used as a platelet marker. Therefore, in order to count the induced platelets, gating was carried out so that the CD41a (+) CD42a (+) cells had the same size as fresh platelets obtained from human peripheral blood, and included the CD41a and CD42a positive platelets. In addition, the CD41a (+) CD42a (+) CD42b (+) cells were counted by carrying out gating based on a CD42b expressed amount such that 99% or more of the fresh platelets among the CD41a (+) CD42a (+) cells were included.

### <Evaluation of the Inventive Compound>

Platelet induction was carried out on iPS cell-derived hematopoietic progenitor cells obtained as described above based on the conditions shown in <Protocol 1> of FIG. 1. After the iPS cell-derived hematopoietic progenitor cells had been reseeded, the test substance was added to the medium in "d14 or d15" shown in Protocol 1, and treated for 9 to 10 days. As the test substance, the compound (+)-I-10 shown as a representative example of the compound according to the present invention (hereinafter referred to as S-108470) was used. As a positive control, a TACE inhibitor described in WO2012/036257 (hereinafter referred to as "TACE inhibitor S") was used. As a negative control, DMSO (medium control) was used. After cultivation had finished, the cells were collected as described above, and then the number of CD41a (+) CD42a (+) platelets and the number of CD41a (+) CD42a (+) CD42b (+) platelets were counted by flow cytometry. One well was provided for each group for each test. The test was repeated 7 times, and the average and standard deviation of the obtained data were calculated.

As shown in FIG. 2, although the number of obtained CD41a (+) CD42a (+) platelets (10 and 30 µM) was slightly larger for the S-108470 group than the medium control group, no larger difference was found between the groups. Note that, for the TACE inhibitor group (15 µM) as well, no large difference was found in the number of obtained CD41a (+) CD42a (+) platelets compared with the medium control group (not shown). Next, the percentage of CD41a (+) CD42a (+) CD42b (+) platelets in each group was investigated. As shown in FIG. 3, there was a significant increase in the percentage of CD41a (+) CD42a (+) CD42b (+) platelets for the S-108470 group (10 and 30 µM) compared with the medium control group. As is clear from this result, S-108470, which is the compound according to the present invention, was confirmed to inhibit ADAM 17-mediated GPIbα shedding, without exhibiting cytotoxicity or having any impact on platelet differentiation in a culture system using iPS cells. In other words, S-108470, which is the compound according to the present invention, was confirmed to maintain a function of platelets when added in a culture solution.

Note that, the TACE inhibitor S (15 µM) also exhibited a significant increase in the percentage of CD41a (+) CD42a (+) CD42b (+) platelets.

### (Test Example 3) Verification of Concentration Dependence of Function-Maintaining Effect on Human Peripheral Blood-Derived Platelets

It was verified whether the compound according to the present invention demonstrated a function-maintaining effect on peripheral blood-derived platelets obtained by collecting blood in the same way as platelets obtained using a culture system such as that described above. First, ACD was added in a ratio of 1/10 to human peripheral blood obtained by collecting blood, and centrifuged at 900 rpm for 10 minutes without a break. The supernatant obtained by the centrifugation was further centrifuged at 1,500 rpm for 10 minutes without a break, and then the supernatant was removed. The obtained precipitate was suspended in a Tyrode-HEPES buffer (not containing calcium). The number of platelets in the suspension was counted, and the platelet-containing suspension (Washed Platelet) was adjusted to have a fixed number(about 5 x 10⁷) of platelets in 200 µL of the Tyrode-HEPES buffer (not containing calcium). Next, as described in Protocol 2 shown in FIG. 1, one of either S-108470 (0.1 to 100 µM), TACE inhibitor S (50 µM), or DMSO was added together with 100 µM of CCCP (carbonyl cyanide m-chlorophenyl hydrazone) to the washed platelets. After incubation at 37°C for 24 hours, the number of CD42b (GPIbα) (+) cells in the CD41a (+) platelets was measured by flow cytometry. Note that, it is known that addition of CCCP enhances GPIbα shedding on the surface of platelets (see Bergmeier et al., Blood, 2003, vol. 102, pp. 4229-4235).

As shown in FIG. 4, S-108470 (0.1 to 100 µM) inhibits GPIbα shedding in a concentration-dependent manner. Thus, S-108470, which is the compound according to the present invention, was confirmed to have a function-maintaining effect on human peripheral blood-derived platelets.

Note that, inhibition of GPIbα shedding was confirmed for the TACE inhibitor S (50 µM) as well (not shown).

As described above, according to the present invention, a function of platelets can be maintained by specifically suppressing metalloprotease activity of ADAM 17 and suppressing GPIbα shedding. As a result, the compound according to the present invention is useful as a reagent for producing, efficiently and in a large amount without requiring complicated actions, highly-safe platelets that are functionally stable, a medicine additive for stabilizing the quality of a blood product by maintaining a function of platelets in the blood product.

## Claims

1. A composition for maintaining a function of platelets, the composition comprising as an active ingredient a pyridone derivative, or a salt thereof, represented by formula (I), 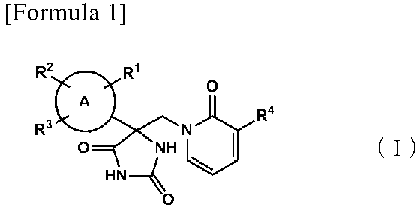 [wherein ring A represents an aryl, a heteroaryl, or a group represented by the following formula (a), 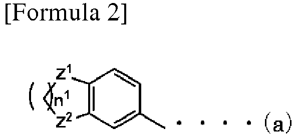
(wherein Z¹ and Z² each independently represent -CH₂- or -O-, and n¹ denotes an integer of 1 to 3),
R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO-₂NR⁶-, - NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 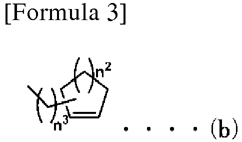
(wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
R³ represents a hydrogen atom, a halogen atom, or a C1-C6 alkyl group, and
R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].

2. The composition according to claim 1, wherein a compound represented by the formula (I) is (+)-5-(3,4-difluorophenyl)-5-[(3-methyl-2-oxopyridin-1(2H)-yl)methyl]imidazolidine-2,4-dione.

3. The composition according to claim 1 or 2, wherein the composition is a reagent for maintaining a function of platelets or is an additive to a platelet-containing blood product.

4. A method for preparing platelets, the method comprising adding, to a culture system for differentiating megakaryocytes from cells capable of differentiating into megakaryocytes and producing platelets from the megakaryocytes, a pyridone derivative, or a salt thereof, represented by formula (I), 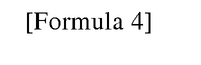 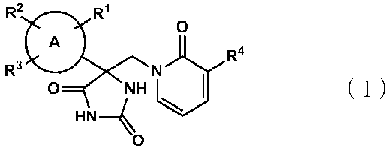 [wherein ring A represents an aryl, a heteroaryl, or a group represented by the following formula (a), 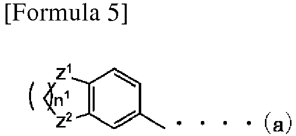 (wherein Z¹ and Z² each independently represent -CH₂- or -O-, and n¹ denotes an integer of 1 to 3),
R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO-₂NR⁶-, - NR⁶CO-, -CONR⁶-, -NR⁶COO-, -OCONR⁶-, -NR⁶CONR⁷-, or -NR ⁶SO₂NR⁷- (wherein R⁶ _{and} R⁷ each independently represent a hydrogen atom or a C1-C6 alkyl group),
R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 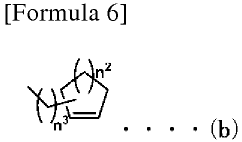 (wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
R³ represents a hydrogen atom, a halogen atom, or a C1-C6 alkyl group, and
R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].

5. The method according to claim 4, wherein a compound represented by the formula (I) is (+)-5-(3,4-difluorophenyl)-5-[(3-methyl-2-oxopyridin-1(2H)-yl)methyl]imidazolidine-2,4-dione.

6. The method according to claim 4 or 5, wherein a culture temperature in the culture system is from 35 to 39°C.

7. A culture obtained by adding, to a culture system for differentiating megakaryocytes from cells capable of differentiating into megakaryocytes and producing platelets from the megakaryocytes, a pyridone derivative, or a salt thereof, represented by formula (I), 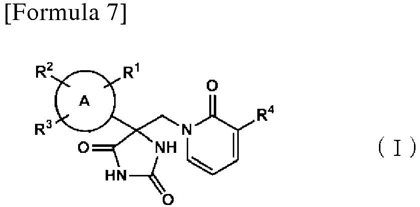 [wherein ring A represents an aryl, a heteroaryl, or a group represented by the following formula (a), 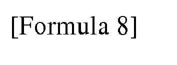 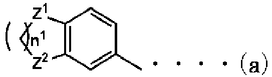
(wherein Z¹ and Z² each independently represent -CH₂- or -O-, and n¹ denotes an integer of 1 to 3),
R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO-₂NR⁶-, - NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 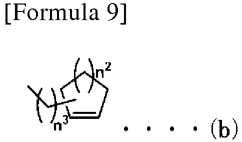
(wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
R³ represents a hydrogen atom, a halogen atom, or a C1-C6 alkyl group, and
R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].

8. The culture according to claim 7, wherein a compound represented by the formula (I) is (+)-5-(3,4-difluorophenyl)-5-[(3-methyl-2-oxopyridin-1(2H)-yl)methyl]imidazolidine-2,4-dione.

9. A platelet-containing blood product to which has been added a pyridone derivative, or a salt thereof, represented by formula (I), 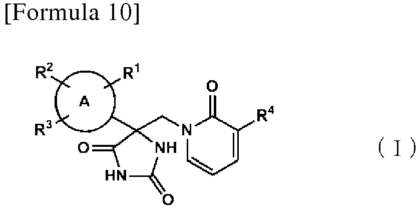
[wherein ring A represents an aryl, a heteroaryl, or a group represented by the following formula (a), 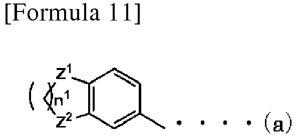
(wherein Z¹ and Z² each independently represent -CH₂- or -O-, and n¹ denotes an integer of 1 to 3),
R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO-₂NR⁶-,-NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 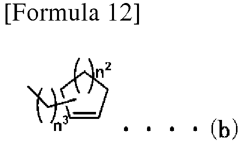
(wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
R³ represents a hydrogen atom, a halogen atom, or a C1-C6 alkyl group, and
R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].

10. The blood product according to claim 9, wherein a compound represented by the formula (I) is (+)-5-(3,4-difluorophenyl)-5-[(3-methyl-2-oxopyridin-1(2H)-yl)methyl]imidazolidine-2,4-dione.

11. A method for maintaining a function of platelets in a blood product, the method comprising adding, to a platelet-containing blood product, a pyridone derivative, or a salt thereof, represented by formula (I), 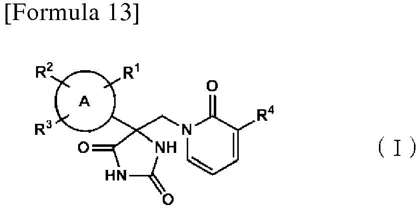
[wherein ring A represents an aryl, a heteroaryl, or a group represented by the following formula (a), 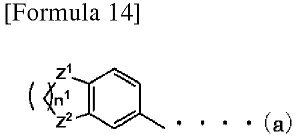
(wherein Z¹ and Z² each independently represent -CH₂- or -O-, and n¹ denotes an integer of 1 to 3),
R¹ represents a hydrogen atom, a halogen atom, a hydroxyl group, a cyano group, a nitro group, a C1-C6 haloalkyl group, a carboxyl group, an optionally substituted C1-C6 alkyl group, an optionally substituted C1-C6 alkoxy group, an optionally substituted C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted cycloalkenyl group, an optionally substituted cycloalkenylalkyl group, an optionally substituted heterocycloalkenyl group, an optionally substituted heterocycloalkenylalkyl group, an optionally substituted C2-C6 alkynyl group, or an optionally substituted alkynylalkyl group, or -J¹-X¹-R⁵ {wherein J¹ represents a single bond, alkylene, alkenylene, or alkynylene, X¹ represents a single bond, an oxygen atom, a sulfur atom, SO, SO₂, -CO-, -NR⁶-, -NR⁶SO₂-, -SO-₂NR⁶-, - NR⁶CO-, -CONR⁶-, -NR⁶COO-, -OCONR⁶-, -NR⁶CONR⁷-, or -NR⁶SO₂NR⁷- (wherein R⁶ and R⁷ each independently represent a hydrogen atom or a C1-C6 alkyl group),
R⁵ represents a hydrogen atom, a trifluoromethyl group, an optionally substituted C1-C6 alkyl group, an optionally substituted C1-C6 alkoxy group, an optionally substituted cycloalkyl group, an optionally substituted cycloalkylalkyl group, an optionally substituted heterocycloalkyl group, an optionally substituted heterocycloalkylalkyl group, a group represented by the following formula (b): 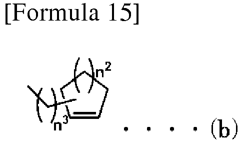
(wherein n² denotes an integer of 1 to 3 and n³ denotes an integer of 0 to 3),an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted C2-C6 alkynyl group, or an optionally substituted alkynylalkyl group},
R² represents a hydrogen atom, a halogen atom, a cyano group, a nitro group, a C1-C6 haloalkyl group, a carboxyl group, an optionally substituted C1-C6 alkyl group, a C1-C6 alkoxy group, a C1-C6 alkoxycarbonyl group, an optionally substituted cycloalkyl group, an optionally substituted cyclohexylalkylalkyl group, an optionally substituted heterocycloalkyl group, an optionally substituted heterocycloalkylalkyl group, an optionally substituted aryl group, an optionally substituted aralkyl group, an optionally substituted heteroaryl group, an optionally substituted heteroarylalkyl group, an optionally substituted C2-C6 alkenyl group, an optionally substituted alkenylalkyl group, an optionally substituted heterocycloalkenyl group, an optionally substituted heterocycloalkenylalkyl group, an optionally substituted C2-C6 alkynyl group, or an optionally substituted alkynylalkyl group,
R³ represents a hydrogen atom, a halogen atom, or a C1-C6 alkyl group, and
R⁴ represents a hydrogen atom, a halogen atom, a cyano group, a C1-C6 haloalkyl group, a C1-C6 alkoxy group, a hydoxymethyl group, a C1-C6 alkyl group, or a C2-C6 alkenyl group].

12. The method according to claim 11, wherein a compound represented by the formula (I) is (+)-5-(3,4-difluorophenyl)-5-[(3-methyl-2-oxopyridin-1(2H)-yl)methyl]imidazolidine-2,4-dione.


SEQUENCE LISTING

<110>  KAKEN PHARMACEUTICAL CO., LTD.

<120>  COMPOSITION FOR MAINTAINING FUNCTION OF PLATELETS

<130>  W7062

<150>  PCT/JP2014/065061
<151>  2014-06-06

<150>  JP 2013-120692
<151>  2013-06-07

<160>  1

<170>  PatentIn version 3.5

<210>  1
<211>  12
<212>  PRT
<213>  Artificial Sequence

<220>
<223>  Synthetic peptide


<220>
<221>  MISC_FEATURE
<222>  (1)..(1)
<223>  N-methylanthranilic acid

<220>
<221>  MISC_FEATURE
<222>  (11)..(11)
<223>  dinitrophenyl

<400>  1

Xaa Leu Ala Gln Ala Val Arg Ser Ser Lys Xaa Arg
1               5                   10